# EUROPEAN PATENT APPLICATION

(11) **EP 4 332 224 A2**
(43) Date of publication of application: **06.03.2024**
(21) Application number: 23214540.9
(22) Date of filing: 06.09.2019
(51) Int. Cl.: C12N 15/113

(54) **RNA AND DNA BASE EDITING VIA ENGINEERED ADAR RECRUITMENT**

(30) Priority: 06.09.2018 US 201862728007 P; 17.10.2018 US 201862766433 P; 29.11.2018 US 201862773146 P; 29.11.2018 US 201862773150 P; 15.12.2018 US 201862780241 P
(62) Divisional of application: 19857915.3
(71) Applicant: The Regents of the University of California, Oakland, CA 94607-5200 (US)
(72) Inventor: MALI, Prashant, La Jolla, 92093 (US); KATREKAR, Dhruva, La Jolla, 92093 (US); MELUZZI, Dario, La Jolla, 92093 (US); CHEN, Genghao, La Jolla, 92093 (US); FORD, Kyle M, La Jolla, 92093 (US)
(74) Representative: Gill Jennings & Every LLP

(57) **Abstract**

Disclosed herein is a system to recruit ADARs to catalyze therapeutic editing of point mutations via the use of engineered RNA scaffolds, engineered DNA scaffolds or DNA-RNA hybrid scaffolds. The system comprises an engineered ADAR2 guide RNA (adRNA) that bears a 20-100bp complementarity with the target RNA and ADAR2 recruiting domain from the GluR2 mRNA at either or both the 5' end or the 3' end.

## Description

### CROSS-REFERENCE

This application claims the benefit of U.S. Provisional Application No. 62/728,007, filed September 6, 2018, U.S. Provisional Application No. 62/766,433, filed October 17, 2018, and U.S. Provisional Application No. 62/780,241, filed December 15, 2018, which are incorporated by reference herein in their entirety.

### STATEMENT REGARDING GOVERNMENT SUPPORT

This disclosure was made with government support under grant numbers CA222826, GM123313, and HG009285 awarded by the National Institutes of Health. The government has certain rights in the invention.

### SUMMARY

An aspect of the disclosure provides a vector. In some cases, the vector can comprise a nucleic acid with a polynucleotide sequence encoding for at least one RNA editing entity recruiting domain, wherein: (a) the polynucleotide sequence encoding for the at least one RNA editing entity recruiting domain does not form a secondary structure comprising a stem-loop, or (b) wherein the polynucleotide sequence encoding for the at least one RNA editing entity recruiting domain comprises at least about 80% sequence identity to at least one sequence selected from: an Alu domain encoding sequence, an Apolipoprotein B mRNA Editing Catalytic Polypeptide-like (APOBEC) recruiting domain encoding sequence, and any combination thereof. In some cases, the polynucleotide sequence encoding for the RNA editing entity recruiting domain can comprise at least about 80% sequence identity to the Alu domain sequence. In some cases, the polynucleotide sequence encoding for the RNA editing entity recruiting domain can comprise at least about 80% sequence identity to the APOBEC recruiting domain encoding sequence. In some cases, the vector can be a viral vector. In some cases, the vector can be a liposome. In some cases, the vector can be a nanoparticle. In some cases, the RNA editing entity recruiting domain can be configured to recruit an ADAR protein. In some cases, the ADAR protein can be an ADAR1, ADAR2, or ADAR3 protein. In some cases, the ADAR protein can be a human ADAR protein. In some cases, the ADAR protein can be a recombinant ADAR protein. In some cases, the ADAR protein can be a modified ADAR protein. In some cases, the RNA editing entity recruiting domain can be configured to recruit an APOBEC protein. In some cases, the APOBEC protein can be an APOBEC1, APOBEC2, APOBEC3A, APOBEC3B, APOBEC3C, APOBEC3E, APOBEC3F, APOBEC3G, APOBEC3H, or APOBEC4 protein. In some cases, the ADAR protein can be a human ADAR protein. In some cases, the ADAR protein can be a recombinant ADAR protein. In some cases, the ADAR protein can be a modified ADAR protein. In some cases, the at least one RNA editing entity recruiting domain may not form a secondary structure comprising a stem-loop. In some cases, the polynucleotide sequence can encode for at least two RNA editing recruiting domains. In some cases, at least one of the at least two RNA editing recruiting domains can be an Alu domain. In some cases, the Alu domain sequence can form a secondary structure that comprises at least one stem-loop. In some cases, the Alu domain encoding sequence can comprise a plurality of Alu repeats. In some cases, the Alu domain encoding sequence can be at least partially single stranded. In some cases, at least one of the at least two RNA editing recruiting domains can be an APOBEC recruiting domain. In some cases, at least one of the at least two RNA editing recruiting domain encoding sequences can comprise at least about 80% sequence identity to a GluR2 domain encoding sequence. In some cases, at least one of the at least two RNA editing recruiting domains can be a GluR2 domain. In some cases, at least one of the at least two RNA editing recruiting domains can be a Cast3 domain. In some cases, the at least two RNA editing recruiting domains can be the Alu domain and the APOBEC recruiting domain. In some cases, the vector can further comprise a nucleic acid encoding for an RNA that can be complementary to at least a portion of a target RNA. In some cases, the nucleic acid encoding for the RNA that can be complementary to at least the portion of the target RNA can be from about 10 base pairs (bp) to about 1000 bp in length. In some cases, the nucleic acid encoding for the at least one RNA editing entity recruiting domain and the nucleic acid encoding for the RNA that can be complementary to at least the portion of the target RNA can comprise a contiguous nucleic acid of at least about 200 bp in length. In some cases, the nucleic acid can be chemically synthesized. In some cases, the nucleic acid can be genetically encoded. In some cases, the vector can comprise DNA. In some cases, the DNA can be double stranded. In some cases, the DNA can be single stranded. In some cases, the vector can comprise RNA. In some cases, the RNA can comprise a base modification. In some cases, the vector can be an adeno-associated virus (AAV) vector. In some cases, the AAV can be a recombinant AAV (rAAV). In some cases, the AAV can be selected from the group consisting of an AAV1 serotype, an AAV2 serotype, an AAV3 serotype, an AAV4 serotype, an AAV5 serotype, an AAV6 serotype, an AAV7 serotype, an AAV8 serotype, an AAV9 serotype, a derivative of any these, and a combination of any of these. In some cases, the AAV can be the AAV5 serotype or a derivative thereof. In some cases, the derivative of the AAV can comprise a modified VP1 protein. In some cases, the APOBEC recruiting domain can be selected from the group consisting of: an APOBEC1 recruiting domain, an APOBEC2 recruiting domain, an APOBEC3A recruiting domain, an APOBEC3B recruiting domain, an APOBEC3C recruiting domain, an APOBEC3E recruiting domain, an APOBEC3F recruiting domain, an APOBEC3G recruiting domain, an APOBEC3H recruiting domain, an APOBEC4 recruiting domain, and any combination thereof. In some cases, the RNA editing entity recruiting domain can recruit at least two RNA editing entities, and wherein at least one of the at least two polynucleotide sequences encoding for the RNA editing entities comprises at least about 80% identity to an APOBEC protein encoding sequence. In some cases, the RNA editing entity recruiting domain can recruit at least two RNA editing entities, and wherein at least one of the at least two polynucleotide sequences encoding for the RNA editing entities comprises at least about 80% identity to an ADAR protein encoding sequence. In some cases, the RNA recruiting domain encoded by the nucleic acid can comprise at least one stem loop. In some cases, the polynucleotide sequence encoding for the RNA editing recruiting domain can comprise a secondary structure that can be substantially a cruciform. In some cases, the polynucleotide sequence encoding for the RNA editing recruiting domain can comprise at least two secondary structures that are substantially cruciforms. In some cases, the polynucleotide sequence encoding for the RNA editing entity recruiting domain can be positioned between a polynucleotide sequence that forms the at least two secondary structures that are substantially cruciforms. In some cases, the cruciform secondary structure can comprise a stem-loop adjoining at least one pair of at least partially complementary strands of the cruciform secondary structure. In some cases, the polynucleotide sequence encoding for the RNA editing recruiting domain can comprise a secondary structure that can be substantially a toehold. In some cases, a non-naturally occurring RNA can be encoded by the vector. In some cases, a kit can comprise the vector in a container. In some cases, the kit can further comprise a syringe. In some cases, the container can be the syringe. In some cases, an isolated cell can comprise the vector. In some cases, a pharmaceutical composition can comprise the vector in unit dose form. In some cases, the pharmaceutical composition can further comprise a pharmaceutically acceptable excipient, diluent, or carrier. In some cases, the pharmaceutical composition can comprise a second active ingredient. In some cases, a method of treating a disease or condition in a subject comprising administering to the subject the vector. In some cases, the administering can be by intravenous injection, intramuscular injection, an intrathecal injection, an intraorbital injection, a subcutaneous injection, or any combination thereof. In some cases, the method can further comprise administering a second therapy to the subject. In some cases, the disease or condition can be selected from the group consisting of: a neurodegenerative disorder, a muscular disorder, a metabolic disorder, an ocular disorder, a cell proliferative disorder *(e.g.,* a neoplasm) and any combination thereof. In some cases, the disease or condition can be Alzheimer's disease. In some cases, the disease or condition can be muscular dystrophy. In some cases, the disease or condition can be retinitis pigmentosa. In some cases, the disease or condition can be Parkinson disease. In some cases, the disease or condition can be pain. In some cases, the disease or condition can be Stargardt macular dystrophy. In some cases, the disease or condition can be Charcot-Marie-Tooth disease. In some cases, the disease or condition can be Rett syndrome. In some cases, the administering can be sufficient to decrease expression of a gene relative to prior to the administering. In some cases, the administering can be sufficient to edit at least one point mutation in the subject. In some cases, the administering can be sufficient to edit at least one stop codon in the subject, thereby producing a readthrough of the stop codon. In some cases, the administering can be sufficient to produce an exon skip in the subject.

Another aspect of the disclosure provides a vector. In some cases, the vector can comprise a nucleic acid encoding for RNA with a two dimensional shape that can be substantially a cruciform, wherein the RNA comprises at least one sequence encoding an RNA editing entity recruiting domain. In some cases, the vector can further comprise a nucleic acid encoding for RNA with at least one targeting domain encoding sequence that can be complementary to at least a portion of a target RNA sequence. In some cases, the nucleic acid encoding for the RNA with the at least one targeting domain that can be complementary to at least the portion of the target RNA sequence further can comprise a substantially linear two dimensional structure. In some cases, a non-naturally occurring RNA can be encoded by the vector. In some cases, a kit can comprise the vector in a container. In some cases, the kit can further comprise a syringe. In some cases, the container can be the syringe. In some cases, an isolated cell can comprise the vector. In some cases, a pharmaceutical composition can comprise the vector in unit dose form. In some cases, the pharmaceutical composition can further comprise a pharmaceutically acceptable excipient, diluent, or carrier. In some cases, the pharmaceutical composition can comprise a second active ingredient. In some cases, a method of treating a disease or condition in a subject comprising administering to the subject the vector. In some cases, the administering can be by intravenous injection, intramuscular injection, an intrathecal injection, an intraorbital injection, a subcutaneous injection, or any combination thereof. In some cases, the method can further comprise administering a second therapy to the subject. In some cases, the disease or condition can be selected from the group consisting of: a neurodegenerative disorder, a muscular disorder, a metabolic disorder, an ocular disorder, a cell proliferative disorder *(e.g.,* a neoplasm) and any combination thereof. In some cases, the disease or condition can be Alzheimer's disease. In some cases, the disease or condition can be muscular dystrophy. In some cases, the disease or condition can be retinitis pigmentosa. In some cases, the disease or condition can be Parkinson disease. In some cases, the disease or condition can be pain. In some cases, the disease or condition can be Stargardt macular dystrophy. In some cases, the disease or condition can be Charcot-Marie-Tooth disease. In some cases, the disease or condition can be Rett syndrome. In some cases, the administering can be sufficient to decrease expression of a gene relative to prior to the administering. In some cases, the administering can be sufficient to edit at least one point mutation in the subject. In some cases, the administering can be sufficient to edit at least one stop codon in the subject, thereby producing a read-through of the stop codon. In some cases, the administering can be sufficient to produce an exon skip in the subject.

Another aspect of the disclosure provides for a non-naturally occurring RNA. In some cases, the non-naturally occurring RNA can comprise a first domain sequence comprising a two dimensional shape that can be substantially a cruciform and a second domain sequence that has a substantially linear two dimensional structure connected to the first domain sequence, wherein the first domain sequence encodes for an RNA editing entity recruiting domain and the second domain sequence encodes for a targeting domain, wherein the second domain sequence can be complementary to at least a portion of a target RNA. In some cases, the non-naturally occurring RNA can further comprise a third domain sequence attached to the second domain sequence. In some cases, the third domain sequence can comprise an RNA editing entity recruiting domain encoding sequence that forms a secondary structure having a two dimensional shape that can be substantially a cruciform. In some cases, at least one base of the non-naturally occurring RNA can comprise a chemical modification. In some cases, at least one sugar of the non-naturally occurring RNA can comprise a chemical modification. In some cases, a kit can comprise the non-naturally occurring RNA in a container. In some cases, the kit can further comprise a syringe. In some cases, the container can be the syringe. In some cases, an isolated cell can comprise the non-naturally occurring RNA. In some cases, a pharmaceutical composition can comprise the non-naturally occurring RNA in unit dose form. In some cases, the pharmaceutical composition can further comprise a pharmaceutically acceptable excipient, diluent, or carrier. In some cases, the pharmaceutical composition can comprise a second active ingredient. In some cases, a method of treating a disease or condition in a subject comprising administering to the subject the non-naturally occurring RNA. In some cases, the administering can be by intravenous injection, intramuscular injection, an intrathecal injection, an intraorbital injection, a subcutaneous injection, or any combination thereof. In some cases, the method can further comprise administering a second therapy to the subject. In some cases, the disease or condition can be selected from the group consisting of: a neurodegenerative disorder, a muscular disorder, a metabolic disorder, an ocular disorder, a cell proliferative disorder *(e.g.,* a neoplasm) and any combination thereof. In some cases, the disease or condition can be Alzheimer's disease. In some cases, the disease or condition can be muscular dystrophy. In some cases, the disease or condition can be retinitis pigmentosa. In some cases, the disease or condition can be Parkinson disease. In some cases, the disease or condition can be pain. In some cases, the disease or condition can be Stargardt macular dystrophy. In some cases, the disease or condition can be Charcot-Marie-Tooth disease. In some cases, the disease or condition can be Rett syndrome. In some cases, the administering can be sufficient to decrease expression of a gene relative to prior to the administering. In some cases, the administering can be sufficient to edit at least one point mutation in the subject. In some cases, the administering can be sufficient to edit at least one stop codon in the subject, thereby producing a readthrough of the stop codon. In some cases, the administering can be sufficient to produce an exon skip in the subject.

Another aspect of the disclosure provides for a nucleic acid. In some cases, the nucleic acid can comprise an RNA editing entity recruiting domain and an antisense domain sequence, wherein when the nucleic acid can be contacted with an RNA editing entity and a target nucleic acid complementary to at least a portion of the antisense domain, modifies at least one base pair of the target nucleic acid at an efficiency of at least about 4 times greater than a comparable nucleic acid complexed with a Cas13b protein or an active fragment thereof, as determined by Sanger Method sequencing of the target nucleic acid.

Another aspect of the disclosure provides for a nucleic acid. In some cases, the nucleic acid can comprise an RNA editing entity recruiting domain and an antisense domain, wherein the nucleic acid when contacted with an RNA editing entity and a target nucleic acid complementary to at least a portion of the antisense domain, modifies at least one base pair of the target nucleic acid at an efficiency of at least about 4 times greater than a comparable nucleic acid complexed with a GluR2 domain and the antisense domain, as determined by Sanger Method sequencing of the target nucleic acid. In some cases, the nucleic acid can comprise RNA. In some cases, the target nucleic acid can comprise RNA. In some cases, the RNA can be mRNA. In some cases, the mRNA can encode a protein or a portion thereof. In some cases, a dysfunction of the protein or portion thereof can be implicated in a disease or condition. In some cases, the disease or condition can be selected from the group consisting of: a neurodegenerative disorder, a muscular disorder, a metabolic disorder, an ocular disorder, a cell proliferative disorder (*e.g*., a neoplasm) and any combination thereof. In some cases, the RNA can be small interfering RNA (siRNA). In some cases, the RNA editing entity recruiting domain can comprise at least about 80% identity to a GluR2 domain. In some cases, the RNA editing entity recruiting domain can comprise at least about 80% identity to an Alu domain. In some cases, the RNA editing entity recruiting domain can comprise at least about 80% identity to an APOBEC recruiting domain. In some cases, the RNA editing entity recruiting domain can be configured to recruit an ADAR protein. In some cases, the ADAR protein can be an ADAR1, ADAR2, or ADAR3 protein. In some cases, the ADAR protein can be a human ADAR protein. In some cases, the ADAR protein can be a recombinant ADAR protein. In some cases, the ADAR protein can be a modified ADAR protein. In some cases, the RNA editing entity recruiting domain can be configured to recruit an APOBEC protein. In some cases, the APOBEC protein can be an APOBEC1, APOBEC2, APOBEC3A, APOBEC3B, APOBEC3C, APOBEC3E, APOBEC3F, APOBEC3G, APOBEC3H, or APOBEC4 protein. In some cases, the ADAR protein can be a human ADAR protein. In some cases, the ADAR protein can be a recombinant ADAR protein. In some cases, the ADAR protein can be a modified ADAR protein. In some cases, the nucleic acid can be chemically synthesized. In some cases, the nucleic acid can be genetically encoded. In some cases, a kit can comprise the nucleic acid in a container. In some cases, the kit can further comprise a syringe. In some cases, the container can be the syringe. In some cases, an isolated cell can comprise the nucleic acid. In some cases, a pharmaceutical composition can comprise the nucleic acid in unit dose form. In some cases, the pharmaceutical composition can further comprise a pharmaceutically acceptable excipient, diluent, or carrier. In some cases, the pharmaceutical composition can comprise a second active ingredient. In some cases, a method of treating a disease or condition in a subject comprising administering to the subject the nucleic acid. In some cases, the administering can be by intravenous injection, intramuscular injection, an intrathecal injection, an intraorbital injection, a subcutaneous injection, or any combination thereof. In some cases, the method can further comprise administering a second therapy to the subject. In some cases, the disease or condition can be selected from the group consisting of: a neurodegenerative disorder, a muscular disorder, a metabolic disorder, an ocular disorder, a cell proliferative disorder (*e.g*., a neoplasm) and any combination thereof. In some cases, the disease or condition can be Alzheimer's disease. In some cases, the disease or condition can be muscular dystrophy. In some cases, the disease or condition can be retinitis pigmentosa. In some cases, the disease or condition can be Parkinson disease. In some cases, the disease or condition can be pain. In some cases, the disease or condition can be Stargardt macular dystrophy. In some cases, the disease or condition can be Charcot-Marie-Tooth disease. In some cases, the disease or condition can be Rett syndrome. In some cases, the administering can be sufficient to decrease expression of a gene relative to prior to the administering. In some cases, the administering can be sufficient to edit at least one point mutation in the subject. In some cases, the administering can be sufficient to edit at least one stop codon in the subject, thereby producing a read-through of the stop codon. In some cases, the administering can be sufficient to produce an exon skip in the subject.

Another aspect of the disclosure can provide for a nucleic acid. In some cases, the nucleic acid can comprise sequences comprising an antisense domain, a first stem-loop forming sequence, and a second stem-loop forming sequence, wherein the nucleic acid when contacted with (a) a first polypeptide comprising a first portion of an RNA editing entity and a first polynucleotide binding domain configured to bind to the first stem-loop forming sequence, and (b) a second polypeptide comprising a second portion of an RNA editing entity and a second polynucleotide binding domain configured to bind to the second stem-loop forming sequence, and (c) a target nucleic acid complementary to at least a portion of the antisense domain, modifies at least one base pair of the target nucleic acid. In some cases, the first stem-loop or the second stem-loop can be an MS2 stem loop. In some cases, the first stem loop or the second stem-loop can be a BoxB stem-loop. In some cases, the first stem-loop or the second stem-loop can be a U1A stem-loop. In some cases, the first portion of the RNA editing entity or the second portion of the RNA editing entity can comprise an N-terminal fragment of an ADAR deaminase domain encoding sequence. In some cases, the first portion of the RNA editing entity or the second portion of the RNA editing entity can comprise a C-terminal fragment of an ADAR deaminase domain encoding sequence. In some cases, the first polynucleotide binding domain or the second polynucleotide binding domain can comprise an MS2 coat protein. In some cases, the first polynucleotide binding domain or the second polynucleotide binding domain can comprise a Lambda N peptide. In some cases, the first polynucleotide binding domain or the second polynucleotide binding domain can comprise a human nucleic acid binding protein. In some cases, the human nucleic acid binding protein can be a U1A protein, a TBP6.7 protein, a human histone stem-loop binding protein, or a DNA binding domain of a glucocorticoid receptor. In some cases, the RNA editing entity can be capable of performing an adenosine to inosine mutation on the target nucleic acid. In some cases, the RNA editing entity can be capable of performing a cytosine to thymine mutation on the target nucleic acid. In some cases, a kit can comprise the nucleic acid in a container. In some cases, the kit can further comprise a syringe. In some cases, the container can be the syringe. In some cases, an isolated cell can comprise the nucleic acid. In some cases, a pharmaceutical composition can comprise the nucleic acid in unit dose form. In some cases, the pharmaceutical composition can further comprise a pharmaceutically acceptable excipient, diluent, or carrier. In some cases, the pharmaceutical composition can comprise a second active ingredient. In some cases, a method of treating a disease or condition in a subject comprising administering to the subject the nucleic acid. In some cases, the administering can be by intravenous injection, intramuscular injection, an intrathecal injection, an intraorbital injection, a subcutaneous injection, or any combination thereof. In some cases, the method can further comprise administering a second therapy to the subject. In some cases, the disease or condition can be selected from the group consisting of: a neurodegenerative disorder, a muscular disorder, a metabolic disorder, an ocular disorder, a cell proliferative disorder (*e.g*., a neoplasm) and any combination thereof. In some cases, the disease or condition can be Alzheimer's disease. In some cases, the disease or condition can be muscular dystrophy. In some cases, the disease or condition can be retinitis pigmentosa. In some cases, the disease or condition can be Parkinson disease. In some cases, the disease or condition can be pain. In some cases, the disease or condition can be Stargardt macular dystrophy. In some cases, the disease or condition can be Charcot-Marie-Tooth disease. In some cases, the disease or condition can be Rett syndrome. In some cases, the administering can be sufficient to decrease expression of a gene relative to prior to the administering. In some cases, the administering can be sufficient to edit at least one point mutation in the subject. In some cases, the administering can be sufficient to edit at least one stop codon in the subject, thereby producing a read-through of the stop codon. In some cases, the administering can be sufficient to produce an exon skip in the subject.

Another aspect of the disclosure provides a method of treating muscular dystrophy in a subject. In some cases, the method can comprise: administering to the subject a pharmaceutical composition comprising an adeno-associated virus (AAV) vector that comprises a first nucleic acid encoding a second nucleic acid, wherein the second nucleic acid comprises (a) an antisense region that can be at least partially complementary to an RNA sequence implicated in muscular dystrophy, and (b) at least one RNA editing entity recruiting domain, wherein the at least one RNA editing entity recruiting domain does not comprise a stem-loop, or wherein the at least one RNA editing entity recruiting domain comprises at least about 80% sequence identity to at least one of: an Alu domain, an Apolipoprotein B mRNA Editing Catalytic Polypeptide-like (APOBEC) recruiting domain, and any combination thereof. In some cases, the pharmaceutical composition can be in unit dose form. In some cases, the administering can be at least once a week. In some cases, the administering can be at least once a month. In some cases, the administering can be by injection. In some cases, the injection can be subcutaneous, intravenous, infusion, intramuscular, intrathecal, or intraperitoneal injection. In some cases, the administering can be transdermal, transmucosal, oral, or pulmonary. In some cases, the method can further comprise administering a second therapy to the subject.

Another aspect of the disclosure can provide a method of making a vector. In some cases, the method can comprise: cloning at least one copy of a nucleic acid into the vector, wherein the nucleic acid encodes for at least one RNA editing entity recruiting domain, and wherein a sequence encoding the at least one RNA editing entity recruiting domain does not form a secondary structure that comprises a stem-loop, or wherein the nucleic acid that encodes the at least one RNA editing entity recruiting domain comprises at least about 80% sequence identity to a sequence selected from: an Alu domain encoding sequence, an Apolipoprotein B mRNA Editing Catalytic Polypeptide-like (APOBEC) recruiting domain encoding sequence, and any combination thereof. In some cases, the vector can be a viral vector. In some cases, the viral vector can be an AAV vector. In some cases, the viral vector can comprise a modified VP1 protein. In some cases, the vector can be a liposome. In some cases, the vector can be a nanoparticle. In some cases, the method further comprises transfecting or transducing the vector into an isolated human cell.

Aspects of this disclosure relate to an engineered ADAR1 or ADAR2 guide RNA ("adRNA") comprising, or alternatively consisting essentially of, or yet further consisting of, a sequence complementary to a target RNA, that optionally comprises, or consists essentially of, or yet further consists of the engineered ADAR2 and an ADAR2 recruiting domain derived from GluR2 mRNA. In some embodiments, the sequence complementary to the target RNA comprises, or consists essentially of, or yet further consists of, between about 15 to 200 or alternatively from 20 to 100 base pairs. In one aspect, the engineered adRNA comprises, or consists essentially of, or yet further consists of no ADAR recruiting domains. In some embodiments, the ADAR recruiting domains comprise, or alternatively consist essentially of, or yet further consist of GluR2 mRNA, Alu repeat elements or other RNA motifs to which ADAR binds. In one aspect, the engineered adRNA comprises, or consists essentially of, or yet further consists of between about 1 to 10 ADAR recruiting domains. In another aspect, the ADAR2 recruiting domain can be derived from GluR2 mRNA and can be located at the 5' end or the 3' end of the engineered adRNA. In still further embodiments, the engineered adRNA comprises, or consists essentially of, or yet further consists of, the GluR2 mRNA at both the 5' end and the 3' end. In a further aspect, the engineered adRNA of this disclosure further comprises, or alternatively consists essentially of, or yet further consists of two MS2 hairpins flanking the sequence complementary to a target RNA.

In some embodiments, the target RNA can be ornithine transcarbamylase. Also provided herein is a complex comprising, or alternatively consisting essentially of, or yet further consisting of, an AdRNA as disclosed herein hybridized to a complementary polynucleotide under conditions of high stringency. In one aspect, the polynucleotide can be DNA. In another aspect, the polynucleotide can be RNA.

In one aspect, the engineered adRNA of this disclosure, further comprises, or alternatively consists essentially of, or yet further consists of an editing inducer element.

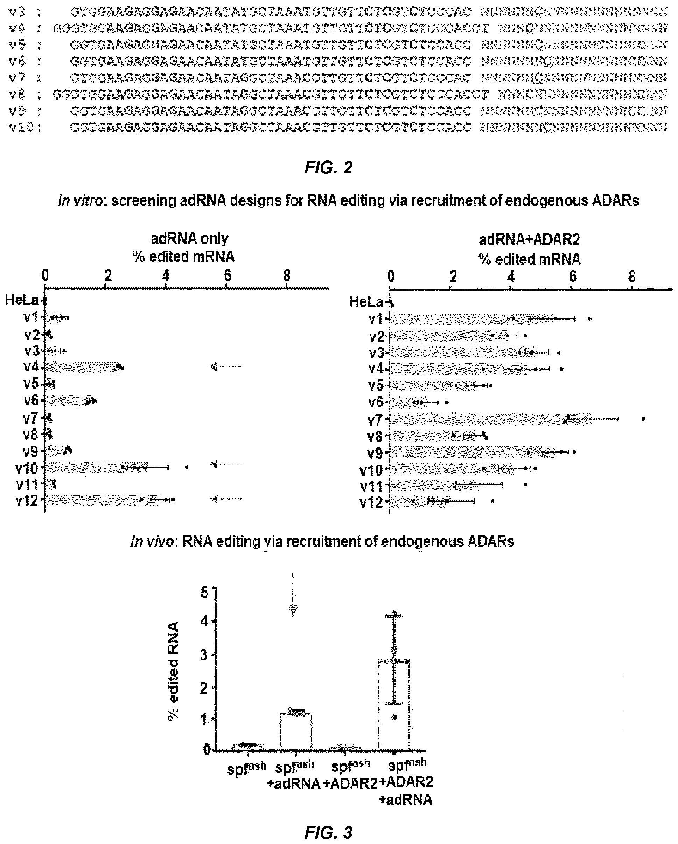

Further aspects relate to an engineered ADAR2 guide RNA ("adRNA") encoded by a sequence selected from the group of sequences provided in **TABLE 1** or **FIG. 2****.** The adRNAs can be combined with a carrier, such as a pharmaceutically acceptable carrier, examples of such are provided herein.

Also disclosed herein is an engineered adRNA-snRNA (small nuclear RNA) fusion. In one aspect, the engineered adRNA further comprises, or alternatively consists essentially of, or yet further consists of an N-terminal mitochondrial targeting sequence (MTS) to facilitate localization of the engineered adRNA to the mitochondria. In another aspect, provided herein can be an engineered further comprising, or alternatively consisting essentially of, or yet further consisting of a cis-acting zipcode to facilitate localization of the engineered adRNA into peroxisomes, endosomes and exosomes.

Further provided herein is small molecule regulatable engineered adRNA. In one aspect, disclosed herein are engineered adRNA-aptamer fusions. Non-limiting examples of aptamers that can be used for this purpose include aptamers that bind flavin mononucleotide, guanine, other natural metabolites, or sugars. Also disclosed herein is a U1A-ADAR fusion, entirely of human origin.

Also disclosed herein is a complex comprising, or alternatively consisting essentially of, or yet further consisting of an engineered adRNA of this disclosure hybridized to a complementary polynucleotide under conditions of high stringency.

Also provided herein is a vector comprising, or alternatively consisting essentially of, or yet further consisting of one or more of the isolated polynucleotide sequence encoding the engineered adRNA of this disclosure and optionally regulatory sequences operatively linked to the isolated polynucleotide. Non-limiting examples of a vector include a plasmid or a viral vector such as a retroviral vector, a lentiviral vector, an adenoviral vector, or an adeno-associated viral vector.

Further disclosed herein is a recombinant cell further comprising or alternatively consisting essentially of, or yet further consisting of the vector described above, wherein the engineered adRNA can be recombinantly expressed.

Compositions comprising one or more of the above-noted compounds and a carrier are provided. In one embodiment, the composition can be a pharmaceutical composition and therefore further comprises at least a pharmaceutically acceptable carrier or a pharmaceutically acceptable excipient. The compositions are formulated for various delivery modes, e.g., systemic (oral) or local.

Also provided herein is a method of modifying protein expression comprising, or alternatively consisting essentially of, or yet further consisting of contacting a polynucleotide encoding the protein, the expression of which is to be modified, with the engineered adRNA of this disclosure.

Still further aspects relate to methods of treating a disease or disorder associated with aberrant protein expression comprising, or alternatively consisting essentially of, or yet further consisting of, administering an effective amount of any one or more of the engineered adRNA disclosed herein and/or uses for an effective amount of any one or more of the engineered adRNA disclosed herein to a subject in need thereof and for treating a disease or disorder associated with aberrant protein expression. In one particular aspect, provided herein is a method of treating Duchenne Muscular Dystrophy comprising, or alternatively consisting essentially of, or yet further consisting of administering to a subject in need of such treatment an effective amount of one or more of the engineered adRNA of this disclosure.

The disclosure demonstrates validation of this approach *in vivo* in the spf-ash mouse model of ornithine transcarbamylase deficiency. This model bears a G->A point mutation in the last nucleotide of exon 4. Upon delivery of only adRNA via AAVs, up to 1% correction of the point mutation in the absence of the overexpression of the ADAR enzymes was observed.

Additional aspects relate to the same or similar structures comprising, or alternatively consisting essentially of, or yet further consisting of, DNA or a combination of DNA and RNA. Further aspects relate to kits comprising any one or more of the embodiments above and instructions for use *in vitro* and/or *in vivo.*

Aspects of the disclosure can relate to ADAR and APOBEC systems for gene editing. Some aspects relate to an ADAR system for exon skipping comprising an adRNA targeting a splice acceptor and/or a branch point in an intron and, optionally, an ADAR enzyme. In some embodiments, the ADAR enzyme can be ADAR1, ADAR2, or a mutant or variant each thereof. In some embodiments, the mutant or variant can be selected from ADAR1 (E1008Q) and ADAR2 (E488Q). In some embodiments, the intron can be comprised in a gene selected from dystrophin, SCN9A, or ornithine transcarbamylase. In some cases, the adRNA can be selected from **SEQUENCE SET 1.** Further aspects can relate to a method of treating a disease, disorder, or condition characterized by aberrant gene expression comprising, administering the disclosed ADAR system. In some embodiments, the disease, disorder, or condition can be selected from Duchenne muscular dystrophy or ornithine transcarbamylase deficiency. In some embodiments, the disease, disorder, or condition can be associated with pain.

Additional aspects relate to an APOBEC system for cytosine to thymine editing comprising a pair of gRNA that create alipoprotein B mRNA like structure and, optionally, an APOBEC enzyme. In some embodiments, the pair of gRNA can be the pair of sequences provided in **SEQUENCE SET 2.**

### BRIEF DESCRIPTION OF THE DRAWINGS

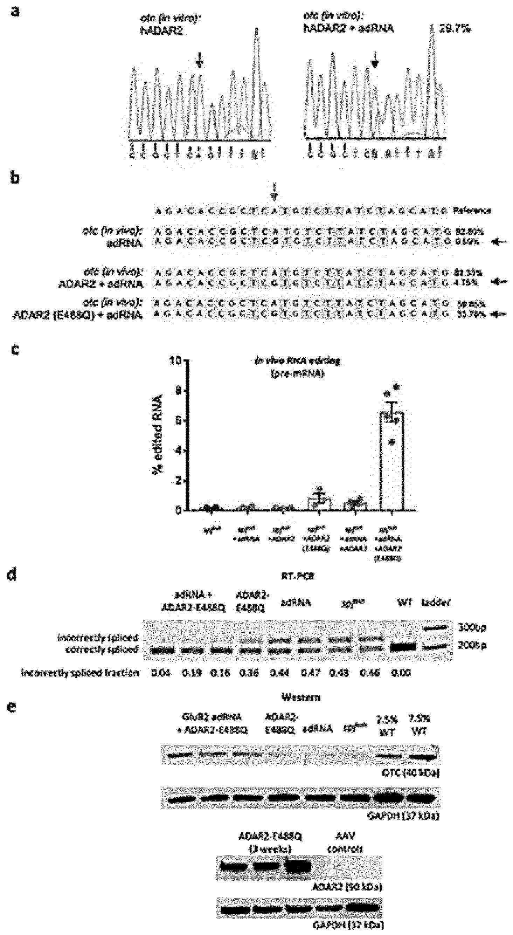

**FIG. 1** is a schematic showing endogenous recruitment of ADARs.
**FIG. 2** provides a listing of stabilized scaffolds being evaluated for (1) improved efficiency and (2) ability to recruit ADAR1s (SEQ ID NOs:2-9).
**FIG. 3** shows the results of *in vitro* and *in vivo* screening of exemplary adRNAs.

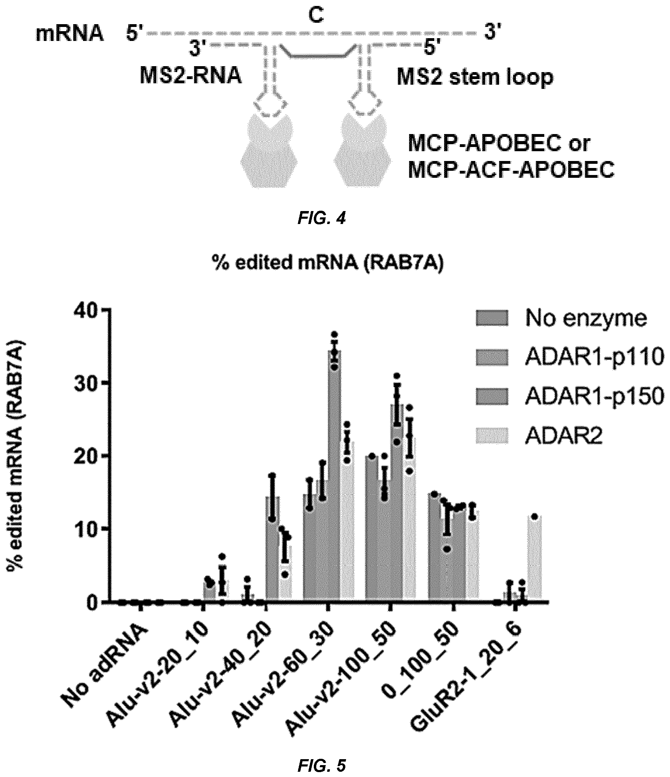

**FIG. 4** is a schematic of how MCP-APOBEC fusions or MCP-ACF-APOBEC fusions are recruited via MS2-RNAs bearing two MS2 stem loops. As shown in the Figure, the target cytosine can be kept single stranded so as to be accessible for APOBEC mediated editing via creation of a bulge. A bulge was created by using either the exact target sequence (20-30 base pairs) or the sequence ACATATATGATACAATTTGATCAGTATATT (SEQ ID NO: 175) in the MS2-RNA between the MS2 stem loops (blue) along with complementary sequences (20-30 base pairs) on either side of the MS2 stem loops (green) that bind to the mRNA of interest. The sequence ACATATATGATACAATTTGATCAGTATATT (SEQ ID NO: 176) is taken from the naturally occurring apoB substrate that the APOBEC edits. The MS2 stem loop sequence used in the designs is aACATGAGGATCACCCATGTc (SEQ ID NO:177).
**FIG. 5** shows percent mRNA editing by a construct comprising an Alu domain in comparison to editing by a construct comprising a GluR2 domain.
**FIG. 6** shows an example of a construct (SEQ ID NO: 10) comprising an Alu domain that was used to generate the data of **FIG. 5****.**
**FIG. 7** shows an example of a construct comprising two cruciforms linked by an antisense domain and comprising an Alu domain. Both exemplary structure (SEQ ID NO:11) and exemplary sequence (SEQ ID NO:1) are provided.
**FIG. 8** shows various construct designs (including those that comprise a GluR2 domain)(SEQ ID NOs: 12-34) and percent mRNA editing of each.

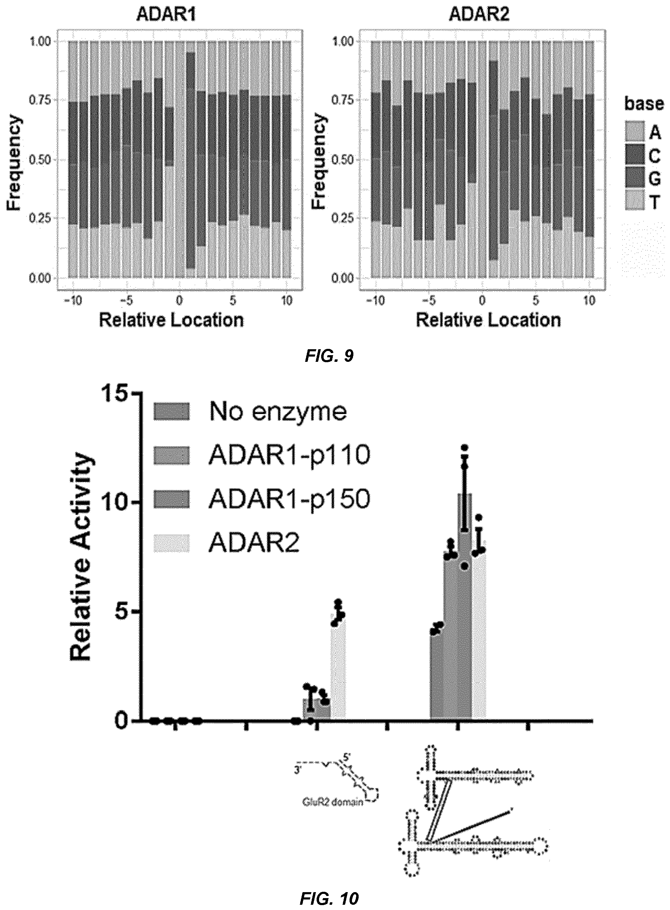

**FIG. 9** shows mapping enzyme targeting preferences for both ADAR1 and ADAR2, for each base (*e.g*., A, C, G, and T).
**FIG. 10** shows engineering next-gen adRNAs with enhanced ADAR1 and ADAR2 recruitment potential. The first series of columns show relative activity employing no adRNA. The second series of columns show relative activity employing a construct comprising a GluR2 domain. The third series of columns show relative activity employing a construct comprising an Alu domain associated with two cruciform structures.

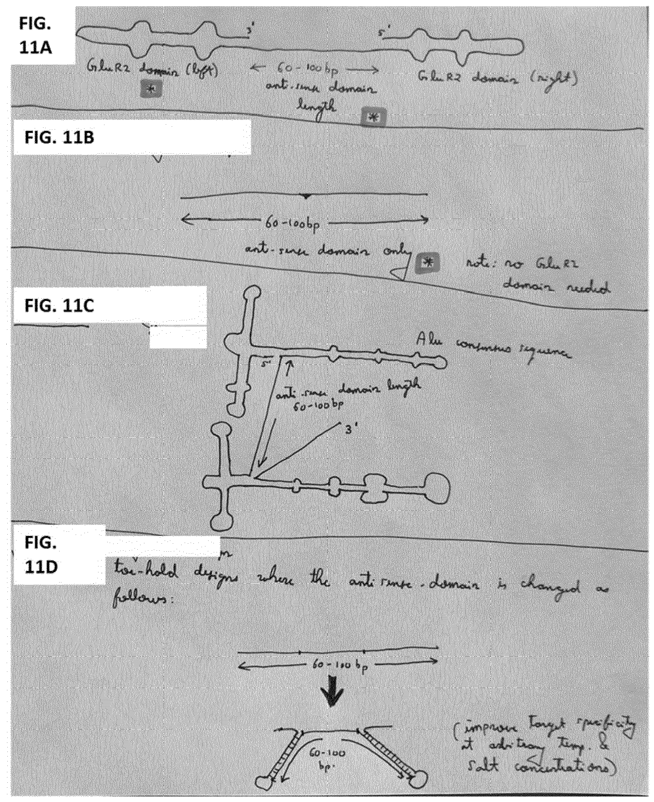

**FIG. 11A-D** shows different construct designs. **FIG. 11A** exemplifies an antisense domain linked to two GluR2 domains. **FIG. 11B** exemplifies an anti-sense domain only. **FIG. 11C** exemplifies an antisense domain linked to two cruciforms. **FIG. 11D** exemplifies a toe-hold

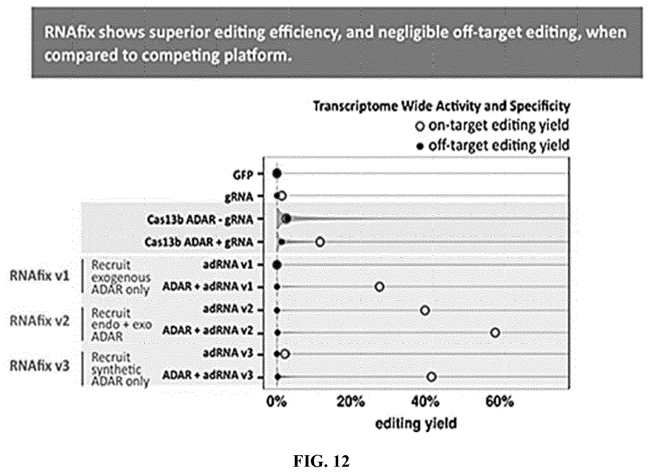

**FIG. 12** shows percent editing yield between different construct designs.
**FIG. 13** shows a comparison of a short antisense oligonucleotide (AON) with mismatched bulges as compared to a longer construct comprising a hairpin structure.
**FIG. 14** shows exemplary adRNA designs (SEQ ID NOs:250 and 251).
**FIG. 15** shows exemplary adRNA structures (SEQ ID NOs: 35-37) having parameters (d, l, m), wherein d = number of GluR2 domains, 1 = length of antisense domain, m = position of mismatch.
**FIG. 16** shows a schematic of RNA editing via recruitment of endogenous ADARs in the presence of adRNA.
**FIG. 17** shows a U6 promoter transcribed adRNAs with progressively longer antisense domain lengths, in combination with zero, one or two GluR2 domains that were evaluated for their ability to induce targeted RNA editing with or without exogenous ADAR2 expression. Values represent mean +/-SEM (n=3). Long adRNA can recruit endogenous ADARs for RNA editing.
**FIG. 18** shows chemically synthesized adRNAs versions tested against a panel of mRNAs with or without exogenous ADAR2 expression. Chemical modifications are identified along with the source of adRNA. Values represent mean +/- SEM (n=3).
**FIG. 19** shows *in vivo* RNA correction efficiencies in correctly spliced OTC mRNA in the livers of treated adult spf^{ash} mice (reto-orbital injections). RNA editing levels of 0.6% are seen in mice injected with U6 transcribed short adRNA.
**FIG. 20** shows a design of an Alu adRNA. Left: a structure of an Alu element. Middle: a design as described herein that comprises a locus-specific antisense sequence with a C mismatch opposite a target A. Right: Recruitment of an RNA editing enzyme ADAR to the target.
**FIG. 21** shows exemplary Alu guide sequences (SEQ ID NOs:38-41).
**FIG. 22** shows a schematic of the split-ADAR2 DD system.
**FIG. 23** shows an exemplary sequence (SEQ ID NO:42) of the split-ADAR2 DD with potential sites for splitting highlighted.
**FIG. 24** shows pairs of fragments 1-16 assayed via a cypridina luciferase reporter (Clue W85X).
**FIG. 25** shows fragments 9 and 10 assayed against a Clue reporter.
**FIG. 26** shows exemplary sequences (SEQ ID NOs:43-55).
**FIG. 27** shows a schematic of ADAR recruitment via U1A (SEQ ID NO:56).
**FIG. 28** shows exemplary sequences (SEQ ID NOs:57-68) for several fusion constructs or one or more APOBEC family members (SEQ ID NOs:57-68).
**FIG. 29** shows exemplary sequences (SEQ ID NOs:69-73) of engineered apRNAs configured to recruit APOBEC3A.
**FIG. 30** shows exemplary sequences (SEQ ID NOs:74-78) of engineered MS2-apRNAs configured to recruit MCP-APOBEC3A.
**FIG. 31** shows two different scenarios of no ADAR recruitment and ADAR recruitment to permit ribosomal read-through that results in normal luciferase expression.

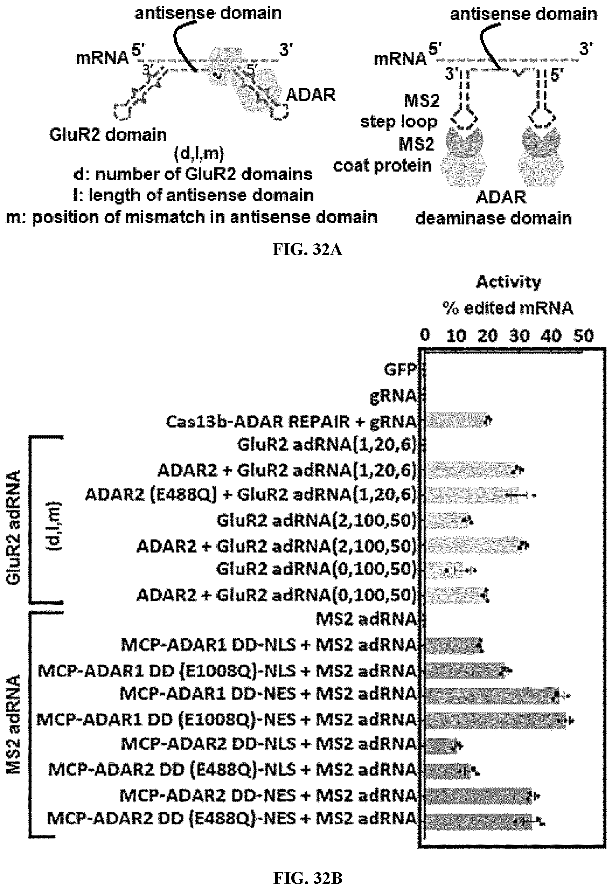

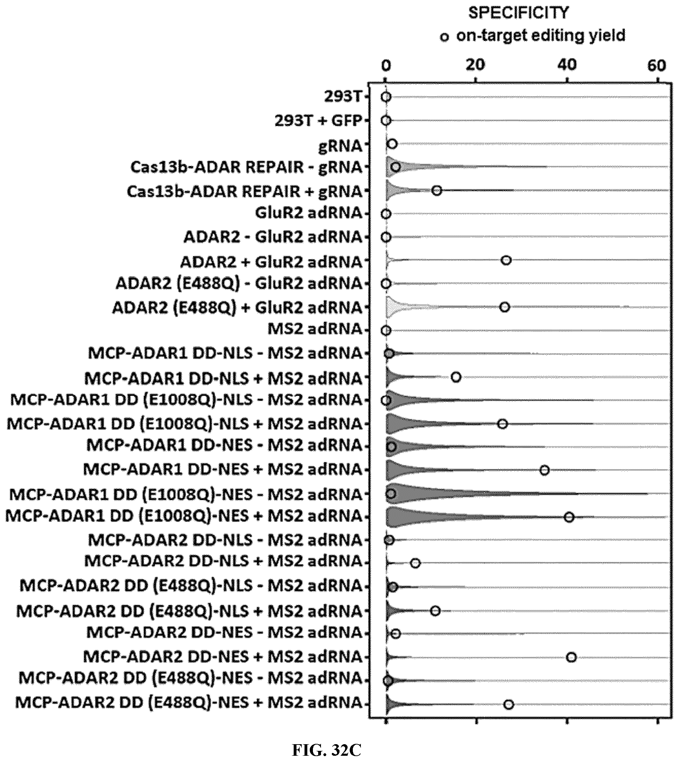

**FIG. 32A-C** shows engineering programmable RNA editing and characterizing specificity profiles: **(****FIG. 32A****)** Schematics of RNA editing via constructs utilizing the full length ADAR2 and an engineered adRNA derived from the GluR2 transcript, or MS2 Coat Protein (MCP) fusions to the ADAR1/2 deaminase domains and the corresponding MS2 hairpin bearing adRNA. **(****FIG. 32B****)** Comparison of RNA editing efficiency of the endogenous RAB7A transcript by different RNA editing constructs quantified by Sanger sequencing (efficiency calculated as a ratio of Sanger peak heights G/(A+G)). Experiments were carried out in HEK 293T cells. Values represent mean +/- SEM (n=3). **(****FIG. 32C****)** Violin plots representing distributions of A->G editing yields observed at reference sites where at least one treatment sample was found to have a significant change (Fisher's exact test, FDR = 1%) in editing yield relative to the control sample. Blue circles indicate editing yields at the target A-site within the RAB7A transcript. Black dots represent median off-target editing yields. To better visualize the shapes of the distributions, their maximum extent along the y-axis was equalized across all plots, and were truncated at 60% yield..

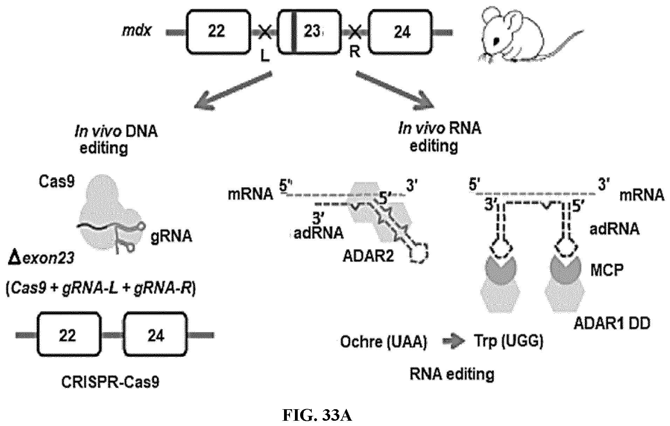

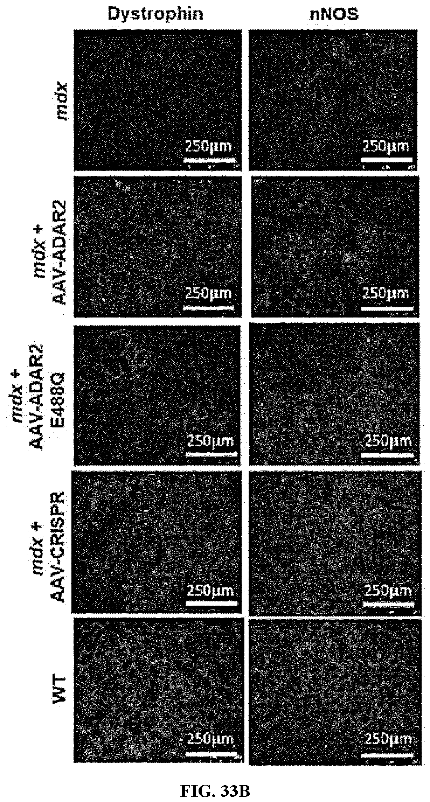

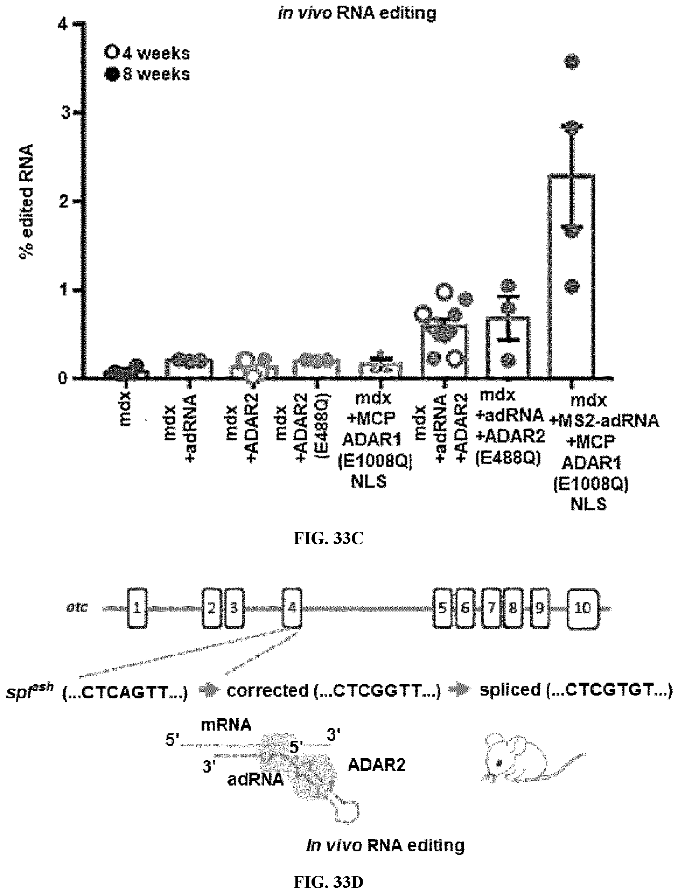

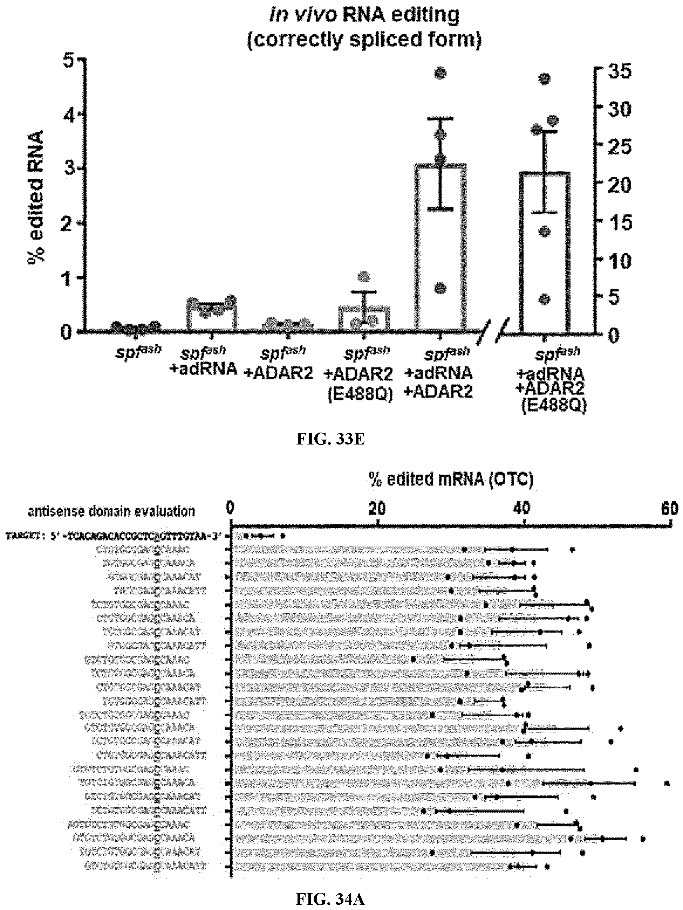

**FIG. 33A-E** shows *in vivo* RNA editing in mouse models of human disease. **(****FIG. 33A****)** Schematic of the DNA and RNA targeting approaches to restore dystrophin expression in the *mdx* mouse model of Duchenne Muscular Dystrophy: (i) a dual gRNA-CRISPR based approach leading to in frame excision of exon 23 and (ii) ADAR2 and MCP-ADAR1 based editing of the ochre codon. **(****FIG. 33B****)** Immunofluorescence staining for dystrophin in the TA muscle shows partial restoration of expression in treated samples (intra-muscular injections of AAV8-ADAR2, AAV8-ADAR2 (E488Q), and AAV8-CRISPR). Partial restoration of nNOS localization is also seen in treated samples (scale bar: 250µm). **(****FIG. 33C****)** *In vivo* TAA->TGG/TAG/TGA RNA editing efficiencies in corresponding treated adult *mdx* mice. Values represent mean +/- SEM (n=4, 3, 7, 3, 3, 10, 3, 4 independent TA muscles respectively). **(****FIG. 33D****)** Schematic of the OTC locus in the *spf^{ash}* mouse model of Ornithine Transcarbamylase deficiency which have a G->A point mutation at a donor splice site in the last nucleotide of exon 4, and approach for correction of mutant OTC mRNA via ADAR2 mediated RNA editing. **(****FIG. 33E****)** *In vivo* RNA correction efficiencies in the correctly spliced OTC mRNA in the livers of treated adult *spf^{ash}* mice (retro-orbital injections of AAV8-ADAR2 and AAV8-ADAR2 (E488Q)). Values represent mean +/- SEM (n=4, 4, 3, 3, 4, 5 independent animals respectively).

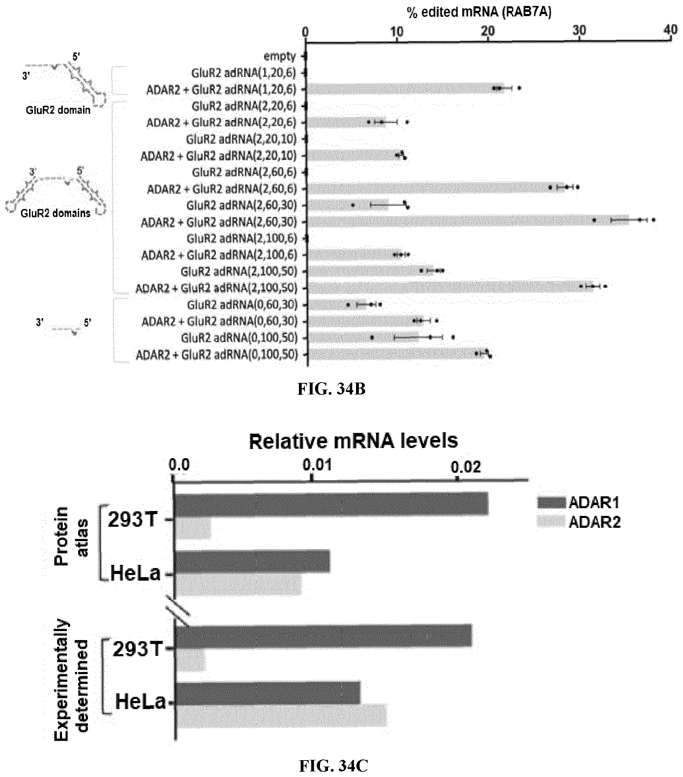

**FIG. 34A-C** shows antisense domain engineering. **(****FIG. 34A****)** Optimization of adRNA antisense region using adRNA scaffold 2: length and distance from the ADAR2 recruiting region were systematically varied. Values represent mean +/- SEM (n=3)(SEQ ID NO:79-102). **(****FIG. 34B****)** U6 promoter transcribed adRNAs with progressively longer antisense domain lengths, in combination with zero, one or two GluR2 domains were evaluated for their ability to induce targeted RNA editing with or without exogenous ADAR2 expression. Values represent mean +/- SEM (n=3). All the above experiments were carried out in HEK 293T cells. **(****FIG. 34C****)** Experimental confirmation of expression of endogenous ADAR1 and ADAR2 (relative to GAPDH) in HEK 293T and HeLa cell lines. Observed levels were similar to those documented in The Human Protein Atlas (see world wide web (www) at proteinatlas.org).
**FIG. 35A-B** shows engineering MS2 adRNAs. (**FIG. 35A**) Systematic evaluation of antisense RNA targeting domain of the MS2 adRNA (SEQ ID NO:103-110). Values represent mean +/- SEM (n=3). (**FIG. 35B**) On-target RNA editing by MCP-ADAR2 DD-NLS requires co-expression of the MS2 adRNA. Values represent mean +/- SEM (n=3). All experiments were carried out in HEK 293T cells.

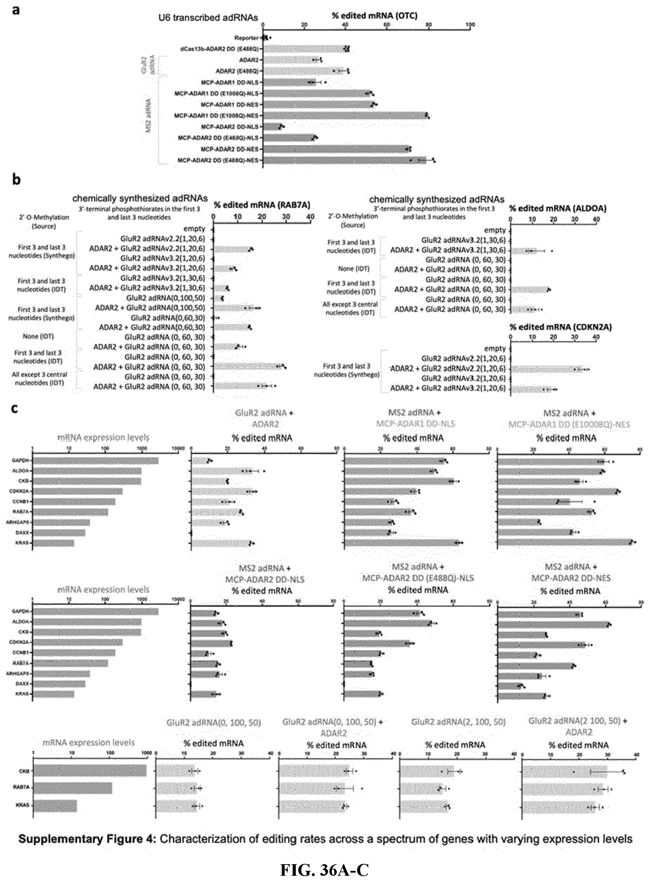

**FIG. 36A-C** shows analysis of RNA editing yields across a panel of targets. (**FIG. 36A**) Comparison of RNA editing efficiency of the OTC reporter transcript by GluR2 adRNA and MS2 adRNA guided RNA editing constructs as well as the Cas13b based REPAIR construct. Values represent mean +/- SEM (n=6 for reporter and Cas13b based constructs, n=3 for all other constructs). (**FIG. 36B**) Chemically synthesized adRNAs versions were tested against a panel of mRNAs with or without exogenous ADAR2 expression. The exact chemical modifications are stated in the figure along with the source of adRNA. Values represent mean +/- SEM (n=3). (**FIG. 36C**) Analysis of RNA editing yields across a spectrum of endogenous targets chosen to cover a range of expression levels. U6 transcribed long adRNAs with none or two GluR2 domains were also evaluated against multiple endogenous mRNA targets with or without exogenous ADAR2 expression. Editing is observed at all tested loci even in the absence of exogenous ADAR2 expression. Values represent mean +/- SEM (n=3). All experiments were carried out in HEK 293T cells.

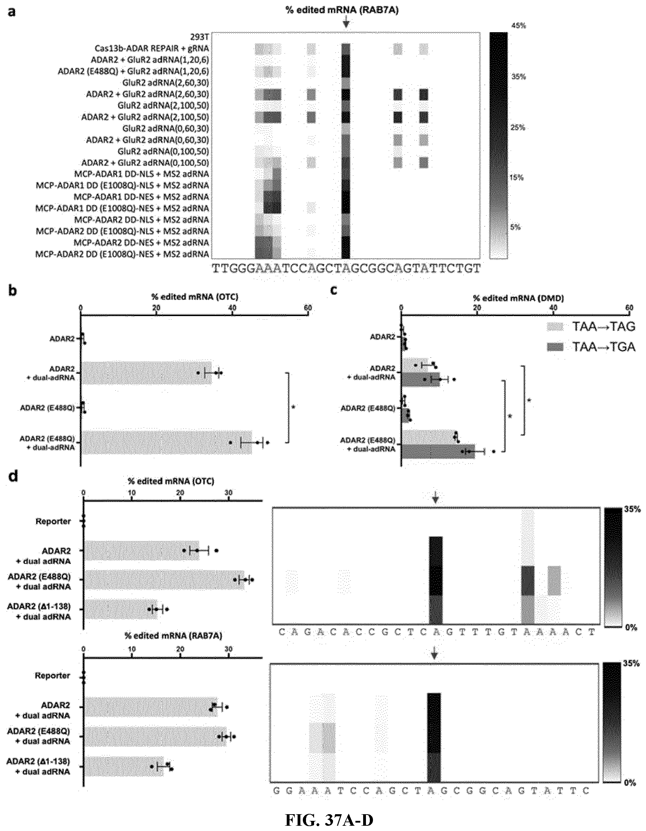

**FIG. 37A-D** shows ADAR2 variants and their impact on editing and specificity. (**FIG. 37A**) Comparison of on target RNA editing and editing in flanking adenosines of the RAB7A transcript by GluR2 adRNA and MS2 adRNA guided RNA editing constructs as well as the Cas13b based REPAIR construct. Mean (n=3) editing yields are depicted (SEQ ID NO:111). All experiments were carried out in in HEK 293T cells and editing efficiency was calculated as a ratio of Sanger peak heights G/(A+G). (**FIG. 37B**) ADAR2 (E488Q) exhibits higher efficiency than the ADAR2 in the *in vitro* editing of the spfash OTC reporter transcript (p=0.037, unpaired t-test, two-tailed); values represent mean +/- SEM (n=3), and (**FIG. 37C**) mdx DMD reporter transcript (p=0.048, p=0.012 respectively, unpaired t-test, two-tailed); values represent mean +/- SEM (n=3). (**FIG. 37D**) Comparison of the editing efficiency and specificity profiles of the ADAR2, ADAR2 (E488Q) and the ADAR2 (Δ1-138) for the OTC reporter transcript (upper panel) and endogenous RAB7A transcript (lower panel). Heatmap indicates the A->G edits in the vicinity of the target (arrow). Values represent mean +/- SEM (n=3). All experiments were carried out in HEK 293T cells and editing efficiency was calculated as a ratio of Sanger peak heights G/(A+G).
**FIG. 38** shows transcriptome scale specificity profiles of RNA editing approaches (Cas13b-ADAR REPAIR +/- gRNA).
**FIG. 39** shows transcriptome scale specificity profiles of RNA editing approaches (ADAR2 +/- adRNA). The version used for these studies is GluR2 adRNA (1,20,6).
**FIG. 40** shows transcriptome scale specificity profiles of RNA editing approaches (MCP-ADAR1 DD +/- adRNA).
**FIG. 41** shows transcriptome scale specificity profiles of RNA editing approaches (MCP-ADAR2 DD +/- adRNA).

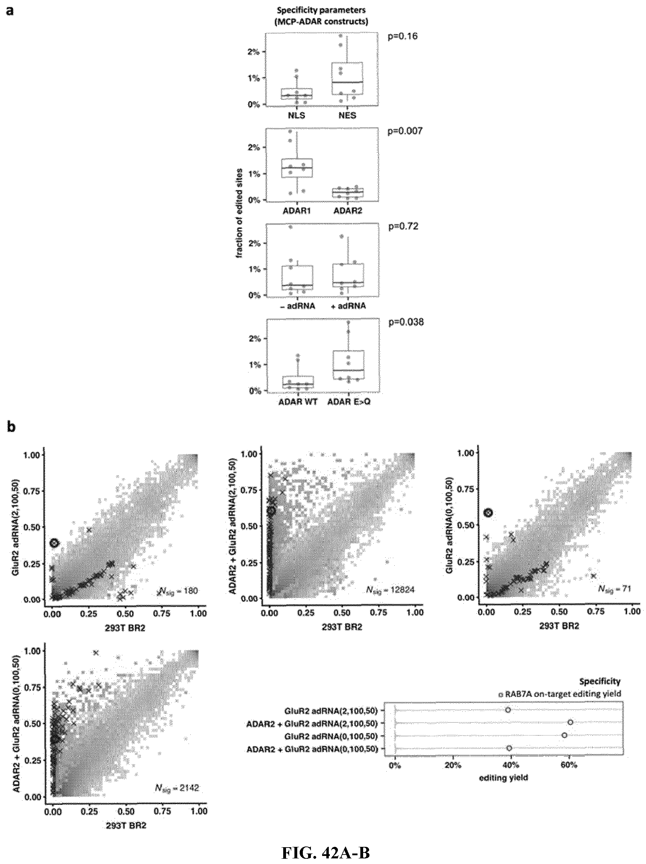

**FIG. 42A-B** shows variation of transcriptome scale editing specificity with construct features. (**FIG. 42A**) Each point in the box plots corresponds to the fraction of edited sites for one of the MCP-ADAR constructs listed in **FIG. 32****.** The fraction of edited sites for each construct was calculated by dividing the number of reference sites with significant changes in A-to-G editing yield (see Table 3) by the total number 8,729,464 of reference sites considered. Construct features indicated on the horizontal axes were compared using the Mann-Whitney U test, yielding p-values of 0.16 for NLS vs. NES, 0.0070 for ADAR1 vs. ADAR2, 0.72 for "- adRNA" vs. "+ adRNA", and 0.038 for "ADAR WT" vs. "ADAR E>Q" (n=8 for all conditions). (**FIG. 42B**) 2D histograms comparing the transcriptome-wide A->G editing yields observed with each construct (y-axis) to the yields observed with the control sample (x-axis). Inset shows violin plots representing distributions of A->G editing yields observed at reference sites where at least one treatment sample was found to have a significant change (Fisher's exact test, FDR = 1%) in editing yield relative to the control sample. Blue circles indicate editing yields at the target A-site within the RAB7A transcript. To better visualize the shapes of the distributions, their maximum extent along the y-axis was equalized across all plots, and were truncated at 60% yield. Samples here correspond to 293Ts transfected with long antisense domain bearing adRNAs that can enable RNA editing via exogenous and/or endogenous ADAR recruitment.

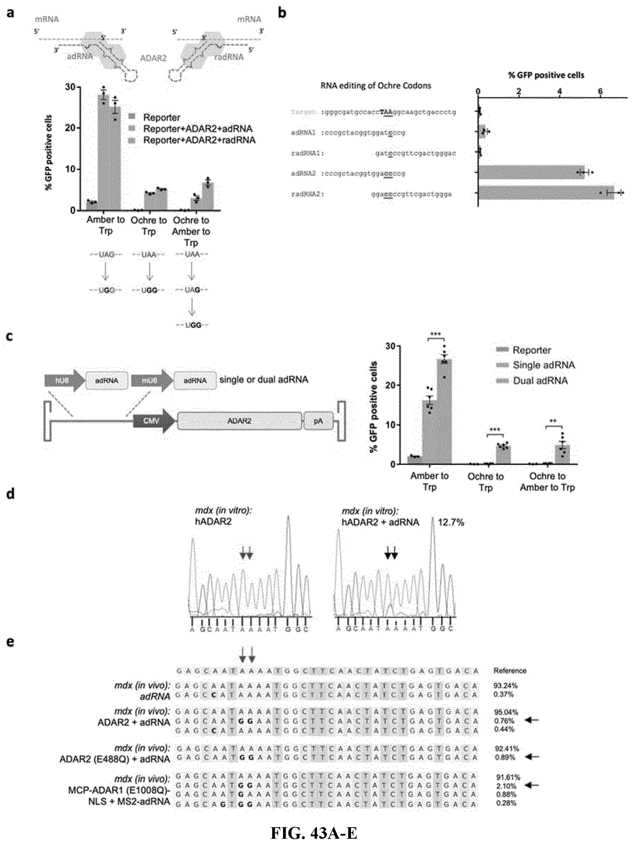

**FIG. 43A-E** shows optimization and evaluation of dystrophin editing experiments *in vitro* and *in vivo* in *mdx* mice. (**FIG. 43A**) Schematic of RNA editing utilizing the full length ADAR2 along with an engineered adRNA or a reverse oriented adRNA (radRNA); (ii) RNA editing efficiencies of amber and ochre stop codons, in one-step and two-steps. Experiments were carried out in HEK 293T cells. Values represent mean +/- SEM (n=3). (**FIG. 43B**) RNA editing of ochre codons requires two cytosine mismatches in the antisense RNA targeting domains of adRNA or radRNA (SEQ ID NOs:112-116) to restore GFP expression. Experiments were carried out in HEK 293T cells. Values represent mean +/- SEM (n=3). (**FIG. 43C**) Schematic of the AAV vectors utilized for *in vivo* delivery of adRNA and ADAR2, and *in vitro* optimization of RNA editing of amber and ochre stop codons in the presence of one or two copies of the adRNA, delivered via an AAV vector (p=0.0003, p=0.0001, p=0.0015 respectively, unpaired t-test, two-tailed). Experiments were carried out in HEK 293T cells. Values represent mean +/- SEM (n=3 for reporters, n=6 for all other conditions). (**FIG. 43D**) Representative Sanger sequencing plot showing editing of the ochre stop codon (TAA->TGG) in the mdx DMD reporter transcript (quantified by NGS)(SEQ ID NO:117-118). Experiments were carried out in HEK 293T cells (n=3). (**FIG. 43E**) Representative example of *in vivo* RNA editing analyses of treated mdx mice (quantified using NGS) (SEQ ID NOs:119-130).

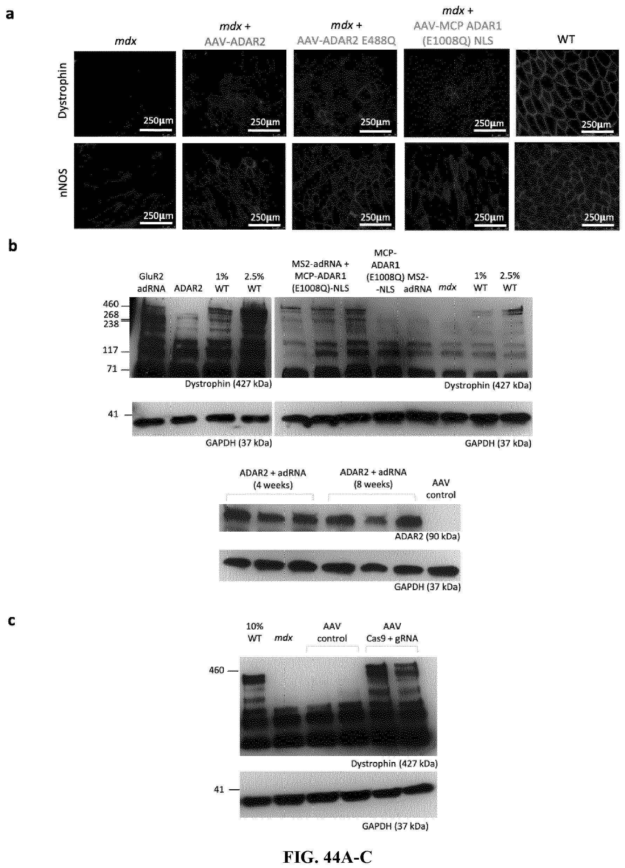

**FIG. 44A-C** shows immunofluorescence and western blot analyses of *in vivo* dystrophin RNA editing experiments in *mdx* mice. (**FIG. 44A**) Immunofluorescence staining for dystrophin in the TA muscle shows partial restoration of expression in treated samples (intra-muscular injections of AAV8-ADAR2, AAV8-ADAR2 (E488Q), AAV8-MCP-ADAR1 (E1008Q) NLS). Partial restoration of nNOS is localization also seen in treated samples (scale bar: 250µm). (**FIG. 44B**) Western blots showing partial recovery of dystrophin expression (1-2.5%) in TA muscles of mdx mice injected with both components of the editing machinery, the enzyme and adRNA, and stable ADAR2 expression in injected TA muscles up to 8 weeks post injections. (**FIG. 44C**) Western blot showing partial restoration of dystrophin expression (10%) using AAV8-CRISPR.
**FIG. 45A-E**
shows optimization and evaluation of OTC RNA editing experiments *in vitro* and *in vivo* in spf^{ash} mice. (**FIG. 45A**) Representative Sanger sequencing plot showing correction of the point mutation in the spfash OTC reporter transcript (quantified using NGS) (SEQ ID NO:131-132). Experiments were carried out in HEK 293T cells (n=3). (**FIG. 45B**) Representative example of *in vivo* RNA editing analyses of treated spfash mice showing correction of the point mutation in the correctly spliced OTC mRNA (quantified using NGS)(SEQ ID NO:133-139). (**FIG. 45C**) *In vivo* RNA correction efficiencies in the OTC pre-mRNA in the livers of treated adult spfash mice (retro-orbital injections of AAV8-ADAR2 and AAV8-ADAR2 (E488Q). Values represent mean +/- SEM (n=4, 4, 3, 3, 4, 5 independent animals respectively). (**FIG. 45D**) PCR products showing the correctly and incorrectly spliced OTC mRNA. The incorrectly spliced mRNA is elongated by 48 base pairs. Fraction of incorrectly spliced mRNA is reduced in mice treated with adRNA+ADAR2 (E488Q). **(****FIG. 45E****)** Western blot for OTC shows partial restoration (2.5%-5%) of expression in treated adult spfash mice and stable ADAR2 (E488Q) expression three weeks post injections.

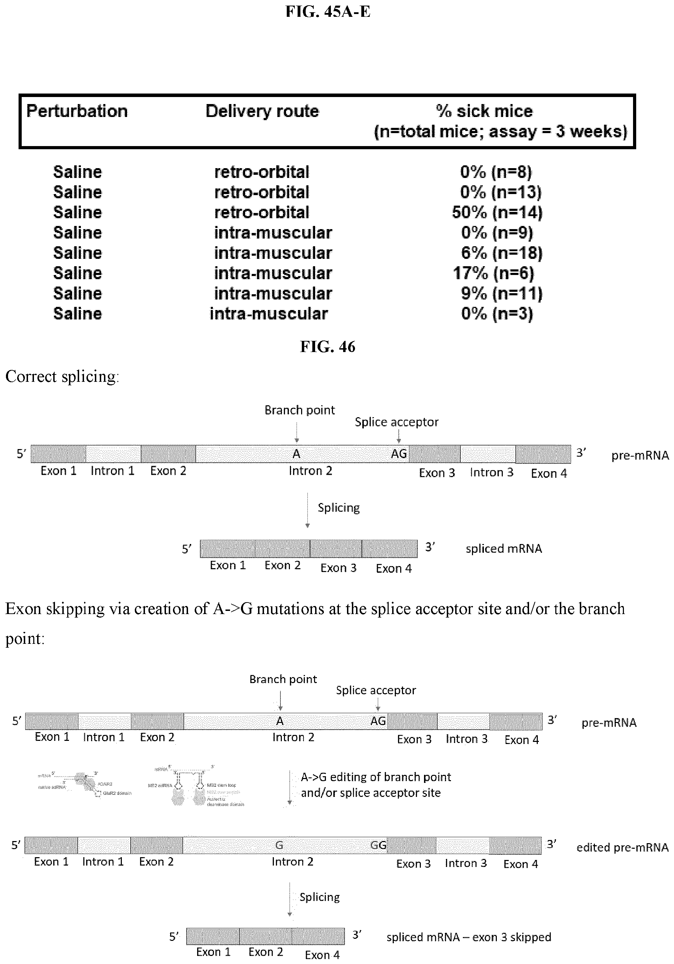

**FIG. 46** shows toxicity analyses of *in vivo* RNA editing experiments.
**FIG. 47** is a schematic showing exon skipping via creation of a splice acceptor and/or branch point mutation.

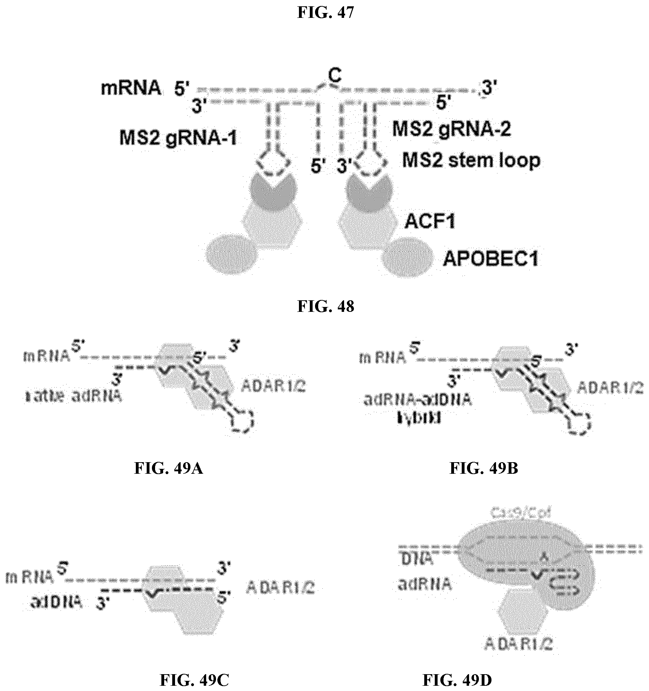

**FIG. 48** is a schematic of C → T editing via APOBECs.
**FIG. 49A-D** shows schematics of editing DNA and both strands of DNA/RNA hybrids.

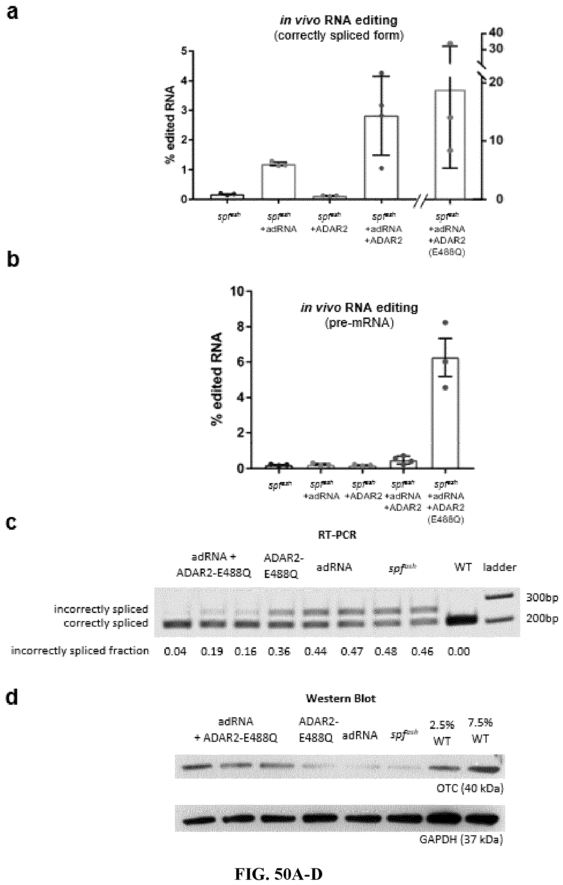

**FIG. 50A-D** shows the results of a study in a model for ornithine transcarbamylase deficiency. **FIG. 50A** depicts *in vivo* RNA correction efficiencies in the livers of treated adult spf^{ash} mice (retro-orbital injections of AAV8-ADAR2 and AAV8-ADAR2 (E488Q)). Each data point represents an independent animal. Editing efficiencies measured in the spliced OTC mRNA. Error bars represent +/- SEM. **FIG. 50B** depicts *in vivo* RNA correction efficiencies in the OTC pre-mRNA in the livers of treated adult spf^{ash} mice (retro-orbital injections of AAV8-ADAR2 and AAV8-ADAR2 (E488Q). Each data point represents an independent animal. **FIG. 50C** shows PCR products showing the correctly and incorrectly spliced OTC mRNA. The incorrectly spliced mRNA is elongated by 48 base pairs. The fraction of incorrectly spliced mRNA is reduced in mice treated with adRNA+ADAR2 (E488Q). **Fig. 50D** is a Western blot for *OTC* shows partial restoration (2.5%-5%) of expression in treated adult spf^{ash} mice.

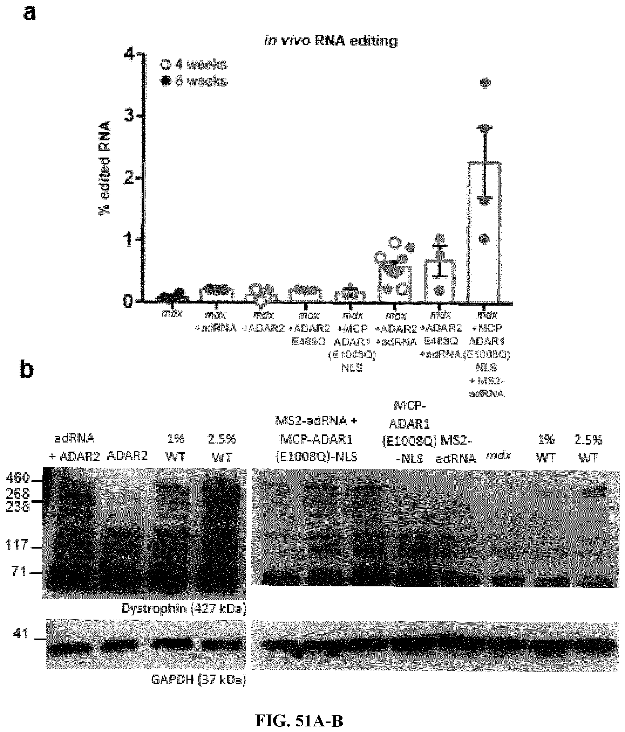

**FIG. 51A-B** shows the results of a study in a model of Duchenne muscular dystrophy. **FIG. 51A** depicts *in vivo* TAA->TGG/TAG/TGA RNA editing efficiencies in corresponding treated adult *mdx* mice. Each data point represents an independent TA muscle. Error bars represent +/- SEM. **FIG. 51B** is a Western blot for dystrophin shows partial restoration (1-2.5%) of expression in corresponding treated adult *mdx* mice.

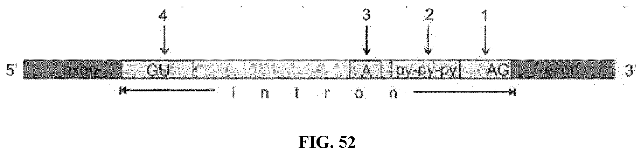

**FIG. 52** provides further information about potential branch point locations.

### DETAILED DESCRIPTION

As aspect of the disclosure provides for nucleic acids, non-naturally occurring RNAs, vectors comprising nucleic acids, compositions, and pharmaceutical compositions for RNA editing. Any of the above or as described herein can be configured for an A (adenosine) to I (inosine) edit, a C (cytosine) to T (thymine) edit, or a combination thereof. In some cases, an A to I edit can be interpreted or read as a C to U mutation. In some cases, an A to I edit can be interpreted or read as an A to G mutation. Nucleic acids, non-naturally occurring RNAs, vectors comprising nucleic acids, compositions, and pharmaceutical compositions as described herein can provide enhanced editing efficiencies as compared to native systems, reduced off-target editing, enhanced stability or *in vivo* half-lives, or any combination thereof.

An aspect of the disclosure provides for a vector. The vector can comprise a nucleic acid with a polynucleotide sequence encoding (i) an RNA editing entity recruiting domain, (ii) a targeting domain complementary to at least a portion of a target RNA, (iii) more than one of either domain, or (iv) any combination thereof. In some cases, the vector can be administered to a subject, such as a subject in need thereof. In some cases, the vector can be administered as part of a pharmaceutical composition to a subject, such as a subject in need thereof.

An aspect of the disclosure provides for a non-naturally occurring RNA. The non-naturally occurring RNA can comprise (i) an RNA editing entity recruiting domain, (ii) a targeting domain complementary to at least a portion of a target RNA, (iii) more than one of either domain, or (iv) any combination thereof. In some cases, the non-naturally occurring RNA can be administered to a subject, such as a subject in need thereof. In some cases, the non-naturally occurring RNA can be administered as part of a pharmaceutical composition to a subject, such as a subject in need thereof. In some cases, the non-naturally occurring RNA can be formulated in a vector for administration. The vector can comprise a viral vector, a liposome, a nanoparticle, or any combination thereof. In some cases, the non-naturally occurring RNA can comprise at least one base, at least one sugar, more than one of either, or a combination thereof having a modification, such as a chemical modification.

An aspect of the disclosure provides for a nucleic acid. The nucleic acid can comprise (i) an RNA editing entity recruiting domain, (ii) a targeting domain complementary to at least a portion of a target RNA, (iii) more than one of either domain, or (iv) any combination thereof. In some cases, the nucleic can be administered to a subject, such as a subject in need thereof. In some cases, the nucleic acid can be administered as part of a pharmaceutical composition to a subject, such as a subject in need thereof. In some cases, the nucleic acid can be formulated in a vector for administration. The vector can comprise a viral vector, a liposome, a nanoparticle, or any combination thereof. The nucleic acid can be genetically encoded. The nucleic acid can be chemically synthesized.

A nucleic acid can comprise one or more domains, such as 1, 2, 3, 4, 5 or more domains. In some cases, a nucleic acid can comprise a recruiting domain, a targeting domain, more than one of either, or a combination thereof. In some cases, a nucleic acid can comprise a targeting domain and a recruiting domain. In some cases, a nucleic acid can comprise a targeting domain and two recruiting domains.

A domain can form a two dimensional shape or secondary structure. For example, a targeting domain, a recruiting domain or a combination thereof can form a secondary structure that can comprise a linear region, a cruciform or portion thereof, a toe hold, a stem loop, or any combination thereof. The domain itself can form a substantially linear two dimensional structure. The domain can form a secondary structure that can comprise a cruciform. The domain can form a secondary structure that can comprise a stem loop. The domain can form a secondary structure that can comprise a toehold.

In some cases, a targeting domain can be positioned adjacent to a recruiting domain, including immediately adjacent or adjacent to but separated by a number of nucleotides. In some cases, a targeting domain can be flanked by two recruiting domains. In some cases, two or more recruiting domains can be adjacent one another.

An aspect of the disclosure includes reducing off target editing. One approach as described herein includes restricting catalytic activity of an ADAR or APOBEC by a split reassembly approach. In such a design, a first domain (such as a recruiting domain) can be catalytically inactive by itself and a second domain can be catalytically inactive by itself but when brought together in a reassembly the two domains together provide catalytic activity to recruit an ADAR or APOBEC. A nucleic acid comprising two domains can be split at any number of locations, such as a location between the two domains. In some cases, a first domain or second domain can comprise an MS2 stem loop, a BoxB stem-loop, a U1A stem-loop, a modified version of any of these, or any combination thereof.

Two dimensional shape or secondary structure of a domain can influence efficiency of editing, off target effects, or a combination thereof as compared to a nucleic acid that can form a different two dimension shape or secondary structure. Therefore, an aspect of the disclosure includes modifying nucleic acids such that two dimensional shapes can be advantageously designed to enhance efficiency of editing and reduce off target effects. Modifications to a sequence comprising a naturally occurring recruiting domains can also enhance editing efficiency and reduce off target effects. Therefore, an aspect of the disclosure includes modifying nucleic acids such that a sequence (such as a synthetic sequence) can be advantageously designed to enhance efficiency of editing and reduce off target effects. Modifications can include altering a length of a domain (such as extending a length), altering a native sequence that results in a change in secondary structure, adding a chemical modification, or any combination thereof. Nucleic acids as described herein can provide these advantages.

In some cases, a nucleic acid as described herein can modify at least one base pair of a target nucleic acid at an efficiency of at least about: 3, 4, or 5 times greater than a comparable nucleic acid complexed with a native recruiting domain and an antisense domain (complementary to the target nucleic acid). In some cases, a nucleic acid as described herein can modify at least one base pair of a target nucleic acid at an efficiency of at least about: 3, 4, or 5 times greater than a comparable nucleic acid complex with a GluR2 domain and an antisense domain (complementary to the target nucleic acid). In some cases, a nucleic acid as described herein can modify at least one base pair of a target nucleic acid at an efficiency of at least about: 3, 4, or 5 times greater than a comparable nucleic acid complex with a Cas13b protein or active fragment thereof and an antisense domain (complementary to the target nucleic acid.) An improvement in efficiency can be measured by a sequencing method, such as Sanger Method.

An aspect of the disclosure provides for a vector. The vector can comprise a nucleic acid with a polynucleotide sequence encoding for at least one RNA editing entity recruiting domain. In some cases, the polynucleotide sequence may not form a secondary structure comprising a stem-loop. In some cases, the polynucleotide sequence can form one or more stem-loops. In some cases, the polynucleotide sequence can form a secondary structure comprising a cruciform. In some cases, the polynucleotide sequence can form a secondary structure that can be substantially linear. In some cases, the polynucleotide sequence can comprise at least about 80% sequence identity to one or more sequences comprising: an Alu domain encoding sequence, an Apolipoprotein B mRNA Editing Catalytic Polypeptide-like (APOBEC) recruiting domain encoding sequence, and any combination thereof. In some cases, the nucleic acid can be genetically encoded. In some cases, the nucleic acid can be chemically synthesized.

In some cases, a polynucleotide sequence can comprise at least about 80% sequence identity to an Alu domain encoding sequence. In some cases, a polynucleotide sequence can comprise at least about 85% sequence identity to an Alu domain encoding sequence. In some cases, a polynucleotide sequence can comprise at least about 90% sequence identity to an Alu domain encoding sequence. In some cases, a polynucleotide sequence can comprise at least about 95% sequence identity to an Alu domain encoding sequence. In some cases, the Alu domain encoding sequence can be a non-naturally occurring sequence. In some cases, the Alu domain encoding sequence can comprise a modified portion. In some cases, the Alu domain encoding sequence can comprise a portion of a naturally occurring Alu domain sequence.

In some cases, a polynucleotide sequence can comprise at least about 80% sequence identity to an APOBEC domain encoding sequence. In some cases, a polynucleotide sequence can comprise at least about 85% sequence identity to an APOBEC domain encoding sequence. In some cases, a polynucleotide sequence can comprise at least about 90% sequence identity to an APOBEC domain encoding sequence. In some cases, a polynucleotide sequence can comprise at least about 95% sequence identity to an APOBEC domain encoding sequence. In some cases, the APOBEC domain encoding sequence can be a non-naturally occurring sequence. In some cases, the APOBEC domain encoding sequence can comprise a modified portion. In some cases, the APOBEC domain encoding sequence can comprise a portion of a naturally occurring APOBEC domain sequence.

In some cases, a polynucleotide sequence can comprise at least about 80% sequence identity to a GluR2 domain encoding sequence. In some cases, a polynucleotide sequence can comprise at least about 85% sequence identity to a GluR2 domain encoding sequence. In some cases, a polynucleotide sequence can comprise at least about 90% sequence identity to a GluR2 domain encoding sequence. In some cases, a polynucleotide sequence can comprise at least about 95% sequence identity to a GluR2 domain encoding sequence. In some cases, the GluR2 domain encoding sequence can be a non-naturally occurring sequence. In some cases, the GluR2 domain encoding sequence can comprise a modified portion. In some cases, the GluR2 domain encoding sequence can comprise a portion of a naturally occurring GluR2 domain sequence.

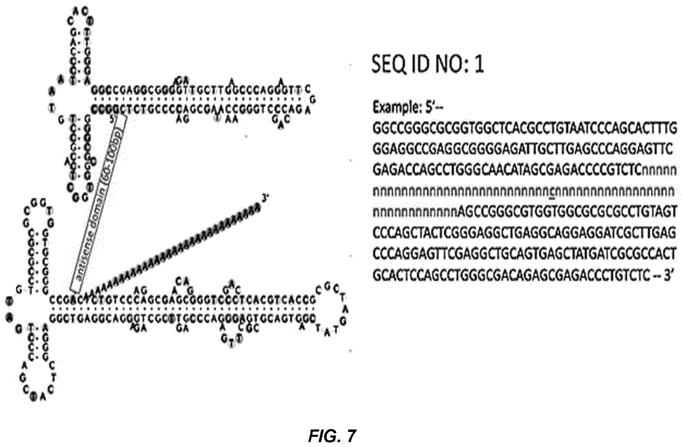

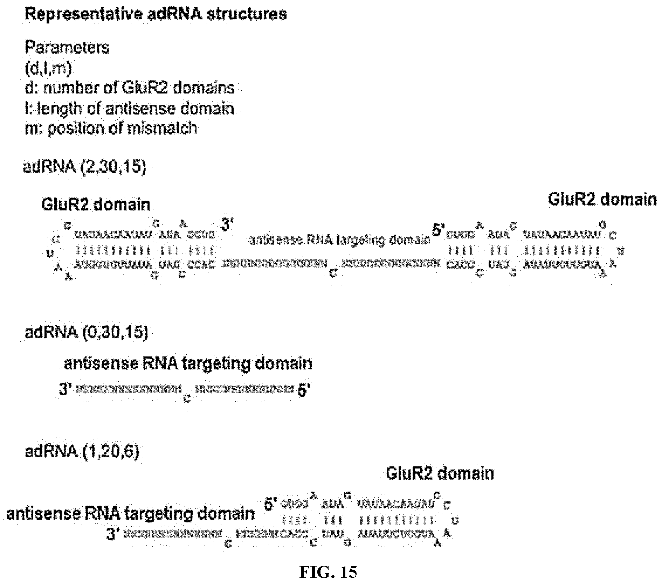

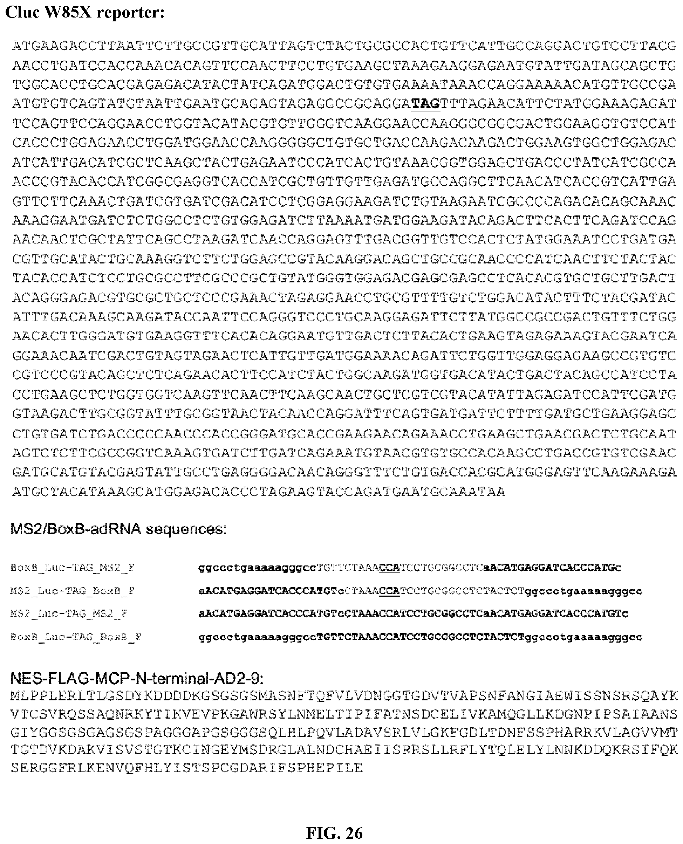

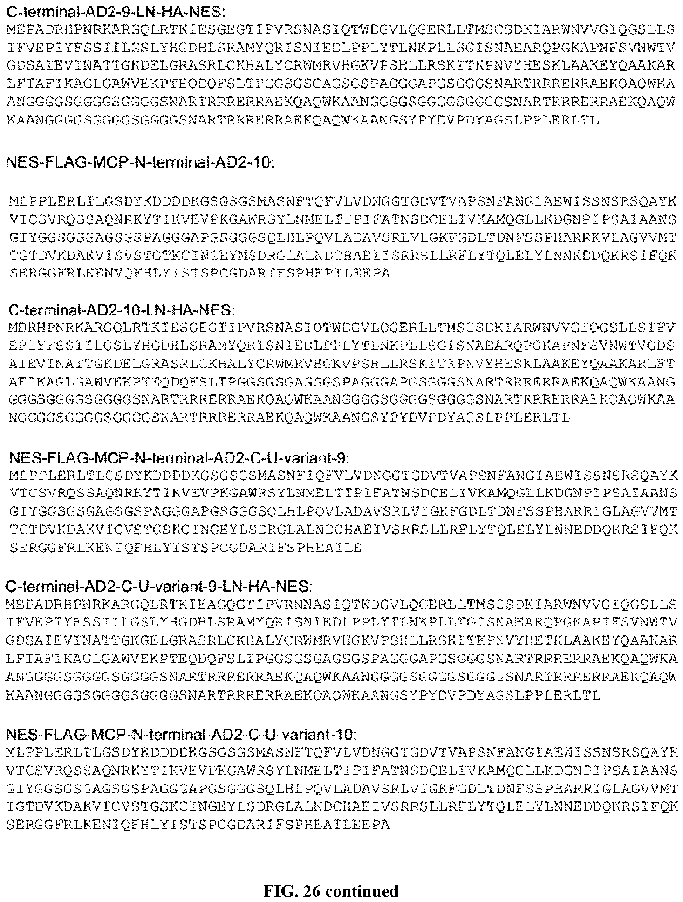

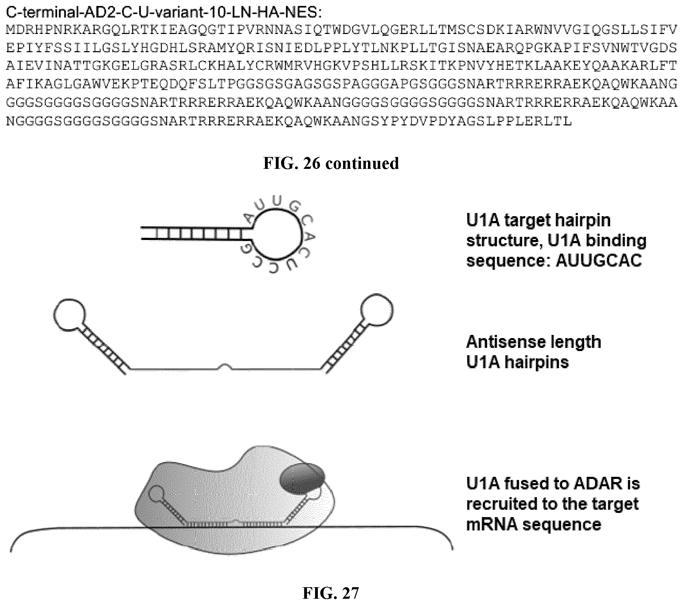

In some cases, a polynucleotide sequence can comprise at least about 80% sequence identify to an encoding sequence that recruits an ADAR. A polynucleotide sequence encoding for at least one RNA editing entity recruiting domain can be isolated and purified or can be synthesized. Such a polynucleotide sequence can be configured specifically to recruit an ADAR to a target site. The recruitment can include exogenous ADAR recruitment (that can be co delivered or separately delivered), endogenous ADAR recruitment, or a combination thereof. In some cases, a polynucleotide sequence can be configured specifically to enhance recruitment of ADAR or enhance specificity of ADAR recruitment to a particular site as compared to a naturally occurring recruiting domain. In some cases, the encoding sequence can be non-naturally occurring sequence. In some cases, the encoding sequence can comprise a modified portion. In some cases, the encoding sequence can comprise a portion of a naturally occurring ADAR recruiting domain sequence. Any sequence, either natural or synthetic, that recruits ADAR can be envisioned to be included in the polynucleotide sequence. In some cases, a polynucleotide sequence can comprise an exemplary sequence as described herein. **FIG. 2****,** **FIG. 6****,** **FIG. 7****,** **FIG. 8****,** **FIG. 14****,** **FIG. 15****,** **FIG. 21****,** **FIG. 26****,** **FIG. 28****,** **FIG. 29****,** **FIG. 30****,** and **Table 1** include exemplary sequences. The sequences provided herein include sequences having at least a portion that can encode for at least one RNA editing entity recruiting domain.

**Table 1: DNA encoding adRNA sequences. The adRNA sequence produced from these sequences is identical to the DNA sequence but T is replaced with U.:**

| | |
|---|---|
| Dual_ V2_R ab7a_ 20 6 | GTGGAATAGTATAACAATATGCTAAATGTTGTTATAGTATCCCACT GCCGCCAGCTGGATTTCCCAATTCTGAGTGTGGAATAGTATAACAAT ATGCTAAATGTTGTTATAGTATCCCAC (SEQ ID NO:178) |
| Dual_ V2_R ab7a_ 40 6 | GTGGAATAGTATAACAATATGCTAAATGTTGTTATAGTATCCCACT GCCGCCAGCTGGATTTCCCAATTCTGAGTAACACTCTGCGTGGAATAG TATAACAATATGCTAAATGTTGTTATAGTATCCCAC(SEQ ID NO:179) |
| Dual_ V2_R ab7a_ 60 6 | GTGGAATAGTATAACAATATGCTAAATGTTGTTATAGTATCCCACT GCCGCCAGCTGGATTTCCCAATTCTGAGTAACACTCTGCAATCCAAAC AGGGTTCAACCGTGGAATAGTATAACAATATGCTAAATGTTGTTATA GTATCCCAC(SEQ ID NO:180) |
| Dual_ V2_R ab7a_ 80_6 | GTGGAATAGTATAACAATATGCTAAATGTTGTTATAGTATCCCACT GCCGCCAGCTGGATTTCCCAATTCTGAGTAACACTCTGCAATCCAAAC AGGGTTCAACCCTCCACCTTACAGGCCTGCAGTGGAATAGTATAACA ATATGCTAAATGTTGTTATAGTATCCCAC(SEQ ID NO:181) |
| Dual_ V2_R ab7a_ 100_6 | GTGGAATAGTATAACAATATGCTAAATGTTGTTATAGTATCCCACT GCCGCCAGCTGGATTTCCCAATTCTGAGTAACACTCTGCAATCCAAAC AGGGTTCAACCCTCCACCTTACAGGCCTGCATTACAGGACTTAAACAC ATAGTGGAATAGTATAACAATATGCTAAATGTTGTTATAGTATCCC AC (SEQ ID NO:182) |
| Dual_ V2_R ab7a_ 120_6 | GTGGAATAGTATAACAATATGCTAAATGTTGTTATAGTATCCCACT GCCGCCAGCTGGATTTCCCAATTCTGAGTAACACTCTGCAATCCAAAC AGGGTTCAACCCTCCACCTTACAGGCCTGCATTACAGGACTTAAACAC ATAATCCAAGAATTTCTTACACTGTGGAATAGTATAACAATATGCTA AATGTTGTTATAGTATCCCAC (SEQ ID NO:183) |
| Dual_ V2_R ab7a_ 20_10 | GTGGAATAGTATAACAATATGCTAAATGTTGTTATAGTATCCCACA TACTGCCGCCAGCTGGATTGTGGAATAGTATAACAATATGCTAAATG TTGTTATAGTATCCCAC (SEQ ID NO:184) |
| Dual_ V2_R ab7a_ 40_20 | GTGGAATAGTATAACAATATGCTAAATGTTGTTATAGTATCCCACA CTGTACAGAATACTGCCGCCAGCTGGATTTCCCAATTCTGTGGAATAG |
| | TATAACAATATGCTAAATGTTGTTATAGTATCCCAC (SEQ ID NO:185) |
| Dual_ V2_R ab7a_ 60_30 | GTGGAATAGTATAACAATATGCTAAATGTTGTTATAGTATCCCACT CTTGTGTCTACTGTACAGAATACTGCCGCCAGCTGGATTTCCCAATTCT GAGTAACACTGTGGAATAGTATAACAATATGCTAAATGTTGTTATA GTATCCCAC (SEQ ID NO:186) |
| Dual_ V2_R ab7a_ 80_40 | GTGGAATAGTATAACAATATGCTAAATGTTGTTATAGTATCCCACC GTACATAATTCTTGTGTCTACTGTACAGAATACTGCCGCCAGCTGGATT TCCCAATTCTGAGTAACACTCTGCAATCCAGTGGAATAGTATAACAAT ATGCTAAATGTTGTTATAGTATCCCAC (SEQ ID NO:187) |
| Dual_ V2_R ab7a_ 100_5 0 | GTGGAATAGTATAACAATATGCTAAATGTTGTTATAGTATCCCACT GATAAAAGGCGTACATAATTCTTGTGTCTACTGTACAGAATACTGCCG CCAGCTGGATTTCCCAATTCTGAGTAACACTCTGCAATCCAAACAGGG TTCGTGGAATAGTATAACAATATGCTAAATGTTGTTATAGTATCCC AC (SEQ ID NO:188) |
| Dual_ V2_R ab7a_ 120_6 0 | GTGGAATAGTATAACAATATGCTAAATGTTGTTATAGTATCCCACC TTAAGTCTTTGATAAAAGGCGTACATAATTCTTGTGTCTACTGTACAGA ATACTGCCGCCAGCTGGATTTCCCAATTCTGAGTAACACTCTGCAATCC AAACAGGGTTCAACCCTCCACGTGGAATAGTATAACAATATGCTAAA TGTTGTTATAGTATCCCAC (SEQ ID NO:189) |
| Dual_ V7_Ra b7a_20 6 | GTGGAAGAGGAGAACAATAGGCTAAACGTTGTTCTCGTCTCCCA CTGCCGCCAGCTGGATTTCCCAATTCTGAGTGTGGAAGAGGAGAAC AATAGGCTAAACGTTGTTCTCGTCTCCCAC(SEQ ID NO:190) |
| Dual_V 7_Rab7 a 40 6 | GTGGAAGAGGAGAACAATAGGCTAAACGTTGTTCTCGTCTCCCA CTGCCGCCAGCTGGATTTCCCAATTCTGAGTAACACTCTGCGTGGAA GAGGAGAACAATAGGCTAAACGTTGTTCTCGTCTCCCAC(SEQ ID NO:191) |
| Dual_V 7_Rab7 a 60 6 | GTGGAAGAGGAGAACAATAGGCTAAACGTTGTTCTCGTCTCCCA CTGCCGCCAGCTGGATTTCCCAATTCTGAGTAACACTCTGCAATCCA AACAGGGTTCAACCGTGGAAGAGGAGAACAATAGGCTAAACGTTG TTCTCGTCTCCCAC(SEQ ID NO:192) |
| Dual_V 7_Rab7 a 80 6 | GTGGAAGAGGAGAACAATAGGCTAAACGTTGTTCTCGTCTCCCA CTGCCGCCAGCTGGATTTCCCAATTCTGAGTAACACTCTGCAATCCA AACAGGGTTCAACCCTCCACCTTACAGGCCTGCAGTGGAAGAGGAG AACAATAGGCTAAACGTTGTTCTCGTCTCCCAC(SEQ ID NO:193) |
| Dual_V 7_Rab7 a_100_ 6 | GTGGAAGAGGAGAACAATAGGCTAAACGTTGTTCTCGTCTCCCA CTGCCGCCAGCTGGATTTCCCAATTCTGAGTAACACTCTGCAATCCA AACAGGGTTCAACCCTCCACCTTACAGGCCTGCATTACAGGACTTAA ACACATAGTGGAAGAGGAGAACAATAGGCTAAACGTTGTTCTCGT CTCCCAC (SEQ ID NO:194) |
| Dual_V 7_Rab7 a_120_ 6 | GTGGAAGAGGAGAACAATAGGCTAAACGTTGTTCTCGTCTCCCA CTGCCGCCAGCTGGATTTCCCAATTCTGAGTAACACTCTGCAATCCA AACAGGGTTCAACCCTCCACCTTACAGGCCTGCATTACAGGACTTAA ACACATAATCCAAGAATTTCTTACACTGTGGAAGAGGAGAACAATA GGCTAAACGTTGTTCTCGTCTCCCAC (SEQ ID NO:195) |
| Dual_V 7_Rab7 a_20_1 0 | GTGGAAGAGGAGAACAATAGGCTAAACGTTGTTCTCGTCTCCCA CATACTGCCGCCAGCTGGATTGTGGAAGAGGAGAACAATAGGCTA AACGTTGTTCTCGTCTCCCAC (SEQ ID NO:196) |
| Dual_V 7_Rab7 a_40_2 0 | GTGGAAGAGGAGAACAATAGGCTAAACGTTGTTCTCGTCTCCCA CACTGTACAGAATACTGCCGCCAGCTGGATTTCCCAATTCTGTGGAA GAGGAGAACAATAGGCTAAACGTTGTTCTCGTCTCCCAC (SEQ ID NO:197) |
| Dual_V 7_Rab7 a_60_3 0 | GTGGAAGAGGAGAACAATAGGCTAAACGTTGTTCTCGTCTCCCA CTCTTGTGTCTACTGTACAGAATACTGCCGCCAGCTGGATTTCCCAAT TCTGAGTAACACTGTGGAAGAGGAGAACAATAGGCTAAACGTTGT TCTCGTCTCCCAC (SEQ ID NO:198) |
| Dual_V 7_Rab7 a_80_4 0 | GTGGAAGAGGAGAACAATAGGCTAAACGTTGTTCTCGTCTCCCA CCGTACATAATTCTTGTGTCTACTGTACAGAATACTGCCGCCAGCTGG ATTTCCCAATTCTGAGTAACACTCTGCAATCCAGTGGAAGAGGAGA ACAATAGGCTAAACGTTGTTCTCGTCTCCCAC (SEQ ID NO:199) |
| Dual_V 7_Rab7 a_100_ 50 | GTGGAAGAGGAGAACAATAGGCTAAACGTTGTTCTCGTCTCCCA CTGATAAAAGGCGTACATAATTCTTGTGTCTACTGTACAGAATACTG CCGCCAGCTGGATTTCCCAATTCTGAGTAACACTCTGCAATCCAAAC AGGGTTCGTGGAAGAGGAGAACAATAGGCTAAACGTTGTTCTCGT CTCCCAC (SEQ ID NO:200) |
| Dual_V 7_Rab7 a_120_ 60 | GTGGAAGAGGAGAACAATAGGCTAAACGTTGTTCTCGTCTCCCA CCTTAAGTCTTTGATAAAAGGCGTACATAATTCTTGTGTCTACTGTAC AGAATACTGCCGCCAGCTGGATTTCCCAATTCTGAGTAACACTCTGC AATCCAAACAGGGTTCAACCCTCCACGTGGAAGAGGAGAACAATA GGCTAAACGTTGTTCTCGTCTCCCAC (SEQ ID NO:201) |

A polynucleotide sequence encoding for an RNA editing entity recruiting domain, can include recruitment of any ADAR protein (such as ADAR1, ADAR2, ADAR3 or any combination thereof), any APOBEC protein (such as APOBEC1, APOBEC2, APOBEC3A, APOBEC3B, APOBEC3C, APOBEC3E, APOBEC3F, APOBEC3G, APOBEC3H, APOBEC4, or any combination thereof), or a combination thereof. In some cases, the ADAR or APOBEC protein recruited can be mammalian. In some cases, the ADAR or APOBEC protein recruited can be human. In some cases, the ADAR or APOBEC protein recruited can be recombinant (such as an exogenously delivered ADAR or APOBEC), modified (such as an exogenously delivered ADAR or APOBEC), endogenous (such as an endogenous ADAR or APOBEC), or any combination thereof.

In some cases, the at least one RNA editing entity recruiting domain does not form a second structure comprising a stem-loop. In some cases, the at least one RNA editing entity recruiting domain forms a second structure comprising a stem-loop. In some cases, the at least one RNA editing entity recruiting domain forms a second structure that does not comprise a stem-loop. In some cases, the at least one RNA editing entity recruiting domain forms a secondary structure comprising a linear portion. In some cases, the at least one RNA editing entity recruiting domain forms a secondary structure comprising a cruciform or portion thereof.

A polynucleotide sequence can encode for more than one RNA editing recruiting domains. In some cases, a polynucleotide sequence can encode for a plurality of recruiting domains. In some cases, a polynucleotide sequence can encode for 2, 3, 4, 5, 6 or more recruiting domains. A recruiting domain of a plurality can include an Alu domain, an APOBEC domain, a GluR2 domain, Cas13 domain, or any combination thereof. In some case, the Alu domain, APOBEC domain, Cas13 domain, or GluR2 domain can be a naturally occurring recruiting domain. In some cases, the Alu domain, the APOBEC domain, Cast3 domain, or the GluR2 domain can be non-naturally occurring, can be modified from a native sequence, or can be recombinant. At least one of the plurality of recruiting domains can comprise a single stranded sequence. At least one of the plurality of recruiting domains can comprise a plurality of Alu repeats. At least one of the plurality of recruiting domains can form a secondary structure comprising a stem-loop. At least one of the plurality of recruiting domains can form a secondary structure that does not comprise a stem-loop. At least one of the plurality of recruiting domains can form a secondary structure that comprises a cruciform or portion thereof. At least one of the plurality of recruiting domains can form a secondary structure that comprises a toe hold.

In some cases, a nucleic acid can encode for at least one RNA editing entity recruiting domain. In some cases, the nucleic acid can encode for an RNA that is complementary to at least a portion of a target RNA. In some cases, the nucleic acid can encode for a recruiting domain and a targeting domain. In some cases, the nucleic acid can encode for a recruiting domain and a nucleic acid can encode for a targeting domain. The portion of the target RNA can comprise a single base. The portion of the target RNA can comprise a plurality of bases. The portion of the target RNA can comprise about: 2, 3, 4, 5, 6, 7, 8, 9, 10, 20, 30, 40, 50, 60, 70, 100, 200, 300, 400, 500, 600, 700, 800, 900, 100 base pairs or more. In some cases, the target RNA can comprise from about 1 bps to about 10 bps. In some cases, the target RNA can comprise from about 10 bps to about 100 bps. In some cases, the target RNA can comprise from about 10 bps to about 500 bps. In some cases, the target RNA can comprise from about 10 bps to about 1000 bps. A nucleic acid comprising a targeting domain and a recruiting domain can comprise a contiguous sequence of at least about 200bp in length. A nucleic acid comprising a targeting domain and a recruiting domain can comprise a contiguous sequence of at least about 150bp in length. A nucleic acid comprising a targeting domain and a recruiting domain can comprise a contiguous sequence of at least about 250bp in length. A nucleic acid comprising a targeting domain and a recruiting domain can comprise a contiguous sequence of at least about 275bp in length. A nucleic acid comprising a targeting domain and a recruiting domain can comprise a contiguous sequence of at least about 300bp in length. A nucleic acid comprising a targeting domain and a recruiting domain can comprise a contiguous sequence of at least about 400bp in length. A nucleic acid comprising a targeting domain and a recruiting domain can comprise a contiguous sequence of at least about 500bp in length.

A vector can be employed to deliver a nucleic acid. A vector can comprise DNA, such as double stranded DNA or single stranded DNA. A vector can comprise RNA. In some cases, the RNA can comprise a base modification. The vector can comprise a recombinant vector. The vector can be a vector that is modified from a naturally occurring vector. The vector can comprise at least a portion of a non-naturally occurring vector. Any vector can be utilized. In some cases, the vector can comprise a viral vector, a liposome, a nanoparticle, an exosome, an extracellular vesicle, or any combination thereof. In some cases, a viral vector can comprise an adenoviral vector, an adeno-associated viral vector (AAV), a lentiviral vector, a retroviral vector, a portion of any of these, or any combination thereof. In some cases, a nanoparticle vector can comprise a polymeric-based nanoparticle, an aminolipid based nanoparticle, a metallic nanoparticle (such as gold-based nanoparticle), a portion of any of these, or any combination thereof. In some cases, a vector can comprise an AAV vector. A vector can be modified to include a modified VP1 protein (such as an AAV vector modified to include a VP1 protein). An AAV can comprise a serotype - such as an AAV1 serotype, an AAV2 serotype, AAV3 serotype, an AAV4 serotype, AAV5 serotype, an AAV6 serotype, AAV7 serotype, an AAV8 serotype, an AAV9 serotype, a derivative of any of these, or any combination thereof.

Unless defined otherwise, all technical and scientific terms used herein have the same meanings as commonly understood by one of ordinary skill in the art to which this disclosure belongs. All nucleotide sequences provided herein are presented in the 5' to 3' direction unless identified otherwise. Although any methods and materials similar or equivalent to those described herein can be used in the practice or testing of the disclosure, the preferred methods, devices, and materials are now described. All technical and patent publications cited herein are incorporated herein by reference in their entirety. Nothing herein is to be construed as an admission that the disclosure is not entitled to antedate such disclosure by virtue of prior disclosure.

The practice of the technology will employ, unless otherwise indicated, conventional techniques of tissue culture, immunology, molecular biology, microbiology, cell biology, and recombinant DNA, which are within the skill of the art. See, e.g., Green and Sambrook eds. (2012) Molecular Cloning: A Laboratory Manual, 4th edition; the series Ausubel et al. eds. (2015) Current Protocols in Molecular Biology; the series Methods in Enzymology (Academic Press, Inc., N.Y.); MacPherson et al. (2015) PCR 1: A Practical Approach (IRL Press at Oxford University Press); MacPherson et al. (1995) PCR 2: A Practical Approach; McPherson et al. (2006) PCR: The Basics (Garland Science); Harlow and Lane eds. (1999) Antibodies, A Laboratory Manual; Greenfield ed. (2014) Antibodies, A Laboratory Manual; Freshney (2010) Culture of Animal Cells: A Manual of Basic Technique, 6th edition; Gait ed. (1984) Oligonucleotide Synthesis; U.S. Pat. No. 4,683,195; Hames and Higgins eds. (1984) Nucleic Acid Hybridization; Anderson (1999) Nucleic Acid Hybridization; Herdewijn ed. (2005) Oligonucleotide Synthesis: Methods and Applications; Hames and Higgins eds. (1984) Transcription and Translation; Buzdin and Lukyanov ed. (2007) Nucleic Acids Hybridization: Modern Applications; Immobilized Cells and Enzymes (IRL Press (1986)); Grandi ed. (2007) In vitro Transcription and Translation Protocols, 2nd edition; Guisan ed. (2006) Immobilization of Enzymes and Cells; Perbal (1988) A Practical Guide to Molecular Cloning, 2nd edition; Miller and Calos eds, (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); Lundblad and Macdonald eds. (2010) Handbook of Biochemistry and Molecular Biology, 4th edition; Herzenberg et al. eds (1996) Weir's Handbook of Experimental Immunology, 5th edition; and/or more recent editions thereof.

The terminology used in the description herein is for the purpose of describing particular embodiments only and is not intended to be limiting of the disclosure.

All numerical designations, e.g., pH, temperature, time, concentration, and molecular weight, including ranges, are approximations which are varied ( + ) or ( - ) by increments of 1.0 or 0.1, as appropriate or alternatively by a variation of +/- 15 %, or alternatively 10% or alternatively 5% or alternatively 2%. It is to be understood, although not always explicitly stated, that all numerical designations are preceded by the term "about". It also is to be understood, although not always explicitly stated, that the reagents described herein are merely exemplary and that equivalents of such are known in the art.

Unless the context indicates otherwise, it is specifically intended that the various features of the disclosure described herein can be used in any combination. Moreover, the disclosure also contemplates that in some embodiments, any feature or combination of features set forth herein can be excluded or omitted. To illustrate, if the specification states that a complex comprises components A, B and C, it is specifically intended that any of A, B or C, or a combination thereof, can be omitted and disclaimed singularly or in any combination.

Unless explicitly indicated otherwise, all specified embodiments, features, and terms intend to include both the recited embodiment, feature, or term and biological equivalents thereof.

### Definitions

As used in the specification and claims, the singular form "a", "an" and "the" include plural references unless the context clearly dictates otherwise. For example, the term "a polypeptide" includes a plurality of polypeptides, including mixtures thereof. Accordingly, unless the contrary is indicated, the numerical parameters set forth in this application are approximations that can vary depending upon the desired properties sought to be obtained by the disclosure.

The term "about," as used herein can mean within an acceptable error range for the particular value as determined by one of ordinary skill in the art, which can depend in part on how the value is measured or determined, e.g., the limitations of the measurement system. For example, "about" can mean plus or minus 10%, per the practice in the art. Alternatively, "about" can mean a range of plus or minus 20%, plus or minus 10%, plus or minus 5%, or plus or minus 1% of a given value. Alternatively, particularly with respect to biological systems or processes, the term can mean within an order of magnitude, within 5-fold, or within 2-fold, of a value. Where particular values are described in the application and claims, unless otherwise stated the term "about" meaning within an acceptable error range for the particular value can be assumed. Also, where ranges and/or subranges of values are provided, the ranges and/or subranges can include the endpoints of the ranges and/or subranges. In some cases, variations can include an amount or concentration of 20%, 10%, 5%, 1 %, 0.5%, or even 0.1 % of the specified amount.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

The terms "adenine", "guanine", "cytosine", "thymine", "uracil" and "hypoxanthine" (the nucleobase in inosine) as used herein refer to the nucleobases as such.

The terms "adenosine", "guanosine", "cytidine", "thymidine", "uridine" and "inosine", refer to the nucleobases linked to the (deoxy)ribosyl sugar.

The term "adeno-associated virus" or "AAV" as used herein refers to a member of the class of viruses associated with this name and belonging to the genus dependoparvovirus, family Parvoviridae. Multiple serotypes of this virus are known to be suitable for gene delivery; all known serotypes can infect cells from various tissue types. At least 11, sequentially numbered, are disclosed in the prior art. Non-limiting exemplary serotypes useful for the purposes disclosed herein include any of the 11 serotypes, e.g., AAV2 and AAV8. The term "lentivirus" as used herein refers to a member of the class of viruses associated with this name and belonging to the genus lentivirus, family Retroviridae. While some lentiviruses are known to cause diseases, other lentivirus are known to be suitable for gene delivery. *See, e.g.,* Tomás et al. (2013) Biochemistry, Genetics and Molecular Biology: "Gene Therapy - Tools and Potential Applications," ISBN 978-953-51-1014-9, DOI: 10.5772/52534.

The term "adenosine deaminases acting on RNA" or "ADAR" as used herein can refer to an adenosine deaminase that can convert adenosines (A) to inosines (I) in an RNA sequence. ADAR1 and ADAR2 are two exemplary species of ADAR that are involved in mRNA editing *in vivo.* Non-limiting exemplary sequences for ADAR1 can be found under the following reference numbers: HGNC: 225; Entrez Gene: 103; Ensembl: ENSG 00000160710; OMIM: 146920; UniProtKB: P55265; and GeneCards: GC01M154554, as well as biological equivalents thereof. Non-limiting exemplary sequences for ADAR2 can be found under the following reference numbers: HGNC: 226; Entrez Gene: 104; Ensembl: ENSG00000197381; OMIM: 601218; UniProtKB: P78563; and GeneCards: GC21P045073, as well as biological equivalents thereof. Further non-limited exemplary sequences of the catalytic domain are provided hereinabove. The forward and reverse RNA used to direct sitespecific ADAR editing are known as "adRNA" and "radRNA," respectively. The catalytic domains of ADAR1 and ADAR2 are comprised in the sequences provided herein below.

### ADAR1 catalytic domain:

KAERMGFTEVTPVTGASLRRTMLLLSRSPEAQPKTLPLTGSTFHDQIAMLSHRCFNTL
TNSFQPSLLGRKILAAIIMKKDSEDMGVVVSLGTGNRCVKGDSLSLKGETVNDCHAE
IISRRGFIRFLYSELMKYNSQTAKDSIFEPAKGGEKLQIKKTVSFHLYISTAPCGDGALF
DKSCSDRAMESTESRHYPVFENPKQGKLRTKVENGEGTIPVESSDIVPTWDGIRLGER
LRTMSCSDKILRWNVLGLQGALLTHFLQPIYLKSVTLGYLFSQGHLTRAICCRVTRD
GSAFEDGLRHPFIVNHPKVGRVSIYDSKRQSGKTKETSVNWCLADGYDLEILDGTRG
TVDGPRNELSRVSKKNIFLLFKKLCSFRYRRDLLRLSYGEAKKAARDYETAKNYFKK
GLKDMGYGNWISKPQEEKNFYLCPV (SEQ ID NO:140)

### ADAR2 catalytic domain:

QLHLPQVLADAVSRLVLGKFGDLTDNFSSPHARRKVLAGVVMTTGTDVKDA
KVISVSTGTKCINGEYMSDRGLALNDCHAEIISRRSLLRFLYTQLELYLNNKDDQKRS
IFQKSERGGFRLKENVQFHLYISTSPCGDARIFSPHEPILEEPADRHPNRKARGQLRTKI
ESGEGTIPVRSNASIQTWDGVLQGERLLTMSCSDKIARWNVVGIQGSLLSIFVEPIYFS
SIILGSLYHGDHLSRAMYQRISNIEDLPPLYTLNKPLLSGISNAEARQPGKAPNFSVNW
TVGDSAIEVINATTGKDELGRASRLCKHALYCRWMRVHGKVPSHLLRSKITKPNVY
HESKLAAKEYQAAKARLFTAFIKAGLGAWVEKPTEQDQFSLT (SEQ ID NO:141)

The double stranded RNA binding domains (dsRBD) of an ADAR is comprised in the sequence provided herein below.

### ADAR dsRBD:

MDIEDEENMSSSSTDVKENRNLDNVSPKDGSTPGPGEGSQLSNGGGGGPGRK
RPLEEGSNGHSKYRLKKRRKTPGPVLPKNALMQLNEIKPGLQYTLLSQTGPVHAPLF
VMSVEVNGQVFEGSGPTKKKAKLHAAEKALRSFVQFPNASEAHLAMGRTLSVNTD
FTSDQADFPDTLFNGFETPDKAEPPFYVGSNGDDSFSSSGDLSLSASPVPASLAQPPLP
VLPPFPPPSGKNPVMILNELRPGLKYDFLSESGESHAKSFVMSVVVDGQFFEGSGRNK
KLAKARAAQSALAAIFN (SEQ ID NO:142)

It is appreciated that further mutations can be made to the sequence of the ADAR and/or its various domains. For example, the disclosure provides E488Q and E1008Q mutants of both ADAR1 and ADAR2, as well as a "promiscuous" variant of ADAR2 - resulting from a C-terminal deletion. This "promiscuous" variant is known as such because it demonstrated promiscuity in edited reads with several A's close to a target sequence showing an A to G conversion (verified across 2 different loci). The sequence of this variant is provided herein below.

### "Promiscuous" ADAR2 variant:

MLRSFVQFPNASEAHLAMGRTLSVNTDFTSDQADFPDTLFNGFETPDKAEPPF
YVGSNGDDSFSSSGDLSLSASPVPASLAQPPLPVLPPFPPPSGKNPVMILNELRPGLKY
DFLSESGESHAKSFVMSVVVDGQFFEGSGRNKKLAKARAAQSALAAIFNLHLDQTPS
RQPIPSEGLQLHLPQVLADAVSRLVLGKFGDLTDNFSSPHARRKVLAGVVMTTGTD
VKDAKVISVSTGTKCINGEYMSDRGLALNDCHAEIISRRSLLRFLYTQLELYLNNKDD
QKRSIFQKSERGGFRLKENVQFHLYISTSPCGDARIFSPHEPILEEPADRHPNRKARGQ
LRTKIESGEGTIPVRSNASIQTWDGVLQGERLLTMSCSDKIARWNVVGIQGSLLSIFVE
PIYFSSIILGSLYHGDHLSRAMYQRISNIEDLPPLYTLNKPLLSGISNAEARQPGKAPNF
SVNWTVGDSAIEVINATTGKDELGRASRLCKHALYCRWMRVHGKVPSHLLRSKITK
PNVYHESKLAAKEYQAAKARLFTAFIKAGLGAWVEKPTEQDQFSLTP* (SEQ ID
NO:143)

Not to be bound by theory, a C-terminal deletion in ADAR1 can produce the same or similar effect.

The term "Alu domain" can refer to a sequence obtained from the Alu transposable element ("Alu element"). Typically the Alu element is about 300 base pairs in length. An Alu element typically comprise a structure: cruciform-polyA5-TAC-polyA6-cruciform-polyA tail, wherein both cruciform domains are similar in nucleotide sequence. An "Alu domain" can comprise a cruciform portion of the Alu element. In some embodiments, two Alu domains comprising cruciform structures are linked by a sequence complementary to a target RNA sequence.

The term "APOBEC" as used herein can refer to any protein that falls within the family of evolutionarily conserved cytidine deaminases involved in mRNA editing - catalyzing a C to T edit, which can be interpreted as a C to U conversion - and equivalents thereof. In some aspects, the term APOBEC can refer to any one of APOBEC1, APOBEC2, APOBEC3A, APOBEC3B, APOBEC3C, APOBEC3E, APOBEC3F, APOBEC3G, APOBEC3H, APOBEC4, or equivalents each thereof. Non-limiting exemplary sequences of fusion proteins comprising one or more APOBEC domains are provided herein both fused to an ADAR domain or fused to alternative domains to render them suitable for use in an RNA editing system. To this end, APOBECs can be considered an equivalent of ADAR - catalyzing editing albeit by a different conversion. Thus, not to be bound by theory, it is believed that all embodiments contemplated herein for use with an ADAR based editing system can be adapted for use in an APOBEC based RNA editing system. In some cases, use of APOBEC can involve certain modifications, such as but not limited to the use of particular guide RNA or "gRNA" to recruit the enzyme.

An "aptamer" can refer to a short single-stranded oligonucleotide capable of binding various molecules with high affinity and specificity. Non-limiting examples of aptamers are described in Lakhin, A. V. et al. (2013). Acta naturae, 5(4), 34-43.

As used herein, the term "comprising" is intended to mean that the compositions and methods include the recited elements, but do not exclude others. Unless otherwise indicated, open terms for example "contain," "containing," "include," "including," and the like mean comprising. "Consisting essentially of' when used to define compositions and methods, shall mean excluding other elements of any essential significance to the combination for the intended use. Thus, a composition consisting essentially of the elements as defined herein may not exclude trace contaminants from the isolation and purification method and pharmaceutically acceptable carriers, such as phosphate buffered saline, preservatives, and the like. "Consisting of' shall mean excluding more than trace elements of other ingredients and substantial method steps for administering the compositions of this disclosure. Embodiments defined by each of these transition terms are within the scope of this disclosure.

"Canonical amino acids" refer to those 20 amino acids found naturally in the human body shown in the table below with each of their three letter abbreviations, one letter abbreviations, structures, and corresponding codons:

| **non-polar, aliphatic residues** | | | | |
|---|---|---|---|---|
| Glycine | Gly | G | 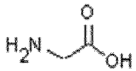 | GGU GGC GGA GGG |
| Alanine | Ala | A | 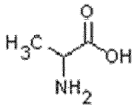 | GCU GCC GCA GCG |
| Valine | Val | V | 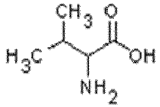 | GUU GUC GUA GUG |
| Leucine | Leu | L | 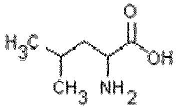 | UUA UUG CUU CUC CUA CUG |
| Isoleucine | Ile | I | 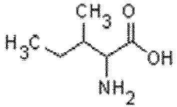 | AUU AUC AUA |
| Proline | Pro | P | 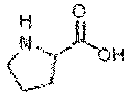 | CCU CCC CCA CCG |

| **aromatic residues** | | | | |
|---|---|---|---|---|
| Phenylalanine | Phe | F | 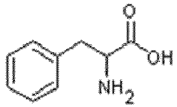 | UUU UUC |
| Tyrosine | Tyr | Y | 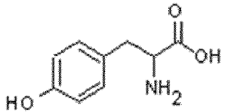 | UAU UAC |
| Tryptophan | Trp | W | 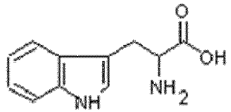 | UGG |

| **polar, non-charged residues** | | | | |
|---|---|---|---|---|
| Serine | Ser | S | 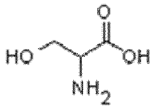 | UCU UCC UCA UCG AGU AGC |
| Threonine | Thr | T | 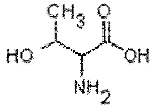 | ACU ACC ACA ACG |
| Cysteine | Cys | C | 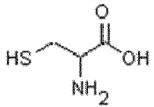 | UGU UGC |
| Methionine | Met | M | 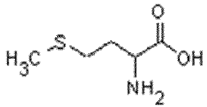 | AUG |
| Asparagine | Asn | N | 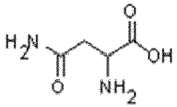 | AAU AAC |
| Glutamine | Gln | Q | 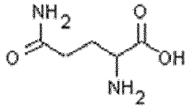 | CAA CAG |

| **positively charged residues** | | | | |
|---|---|---|---|---|
| Lysine | Lys K | 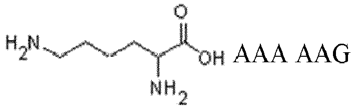 | | |
| Arginine | Arg R | 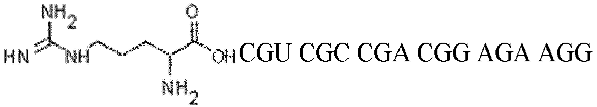 | | |
| Histidine | His H | 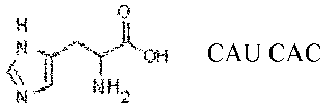 | | |

| **negatively charged residues** | | | | |
|---|---|---|---|---|
| Aspartate | AspD | 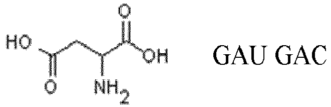 | | |
| Glutamate | Glu E | 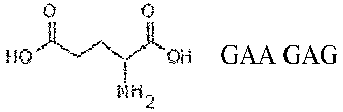 | | |

The term "Cas9" can refer to a CRISPR associated endonuclease referred to by this name. Non-limiting exemplary Cas9s include *Staphylococcus aureus* Cas9, nuclease dead Cas9, and orthologs and biological equivalents each thereof. Orthologs include but are not limited to *Streptococcus pyogenes* Cas9 ("spCas9"), Cas 9 from *Streptococcus thermophiles, Legionella pneumophilia, Neisseria lactamica, Neisseria meningitides, Francisella novicida*; and Cpf1 (which performs cutting functions analogous to Cas9) from various bacterial species including Acidaminococcus spp. and Francisella novicida U112. For example, UniProtKB G3ECR1 (CAS9_STRTR)) as well as dead Cas9 or dCas9, which lacks endonuclease activity (e.g., with mutations in both the RuvC and HNH domain) can be used. The term "Cas9" may further refer to equivalents of the referenced Cas9 having at least about 60%, 65%, 70%, 75%, 80%, 85%, 90%, 95%, or 99% identity thereto, including but not limited to other large Cas9 proteins. In some embodiments, the Cas9 is derived from *Campylobacter jejuni* or another Cas9 orthologs 1000 amino acids or less in length.

As used herein, the term "CRISPR" can refer to a technique of sequence specific genetic manipulation relying on the clustered regularly interspaced short palindromic repeats pathway. CRISPR can be used to perform gene editing and/or gene regulation, as well as to simply target proteins to a specific genomic location. "Gene editing" can refer to a type of genetic engineering in which the nucleotide sequence of a target polynucleotide is changed through introduction of deletions, insertions, single stranded or double stranded breaks, or base substitutions to the polynucleotide sequence. In some aspect, CRISPR-mediated gene editing utilizes the pathways of nonhomologous end-joining (NHEJ) or homologous recombination to perform the edits. Gene regulation can refer to increasing or decreasing the production of specific gene products such as protein or RNA.

The term "deficiency" as used herein can refer to lower than normal (physiologically acceptable) levels of a particular agent. In context of a protein, a deficiency can refer to lower than normal levels of the full-length protein.

As used herein, the term "detectable marker" can refer to at least one marker capable of directly or indirectly, producing a detectable signal. A non-exhaustive list of this marker includes enzymes which produce a detectable signal, for example by colorimetry, fluorescence, luminescence, such as horseradish peroxidase, alkaline phosphatase, β-galactosidase, glucose-6-phosphate dehydrogenase, chromophores such as fluorescent, luminescent dyes, groups with electron density detected by electron microscopy or by their electrical property such as conductivity, amperometry, voltammetry, impedance, detectable groups, for example whose molecules are of sufficient size to induce detectable modifications in their physical and/or chemical properties, such detection can be accomplished by optical methods such as diffraction, surface plasmon resonance, surface variation , the contact angle change or physical methods such as atomic force spectroscopy, tunnel effect, or radioactive molecules such as ³² P, ³⁵ S or ¹²⁵ I.

As used herein, the term "domain" can refer to a particular region of a protein or polypeptide and is associated with a particular function. For example, "a domain which associates with an RNA hairpin motif' can refer to the domain of a protein that binds one or more RNA hairpin. This binding can optionally be specific to a particular hairpin.

The term "dystrophin" as used herein refers to the protein corresponding with that name and encoded by the gene *Dmd*; a non-limiting example of which is found under UniProt Reference Number P11532 (for humans) and P11531 (for mice).

An "editing inducer element" can refer to a structure that is largely a double-stranded RNA, which is necessary for efficient RNA editing. Non-limiting examples of editing inducer elements are described in Daniel, C. et al. (2017) Genome Biol. 18, 195. A further non-limiting example of an editing inducer element is provided by the structure below (SEQ ID NO:15):

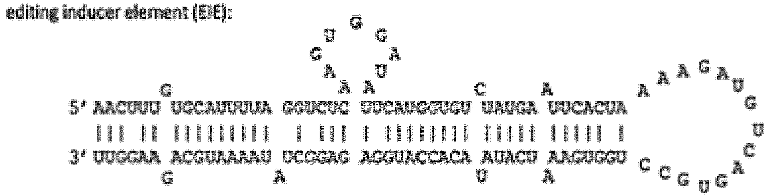

### Modes of Carrying Out the Disclosure

ADARs are naturally occurring RNA editing enzymes that catalyze the hydrolytic deamination of adenosine to inosine that is biochemically recognized as guanosine. APOBECs are enzymes, described herein above, that can perform a similar function but for cytosine to thymine.

The term "encode" as it is applied to polynucleotides can refer to a polynucleotide which is said to "encode" a polypeptide if, in its native state or when manipulated by methods well known to those skilled in the art, it can be transcribed and/or translated to produce the mRNA for the polypeptide and/or a fragment thereof. The antisense strand is the complement of such a nucleic acid, and the encoding sequence can be deduced therefrom.

The terms "equivalent" or "biological equivalent" are used interchangeably when referring to a particular molecule, biological, or cellular material and intend those having minimal homology while still maintaining desired structure or functionality.

"Eukaryotic cells" comprise all of the life kingdoms except monera. They can be easily distinguished through a membrane-bound nucleus. Animals, plants, fungi, and protists are eukaryotes or organisms whose cells are organized into complex structures by internal membranes and a cytoskeleton. The most characteristic membrane-bound structure is the nucleus. Unless specifically recited, the term "host" includes a eukaryotic host, including, for example, yeast, higher plant, insect and mammalian cells. Non-limiting examples of eukaryotic cells or hosts include simian, bovine, porcine, murine, rat, avian, reptilian and human.

As used herein, "expression" can refer to the process by which polynucleotides are transcribed into mRNA and/or the process by which the transcribed mRNA is subsequently being translated into peptides, polypeptides, or proteins. If the polynucleotide is derived from genomic DNA, expression can include splicing of the mRNA in an eukaryotic cell.

As used herein, the term "functional" can be used to modify any molecule, biological, or cellular material to intend that it accomplishes a particular, specified effect.

The term "Glur2 mRNA" as used herein can refer to the mRNA encoding ionotropic AMPA glutamate receptor 2 ("Glur2") which undergoes adenosine to inosine (A -> I) editing. This mRNA recruits ADARs in a site specific manner.

The term "gRNA" or "guide RNA" as used herein can refer to guide RNA sequences used to target specific polynucleotide sequences for gene editing employing the CRISPR technique. Techniques of designing gRNAs and donor therapeutic polynucleotides for target specificity are well known in the art. For example, Doench, J., et al. Nature biotechnology 2014; 32(12): 1262-7, Mohr, S. et al. (2016) FEBS Journal 283: 3232-38, and Graham, D., et al. Genome Biol. 2015; 16: 260. gRNA comprises or alternatively consists essentially of, or yet further consists of a fusion polynucleotide comprising CRISPR RNA (crRNA) and trans-activating CRIPSPR RNA (tracrRNA); or a polynucleotide comprising CRISPR RNA (crRNA) and trans-activating CRIPSPR RNA (tracrRNA). In some aspect, a gRNA is synthetic (Kelley, M. et al. (2016) J of Biotechnology 233 (2016) 74-83).

The terms "hairpin," "hairpin loop," "stem loop," and/or "loop" used alone or in combination with "motif" is used in context of an oligonucleotide to refer to a structure formed in single stranded oligonucleotide when sequences within the single strand which are complementary when read in opposite directions base pair to form a region whose conformation resembles a hairpin or loop.

"Homology" or "identity" or "similarity" can refer to sequence similarity between two peptides or between two nucleic acid molecules. Homology can be determined by comparing a position in each sequence which can be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the disclosure.

Homology refer to a % identity of a sequence to a reference sequence. As a practical matter, whether any particular sequence can be at least 50%, 60%, 70%, 80%, 85%, 90%, 92%, 95%, 96%, 97%, 98% or 99% identical to any sequence described herein (which can correspond with a particular nucleic acid sequence described herein), such particular polypeptide sequence can be determined conventionally using known computer programs such the Bestfit program (Wisconsin Sequence Analysis Package, Version 8 for Unix, Genetics Computer Group, University Research Park, 575 Science Drive, Madison, Wis. 53711). When using Bestfit or any other sequence alignment program to determine whether a particular sequence is, for instance, 95% identical to a reference sequence, the parameters can be set such that the percentage of identity is calculated over the full length of the reference sequence and that gaps in homology of up to 5% of the total reference sequence are allowed.

For example, in a specific embodiment the identity between a reference sequence (query sequence, *i.e.,* a sequence of the disclosure) and a subject sequence, also referred to as a global sequence alignment, can be determined using the FASTDB computer program based on the algorithm of Brutlag et al. (Comp. App. Biosci. 6:237-245 (1990)). In some cases, parameters for a particular embodiment in which identity is narrowly construed, used in a FASTDB amino acid alignment, can include: Scoring Scheme=PAM (Percent Accepted Mutations) 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject sequence, whichever is shorter. According to this embodiment, if the subject sequence is shorter than the query sequence due to N- or C-terminal deletions, not because of internal deletions, a manual correction can be made to the results to take into consideration the fact that the FASTDB program does not account for N- and C-terminal truncations of the subject sequence when calculating global percent identity. For subject sequences truncated at the N- and C-termini, relative to the query sequence, the percent identity can be corrected by calculating the number of residues of the query sequence that are lateral to the N- and C-terminal of the subject sequence, which are not matched/aligned with a corresponding subject residue, as a percent of the total bases of the query sequence. A determination of whether a residue is matched/aligned can be determined by results of the FASTDB sequence alignment. This percentage can be then subtracted from the percent identity, calculated by the FASTDB program using the specified parameters, to arrive at a final percent identity score. This final percent identity score can be used for the purposes of this embodiment. In some cases, only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence are considered for this manual correction. For example, a 90 residue subject sequence can be aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity can be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for.

"Hybridization" can refer to a reaction in which one or more polynucleotides react to form a complex that is stabilized via hydrogen bonding between the bases of the nucleotide residues. The hydrogen bonding can occur by Watson-Crick base pairing, Hoogstein binding, or in any other sequence-specific manner. The complex can comprise two strands forming a duplex structure, three or more strands forming a multi-stranded complex, a single selfhybridizing strand, or any combination of these. A hybridization reaction can constitute a step in a more extensive process, such as the initiation of a PC reaction, or the enzymatic cleavage of a polynucleotide by a ribozyme.

Examples of stringent hybridization conditions include: incubation temperatures of about 25°C to about 37°C; hybridization buffer concentrations of about 6x SSC to about 10x SSC; formamide concentrations of about 0% to about 25%; and wash solutions from about 4x SSC to about 8x SSC. Examples of moderate hybridization conditions include: incubation temperatures of about 40°C to about 50°C; buffer concentrations of about 9x SSC to about 2x SSC; formamide concentrations of about 30% to about 50%; and wash solutions of about 5x SSC to about 2x SSC. Examples of high stringency conditions include: incubation temperatures of about 55°C to about 68°C; buffer concentrations of about lx SSC to about 0.1x SSC; formamide concentrations of about 55% to about 75%; and wash solutions of about lx SSC, 0.1x SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed.

"Inhibit" as used herein refers to the ability to substantially antagonize, prohibit, prevent, restrain, slow, disrupt, alter, eliminate, stop, or reverse the progression or severity of the activity of a particular agent (e.g., infectious agent) or disease.

As used herein, the term "interferon" can refer to a group of signaling proteins known to be associated with the immune response. In context of this application, the interferons of interest are those that result in enhanced expression of an ADAR. The correlation between interferon α and ADAR1 is well known, and, thus, the disclosure contemplates use of interferon α as a means of increasing endogenous ADAR1 expression. Commercial sources of isolated or recombinant interferon α include but are not limited to Sigma-Aldrich, R&D Systems, Abcam, and Thermo Fisher Scientific. Alternatively, interferon α can be produced using a known vector and given protein sequence, e.g., Q6QNB6 (human IFNA).

The term "isolated" as used herein can refer to molecules or biologicals or cellular materials being substantially free from other materials. In one aspect, the term "isolated" can refer to nucleic acid, such as DNA or RNA, or protein or polypeptide (*e.g*., an antibody or derivative thereof), or cell or cellular organelle, or tissue or organ, separated from other DNAs or RNAs, or proteins or polypeptides, or cells or cellular organelles, or tissues or organs, respectively, that are present in the natural source. The term "isolated" also can refer to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and may not be found in the natural state. The term "isolated" is also used herein to refer to polypeptides which are isolated from other cellular proteins and is meant to encompass both purified and recombinant polypeptides. The term "isolated" is also used herein to refer to cells or tissues that are isolated from other cells or tissues and is meant to encompass both cultured and engineered cells or tissues.

"Messenger RNA" or "mRNA" is a nucleic acid molecule that is transcribed from DNA and then processed to remove non-coding sections known as introns. The resulting mRNA is exported from the nucleus (or another locus where the DNA is present) and translated into a protein. The term "pre-mRNA" can refer to the strand prior to processing to remove non-coding sections.

The term "mutation" as used herein, can refer to an alteration to a nucleic acid sequence encoding a protein relative to the consensus sequence of said protein. "Missense" mutations result in the substitution of one codon for another; "nonsense" mutations change a codon from one encoding a particular amino acid to a stop codon. Nonsense mutations often result in truncated translation of proteins. "Silent" mutations are those which have no effect on the resulting protein. As used herein the term "point mutation" can refer to a mutation affecting only one nucleotide in a gene sequence. "Splice site mutations" are those mutations present pre-mRNA (prior to processing to remove introns) resulting in mistranslation and often truncation of proteins from incorrect delineation of the splice site. A mutation can comprise a single nucleotide variation (SNV). A mutation can comprise a sequence variant, a sequence variation, a sequence alteration, or an allelic variant. The reference DNA sequence can be obtained from a reference database. A mutation can affect function. A mutation may not affect function. A mutation can occur at the DNA level in one or more nucleotides, at the ribonucleic acid (RNA) level in one or more nucleotides, at the protein level in one or more amino acids, or any combination thereof. The reference sequence can be obtained from a database such as the NCBI Reference Sequence Database (RefSeq) database. Specific changes that can constitute a mutation can include a substitution, a deletion, an insertion, an inversion, or a conversion in one or more nucleotides or one or more amino acids. A mutation can be a point mutation. A mutation can be a fusion gene. A fusion pair or a fusion gene can result from a mutation, such as a translocation, an interstitial deletion, a chromosomal inversion, or any combination thereof. A mutation can constitute variability in the number of repeated sequences, such as triplications, quadruplications, or others. For example, a mutation can be an increase or a decrease in a copy number associated with a given sequence (*i.e*., copy number variation, or CNV). A mutation can include two or more sequence changes in different alleles or two or more sequence changes in one allele. A mutation can include two different nucleotides at one position in one allele, such as a mosaic. A mutation can include two different nucleotides at one position in one allele, such as a chimeric. A mutation can be present in a malignant tissue. A presence or an absence of a mutation can indicate an increased risk to develop a disease or condition. A presence or an absence of a mutation can indicate a presence of a disease or condition. A mutation can be present in a benign tissue. Absence of a mutation can indicate that a tissue or sample is benign. As an alternative, absence of a mutation may not indicate that a tissue or sample is benign. Methods as described herein can comprise identifying a presence of a mutation in a sample.

The term "non-canonical amino acids" can refer to those synthetic or otherwise modified amino acids that fall outside this group, typically generated by chemical synthesis or modification of canonical amino acids (*e.g*. amino acid analogs). The disclosure employs proteinogenic non-canonical amino acids in some of the methods and vectors disclosed herein. A non-limiting exemplary non-canonical amino acid is pyrrolysine (Pyl or O), the chemical structure of which is provided below:

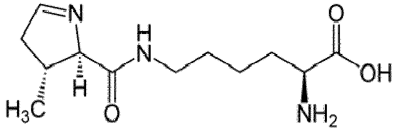

Inosine (I) is another exemplary non-canonical amino acid, which can be found in tRNA and is essential for proper translation according to "wobble base pairing." The structure of inosine is provided above.

The term "ornithine transcarbamylase" or "OTC" as used herein can refer to the protein corresponding with that name and encoded by the gene *Otc*; a non-limiting example of which is found under UniProt Reference Number P00480 (for humans) and P11725 (for mice). OTC deficiency is an X-linked genetic condition resulting in high concentrations of ammonia in blood. In some cases, OTC deficiency is caused by a G->A splice site mutation in the donor splice site of exon 4 that results in mis-splicing of the pre-mRNA. This mutation results in the formation of a protein that either is elongated or bears a point mutation. There is a 15-20 fold reduction in the OTC protein levels. See, *e.g.,* Hodges, P. E. & Rosenberg, L. E. The spfash mouse: a missense mutation in the ornithine transcarbamylase gene also causes aberrant mRNA splicing. Proc. Natl. Acad. Sci. U. S. A. 86, 4142-4146 (1989)) (showing the alternative forms of OTC produced). The sequences thereof are provided below:
*OTC pre-mRNA (wild type):*
   .....CTCACAGACACCGCTC**G**GTTTGTAAAACTTTTCTTC..... (SEQ ID NO: 144)
*OTC pre-mRNA (mutant):*
   .....*CTCACAGACACCGCTC**A**GTTTGTAAAACTTTTCTTC....* (SEQ ID NO: 145)
*OTC mRNA (incorrectly spliced, mutant):*
   .....CTCACAGACACCGCTC**A**GTTTGTAAAACTTTTCTTC.....(SEQ ID NO: 146)
OTC mRNA (correctly spliced, mutant):
   .....*CTCACAGACACCGCTC***A***TGTCTTATCTAGCATGACA.....* (SEQ ID NO: 147)
*OTC mRNA (correctly spliced, wild type):*
   .....CTCACAGACACCGCTC**G***TGTCTTATCTAGCATGACA*..... (SEQ ID NO: 148)

As shown above, a correct splice variant can be produced when the mutation is present; however, such production results in a missense mutation, which also can contribute to OTC deficiency.

The term "protein", "peptide" and "polypeptide" are used interchangeably and in their broadest sense to refer to a compound of two or more subunit amino acids, amino acid analogs or peptidomimetics. The subunits can be linked by peptide bonds. In another embodiment, the subunit can be linked by other bonds, *e.g.,* ester, ether, *etc.* A protein or peptide can contain at least two amino acids and no limitation is placed on the maximum number of amino acids which can comprise a protein's or peptide's sequence. As used herein the term "amino acid" can refer to either natural and/or unnatural or synthetic amino acids, including glycine and both the D and L optical isomers, amino acid analogs and peptidomimetics. As used herein, the term "fusion protein" can refer to a protein comprised of domains from more than one naturally occurring or recombinantly produced protein, where generally each domain serves a different function. In this regard, the term "linker" can refer to a protein fragment that is used to link these domains together - optionally to preserve the conformation of the fused protein domains and/or prevent unfavorable interactions between the fused protein domains which can compromise their respective functions.

The terms "polynucleotide" and "oligonucleotide" are used interchangeably and refer to a polymeric form of nucleotides of any length, either deoxyribonucleotides or ribonucleotides or analogs thereof. Polynucleotides can have any three-dimensional structure and can perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, RNAi, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also can refer to both double- and single-stranded molecules. Unless otherwise specified or required, any embodiment of this disclosure that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. In some embodiments, the polynucleotide can comprise one or more other nucleotide bases, , such as inosine (I), a nucleoside formed when hypoxanthine is attached to ribofuranose via a β-N9-glycosidic bond, resulting in the chemical structure:

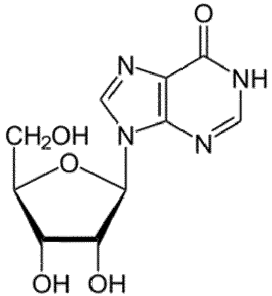

Inosine is read by the translation machinery as guanine (G).

The term "polynucleotide sequence" is the alphabetical representation of a polynucleotide molecule. This alphabetical representation can be input into databases in a computer having a central processing unit and used for bioinformatics applications such as functional genomics and homology searching.

As used herein, the term "purification marker" can refer to at least one marker useful for purification or identification. A non-exhaustive list of this marker includes His, lacZ, GST, maltose-binding protein, NusA, BCCP, c-myc, CaM, FLAG, GFP, YFP, cherry, thioredoxin, poly (NANP), V5, Snap, HA, chitin-binding protein, Softag 1, Softag 3, Strep, or S-protein. Suitable direct or indirect fluorescence marker comprise FLAG, GFP, YFP, RFP, dTomato, cherry, Cy3, Cy 5, Cy 5.5, Cy 7, DNP, AMCA, Biotin, Digoxigenin, Tamra, Texas Red, rhodamine, Alexa fluors, FITC, TRITC or any other fluorescent dye or hapten.

As used herein, the term "recombinant expression system" refers to a genetic construct or constructs for the expression of certain genetic material formed by recombination; the term "construct" in this regard is interchangeable with the term "vector" as defined herein.

As used herein, the term "recombinant protein" can refer to a polypeptide which is produced by recombinant DNA techniques, wherein generally, DNA encoding the polypeptide is inserted into a suitable expression vector which is in turn used to transform a host cell to produce the heterologous protein.

As used herein the term "restoring" in relation to expression of a protein can refer to the ability to establish expression of full length protein where previously protein expression was truncated due to mutation. In the context of "restoring activity" the term includes effecting the expression of a protein to its normal, non-mutated levels where a mutation resulted in aberrant expression (e.g., too low or too high).

The term "sample" as used herein, generally refers to any sample of a subject (such as a blood sample or a tissue sample). A sample or portion thereof can comprise a stem cell. A portion of a sample can be enriched for the stem cell. The stem cell can be isolated from the sample. A sample can comprise a tissue, a cell, serum, plasma, exosomes, a bodily fluid, or any combination thereof. A bodily fluid can comprise urine, blood, serum, plasma, saliva, mucus, spinal fluid, tears, semen, bile, amniotic fluid, or any combination thereof. A sample or portion thereof can comprise an extracellular fluid obtained from a subject. A sample or portion thereof can comprise cell-free nucleic acid, DNA or RNA. A sample or portion thereof can be analyzed for a presence or absence or one or more mutations. Genomic data can be obtained from the sample or portion thereof. A sample can be a sample suspected or confirmed of having a disease or condition. A sample can be a sample removed from a subject via a non-invasive technique, a minimally invasive technique, or an invasive technique. A sample or portion thereof can be obtained by a tissue brushing, a swabbing, a tissue biopsy, an excised tissue, a fine needle aspirate, a tissue washing, a cytology specimen, a surgical excision, or any combination thereof. A sample or portion thereof can comprise tissues or cells from a tissue type. For example, a sample can comprise a nasal tissue, a trachea tissue, a lung tissue, a pharynx tissue, a larynx tissue, a bronchus tissue, a pleura tissue, an alveoli tissue, breast tissue, bladder tissue, kidney tissue, liver tissue, colon tissue, thyroid tissue, cervical tissue, prostate tissue, heart tissue, muscle tissue, pancreas tissue, anal tissue, bile duct tissue, a bone tissue, brain tissue, spinal tissue, kidney tissue, uterine tissue, ovarian tissue, endometrial tissue, vaginal tissue, vulvar tissue, uterine tissue, stomach tissue, ocular tissue, sinus tissue, penile tissue, salivary gland tissue, gut tissue, gallbladder tissue, gastrointestinal tissue, bladder tissue, brain tissue, spinal tissue, a blood sample, or any combination thereof.

The term "sequencing" as used herein, can comprise bisulfite-free sequencing, bisulfite sequencing, TET-assisted bisulfite (TAB) sequencing, ACE-sequencing, highthroughput sequencing, Maxam-Gilbert sequencing, massively parallel signature sequencing, Polony sequencing, 454 pyrosequencing, Sanger sequencing, Illumina sequencing, SOLiD sequencing, Ion Torrent semiconductor sequencing, DNA nanoball sequencing, Heliscope single molecule sequencing, single molecule real time (SMRT) sequencing, nanopore sequencing, shot gun sequencing, RNA sequencing, Enigma sequencing, or any combination thereof.

The term "stop codon" intends a three nucleotide contiguous sequence within messenger RNA that signals a termination of translation. Non-limiting examples include in RNA, UAG, UAA, UGA and in DNA TAG, TAA or TGA. Unless otherwise noted, the term also includes nonsense mutations within DNA or RNA that introduce a premature stop codon, causing any resulting protein to be abnormally shortened. tRNA that correspond to the various stop codons are known by specific names: amber (UAG), ochre (UAA), and opal (UGA).

"Transfer ribonucleic acid" or "tRNA" is a nucleic acid molecule that helps translate mRNA to protein. tRNA have a distinctive folded structure, comprising three hairpin loops; one of these loops comprises a "stem" portion that encodes an anticodon. The anticodon recognizes the corresponding codon on the mRNA. Each tRNA is "charged with" an amino acid corresponding to the mRNA codon; this "charging" is accomplished by the enzyme tRNA synthetase. Upon tRNA recognition of the codon corresponding to its anticodon, the tRNA transfers the amino acid with which it is charged to the growing amino acid chain to form a polypeptide or protein. Endogenous tRNA can be charged by endogenous tRNA synthetase. Accordingly, endogenous tRNA are typically charged with canonical amino acids. Orthogonal tRNA, derived from an external source, require a corresponding orthogonal tRNA synthetase. Such orthogonal tRNAs may be charged with both canonical and non-canonical amino acids. In some embodiments, the amino acid with which the tRNA is charged may be detectably labeled to enable detection *in vivo.* Techniques for labeling are known in the art and include, but are not limited to, click chemistry wherein an azide/alkyne containing unnatural amino acid is added by the orthogonal tRNA/synthetase pair and, thus, can be detected using alkyne/azide comprising fluorophore or other such molecule.

As used herein, the terms "treating," "treatment" and the like are used herein to mean obtaining a desired pharmacologic and/or physiologic effect. The effect can be prophylactic in terms of completely or partially preventing a disease, disorder, or condition or sign or symptom thereof, and/or can be therapeutic in terms of a partial or complete cure for a disorder and/or adverse effect attributable to the disorder.

As used herein, the term "vector" can refer to a nucleic acid construct deigned for transfer between different hosts, including but not limited to a plasmid, a virus, a cosmid, a phage, a BAC, a YAC, *etc.* A "viral vector" is defined as a recombinantly produced virus or viral particle that comprises a polynucleotide to be delivered into a host cell, either *in vivo, ex vivo* or *in vitro.* In some embodiments, plasmid vectors can be prepared from commercially available vectors. In other embodiments, viral vectors can be produced from baculoviruses, retroviruses, adenoviruses, AAVs, *etc.* according to techniques known in the art. In one embodiment, the viral vector is a lentiviral vector. Examples of viral vectors include retroviral vectors, adenovirus vectors, adeno-associated virus vectors, alphavirus vectors and the like. Infectious tobacco mosaic virus (TMV)-based vectors can be used to manufacturer proteins and have been reported to express Griffithsin in tobacco leaves (O'Keefe et al. (2009) Proc. Nat. Acad. Sci. USA 106(15):6099-6104). Alphavirus vectors, such as Semliki Forest virus-based vectors and Sindbis virus-based vectors, have also been developed for use in gene therapy and immunotherapy. See, Schlesinger & Dubensky (1999) Curr. Opin. Biotechnol. 5:434-439 and Ying et al. (1999) Nat. Med. 5(7):823-827. In aspects where gene transfer is mediated by a retroviral vector, a vector construct can refer to the polynucleotide comprising the retroviral genome or part thereof, and a gene of interest. Further details as to modern methods of vectors for use in gene transfer can be found in, for example, Kotterman et al. (2015) Viral Vectors for Gene Therapy: Translational and Clinical Outlook Annual Review of Biomedical Engineering 17. Vectors that contain both a promoter and a cloning site into which a polynucleotide can be operatively linked are well known in the art. Such vectors are capable of transcribing RNA *in vitro* or *in vivo* and are commercially available from sources such as Agilent Technologies (Santa Clara, Calif.) and Promega Biotech (Madison, Wis.). In one aspect, the promoter is a pol III promoter.

The pharmaceutical compositions for the administration of the AdRNA can be conveniently presented in dosage unit form and can be prepared by any of the methods well known in the art of pharmacy. The pharmaceutical compositions can be, for example, prepared by uniformly and intimately bringing the compounds provided herein into association with a liquid carrier, a finely divided solid carrier or both, and then, if necessary, shaping the product into the desired formulation. In the pharmaceutical composition the compound provided herein is included in an amount sufficient to produce the desired therapeutic effect. For example, pharmaceutical compositions of the technology can take a form suitable for virtually any mode of administration, including, for example, topical, ocular, oral, buccal, systemic, nasal, injection, infusion, transdermal, rectal, and vaginal, or a form suitable for administration by inhalation or insufflation.

For topical administration, the compounds can be formulated as solutions, gels, ointments, creams, suspensions, *etc.,* as is well-known in the art.

Systemic formulations include those designed for administration by injection (e.g., subcutaneous, intravenous, infusion, intramuscular, intrathecal, or intraperitoneal injection) as well as those designed for transdermal, transmucosal, oral, or pulmonary administration.

Useful injectable preparations include sterile suspensions, solutions, or emulsions of the compounds provided herein in aqueous or oily vehicles. The compositions can also contain formulating agents, such as suspending, stabilizing, and/or dispersing agents. The formulations for injection can be presented in unit dosage form, *e.g.,* in ampules or in multidose containers, and can contain added preservatives.

Alternatively, the injectable formulation can be provided in powder form for reconstitution with a suitable vehicle, including but not limited to sterile pyrogen free water, buffer, and dextrose solution, before use. To this end, the compounds provided herein can be dried by any art-known technique, such as lyophilization, and reconstituted prior to use.

For transmucosal administration, penetrants appropriate to the barrier to be permeated are used in the formulation. Such penetrants are known in the art.

For oral administration, the pharmaceutical compositions can take the form of, for example, lozenges, tablets, or capsules prepared by conventional means with pharmaceutically acceptable excipients such as binding agents (*e.g*., pregelatinised maize starch, polyvinylpyrrolidone, or hydroxypropyl methyl cellulose); fillers (*e.g*., lactose, microcrystalline cellulose, or calcium hydrogen phosphate); lubricants (*e.g*., magnesium stearate, talc, or silica); disintegrants (*e.g*., potato starch or sodium starch glycolate); or wetting agents (*e.g*., sodium lauryl sulfate). The tablets can be coated by methods well known in the art with, for example, sugars, films, or enteric coatings.

Compositions intended for oral use can be prepared according to any method known to the art for the manufacture of pharmaceutical compositions, and such compositions can contain one or more agents selected from the group consisting of sweetening agents, flavoring agents, coloring agents, and preserving agents in order to provide pharmaceutically elegant and palatable preparations. Tablets contain the compounds provided herein in admixture with non-toxic pharmaceutically acceptable excipients which are suitable for the manufacture of tablets. These excipients can be for example, inert diluents, such as calcium carbonate, sodium carbonate, lactose, calcium phosphate or sodium phosphate; granulating and disintegrating agents (*e.g.,* corn starch or alginic acid); binding agents (*e.g.* starch, gelatin, or acacia); and lubricating agents (*e.g*., magnesium stearate, stearic acid, or talc). The tablets can be left uncoated or they can be coated by known techniques to delay disintegration and absorption in the gastrointestinal tract and thereby provide a sustained action over a longer period. For example, a time delay material such as glyceryl monostearate or glyceryl distearate can be employed. They can also be coated by the techniques well known to the skilled artisan. The pharmaceutical compositions of the technology can also be in the form of oil-in-water emulsions.

Liquid preparations for oral administration can take the form of, for example, elixirs, solutions, syrups, or suspensions, or they can be presented as a dry product for constitution with water or other suitable vehicle before use. Such liquid preparations can be prepared by conventional means with pharmaceutically acceptable additives such as suspending agents (*e.g.*, sorbitol syrup, cellulose derivatives, or hydrogenated edible fats); emulsifying agents (*e.g*., lecithin, or acacia); non-aqueous vehicles (*e.g*., almond oil, oily esters, ethyl alcohol, cremophore^{™}, or fractionated vegetable oils); and preservatives (*e.g*., methyl or propyl-p-hydroxybenzoates or sorbic acid). The preparations can also contain buffer salts, preservatives, flavoring, coloring, and sweetening agents as appropriate.

"Administration" can be effected in one dose, continuously or intermittently throughout the course of treatment. Methods of determining the most effective means and dosage of administration are known to those of skill in the art and can vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents are known in the art. Route of administration can also be determined and method of determining the most effective route of administration are known to those of skill in the art and can vary with the composition used for treatment, the purpose of the treatment, the health condition or disease stage of the subject being treated, and target cell or tissue. Non-limiting examples of route of administration include oral administration, nasal administration, injection, and topical application.

Administration can refer to methods that can be used to enable delivery of compounds or compositions to the desired site of biological action (such an DNA constructs, viral vectors, or others). These methods can include topical administration (such as a lotion, a cream, an ointment) to an external surface of a surface, such as a skin. These methods can include parenteral administration (including intravenous, subcutaneous, intrathecal, intraperitoneal, intramuscular, intravascular or infusion), oral administration, inhalation administration, intraduodenal administration, rectal administration. In some instances, a subject can administer the composition in the absence of supervision. In some instances, a subject can administer the composition under the supervision of a medical professional (*e.g*., a physician, nurse, physician's assistant, orderly, hospice worker, *etc*.). In some cases, a medical professional can administer the composition. In some cases, a cosmetic professional can administer the composition.

Administration or application of a composition disclosed herein can be performed for a treatment duration of at least about at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 days consecutive or nonconsecutive days. In some cases, a treatment duration can be from about 1 to about 30 days, from about 2 to about 30 days, from about 3 to about 30 days, from about 4 to about 30 days, from about 5 to about 30 days, from about 6 to about 30 days, from about 7 to about 30 days, from about 8 to about 30 days, from about 9 to about 30 days, from about 10 to about 30 days, from about 11 to about 30 days, from about 12 to about 30 days, from about 13 to about 30 days, from about 14 to about 30 days, from about 15 to about 30 days, from about 16 to about 30 days, from about 17 to about 30 days, from about 18 to about 30 days, from about 19 to about 30 days, from about 20 to about 30 days, from about 21 to about 30 days, from about 22 to about 30 days, from about 23 to about 30 days, from about 24 to about 30 days, from about 25 to about 30 days, from about 26 to about 30 days, from about 27 to about 30 days, from about 28 to about 30 days, or from about 29 to about 30 days.

Administration or application of composition disclosed herein can be performed at least 1, 2, 3, 4, 5, 6, 7, 8, 9, 10, 11, 12, 13, 14, 15, 16, 17, 18, 19, 20, 21, 22, 23, or 24 times a day. In some cases, administration or application of composition disclosed herein can be performed at least 1, 2, 3, 4, 5, 6, 7, 8, 9, 10, 11, 12, 13, 14, 15, 16, 17, 18, 19, 20, or 21 times a week. In some cases, administration or application of composition disclosed herein can be performed at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 times a month.

In some cases, a composition can be administered/applied as a single dose or as divided doses. In some cases, the compositions described herein can be administered at a first time point and a second time point. In some cases, a composition can be administered such that a first administration is administered before the other with a difference in administration time of 1 hour, 2 hours, 4 hours, 8 hours, 12 hours, 16 hours, 20 hours, 1 day, 2 days, 4 days, 7 days, 2 weeks, 4 weeks, 2 months, 3 months, 4 months, 5 months, 6 months, 7 months, 8 months, 9 months, 10 months, 11 months, 1 year or more.

The term "effective amount" can refer to a quantity sufficient to achieve a desired effect. In the context of therapeutic or prophylactic applications, the effective amount will depend on the type and severity of the condition at issue and the characteristics of the individual subject, such as general health, age, sex, body weight, and tolerance to pharmaceutical compositions. In the context of an immunogenic composition, in some embodiments the effective amount is the amount sufficient to result in a protective response against a pathogen. In other embodiments, the effective amount of an immunogenic composition is the amount sufficient to result in antibody generation against the antigen. In some embodiments, the effective amount is the amount required to confer passive immunity on a subject in need thereof. With respect to immunogenic compositions, in some embodiments the effective amount can depend on the intended use, the degree of immunogenicity of a particular antigenic compound, and the health/responsiveness of the subject's immune system, in addition to the factors described above. The skilled artisan can determine appropriate amounts depending on these and other factors.

In the case of an *in vitro* application, in some embodiments the effective amount can depend on the size and nature of the application in question. It can also depend on the nature and sensitivity of the *in vitro* target and the methods in use. The skilled artisan can determine the effective amount based on these and other considerations. The effective amount can comprise one or more administrations of a composition depending on the embodiment.

It is to be inferred without explicit recitation and unless otherwise intended, that when the disclosure relates to a polypeptide, protein, polynucleotide or antibody, an equivalent or a biologically equivalent of such is intended within the scope of this disclosure. As used herein, the term "biological equivalent thereof' is intended to be synonymous with "equivalent thereof' when referring to a reference protein, antibody, polypeptide or nucleic acid, intends those having minimal homology while still maintaining desired structure or functionality. Unless specifically recited herein, it is contemplated that any polynucleotide, polypeptide or protein mentioned herein also includes equivalents thereof. For example, an equivalent intends at least about 70% homology or identity, or at least 80 % homology or identity and alternatively, or at least about 85 %, or alternatively at least about 90 %, or alternatively at least about 95 %, or alternatively 98 % percent homology or identity and exhibits substantially equivalent biological activity to the reference protein, polypeptide or nucleic acid. Alternatively, when referring to polynucleotides, an equivalent thereof is a polynucleotide that hybridizes under stringent conditions to the reference polynucleotide or its complement.

The disclosure provides polypeptide and/or polynucleotide sequences for use in gene and protein editing techniques described below. It should be understood, although not always explicitly stated that the sequences provided herein can be used to provide the expression product as well as substantially identical sequences that produce a protein that has the same biological properties. These "biologically equivalent" or "biologically active" polypeptides are encoded by equivalent polynucleotides as described herein. They can possess at least 60%, or alternatively, at least 65%, or alternatively, at least 70%, or alternatively, at least 75%, or alternatively, at least 80%, or alternatively at least 85%, or alternatively at least 90%, or alternatively at least 95% or alternatively at least 98%, identical primary amino acid sequence to the reference polypeptide when compared using sequence identity methods run under default conditions. Specific polypeptide sequences are provided as examples of particular embodiments. Modifications to the sequences to amino acids with alternate amino acids that have similar charge. Additionally, an equivalent polynucleotide is one that hybridizes under stringent conditions to the reference polynucleotide or its complement or in reference to a polypeptide, a polypeptide encoded by a polynucleotide that hybridizes to the reference encoding polynucleotide under stringent conditions or its complementary strand. Alternatively, an equivalent polypeptide or protein is one that is expressed from an equivalent polynucleotide.

A "composition" typically intends a combination of the active agent, e.g., an adRNA of this disclosure, a compound or composition, and a naturally-occurring or non-naturally-occurring carrier, inert (for example, a detectable agent or label) or active, such as an adjuvant, diluent, binder, stabilizer, buffers, salts, lipophilic solvents, preservative, adjuvant or the like and include pharmaceutically acceptable carriers. Carriers also include pharmaceutical excipients and additives proteins, peptides, amino acids, lipids, and carbohydrates (*e.g*., sugars, including monosaccharides, di-, tri-, tetra-oligosaccharides, and oligosaccharides; derivatized sugars such as alditols, aldonic acids, esterified sugars and the like; and polysaccharides or sugar polymers), which can be present singly or in combination, comprising alone or in combination 1-99.99% by weight or volume. Exemplary protein excipients include serum albumin such as human serum albumin (HSA), recombinant human albumin (rHA), gelatin, casein, and the like. Representative amino acid/antibody components, which can also function in a buffering capacity, include alanine, arginine, glycine, arginine, betaine, histidine, glutamic acid, aspartic acid, cysteine, lysine, leucine, isoleucine, valine, methionine, phenylalanine, aspartame, and the like. Carbohydrate excipients are also intended within the scope of this technology, examples of which include but are not limited to monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol.

The compositions used in accordance with the disclosure, including cells, treatments, therapies, agents, drugs and pharmaceutical formulations can be packaged in dosage unit form for ease of administration and uniformity of dosage. The term "unit dose" or "dosage" can refer to physically discrete units suitable for use in a subject, each unit containing a predetermined quantity of the composition calculated to produce the desired responses in association with its administration, *i.e*., the appropriate route and regimen. The quantity to be administered, both according to number of treatments and unit dose, depends on the result and/or protection desired. Precise amounts of the composition also depend on the judgment of the practitioner and are peculiar to each individual. Factors affecting dose include physical and clinical state of the subject, route of administration, intended goal of treatment (alleviation of symptoms versus cure), and potency, stability, and toxicity of the particular composition. Upon formulation, solutions can be administered in a manner compatible with the dosage formulation and in such amount as is therapeutically or prophylactically effective. The formulations are easily administered in a variety of dosage forms, such as the type of injectable solutions described herein.

As used herein, the term "reduce or eliminate expression and/or function of' can refer to reducing or eliminating the transcription of said polynucleotides into mRNA, or alternatively reducing or eliminating the translation of said mRNA into peptides, polypeptides, or proteins, or reducing or eliminating the functioning of said peptides, polypeptides, or proteins. In a non-limiting example, the transcription of polynucleotides into mRNA is reduced to at least half of its normal level found in wild type cells.

The phrase "first line" or "second line" or "third line" can refer to the order of treatment received by a patient. First line therapy regimens are treatments given first, whereas second or third line therapy are given after the first line therapy or after the second line therapy, respectively. The National Cancer Institute defines first line therapy as "the first treatment for a disease or condition. In patients with cancer, primary treatment can be surgery, chemotherapy, radiation therapy, or a combination of these therapies. First line therapy is also referred to those skilled in the art as "primary therapy and primary treatment." See National Cancer Institute website at cancer.gov, last visited November 15, 2017. Typically, a patient is given a subsequent chemotherapy regimen because the patient did not show a positive clinical or sub-clinical response to the first line therapy or the first line therapy has stopped.

The term "contacting" means direct or indirect binding or interaction between two or more entities. A particular example of direct interaction is binding. A particular example of an indirect interaction is where one entity acts upon an intermediary molecule, which in turn acts upon the second referenced entity. Contacting as used herein includes in solution, in solid phase, *in vitro, ex vivo,* in a cell and *in vivo.* Contacting *in vivo* can be referred to as administering, or administration.

"Cryoprotectants" are known in the art and include without limitation, *e.g*., sucrose, trehalose, and glycerol. A cryoprotectant exhibiting low toxicity in biological systems is generally used.

Disclosed herein are adRNAs for site-specific editing of RNA in the absence of overexpression of the ADAR enzymes. Further provided herein is engineering A -> G editing of DNA. In addition, provided herein is screening for ADAR2 mutants that enable site-specific C ->T editing of RNA and DNA. Still further provided herein is engineering C->T edits of RNA via the use of APOBEC1 expressed along with ACF.

Compared to other ADAR2 systems, the disclosure is unique as it presents a novel method of recruitment of endogenous ADARs to catalyze therapeutic RNA editing. In addition, none of the prior art systems offer a means to use ADAR enzymes for engineering C -> T edits. Lastly, they do not disclose the use of APOBEC for programmable site-specific RNA editing.

Disclosed herein is an exemplary adRNA comprises an RNA targeting domain, complementary to the target RNA and one or more ADAR recruiting domain. When bound to its target, the adRNA is able to recruit the ADAR enzyme to the target RNA. This ADAR enzyme is then able to catalyze the conversion of a target adenosine to inosine. Not to be bound by theory, it is believed that adRNA can be used analogously to recruit one of the ADAR2 mutants or APOBEC1 to affect C -> T RNA editing.

Also disclosed herein, both *in vitro* and *in vivo* experiments have been carried out using the engineered adRNA to recruit the endogenous ADAR enzymes. Also disclosed herein are experiments showing C->T editing efficiencies of ADAR mutants as well as the APOBEC1/ACF constructs.

A viral vector as described herein can comprise a nucleic acid sequence encoding for at least one RNA editing entity recruiting domain. In some cases, a nucleic acid sequence can encode for more than one RNA editing entity recruiting domain, such as 2, 3, 4 or more. An RNA editing entity recruiting domain can comprise at least about 80% sequence identity to at least one of: an Alu domain, an Apolipoprotein B mRNA Editing Catalytic Polypeptide-like (APOBEC) recruiting domain, a Cas13 domain, a GluR2 domain, or any combination thereof. The recruiting domain can comprise one or more GluR2 domains, one or more Alu domains, one or more APOBEC domains, or any combination thereof. The recruiting domain can comprise more than one GluR2 domain, more than one Alu domain, more than one APOBEC domain, Cas13 domain, or any combination thereof. The recruiting domain may not comprise an Alu domain. The recruiting domain may not comprise an GluR2 domain. The recruiting domain may not comprise an APOBEC domain. The recruiting domain may not comprise a Cast3 domain.

An APOBEC recruiting domain can comprise an APOBEC1 recruiting domain, APOBEC2 recruiting domain, APOBEC3A recruiting domain, APOBEC3B recruiting domain, APOBEC3C recruiting domain, APOBEC3D recruiting domain, APOBEC3E recruiting domain, APOBEC3F recruiting domain, APOBEC3G recruiting domain, APOBEC3H recruiting domain, APOBEC4 recruiting domain, a derivative of any of these, or any combination thereof.

A recruiting domain can comprise at least about 80% sequence identity to any one of the Alu domains as described herein. In some cases, the recruiting domain can comprise at least about: 85%, 90%, 95%, 97%, 98%, or 99% sequence identity to any one of the Alu domains as described herein.

A recruiting domain can comprise at least about 80% sequence identity to any one of the APOBEC domains as described herein. In some cases, the recruiting domain can comprise at least about: 85%, 90%, 95%, 97%, 98%, or 99% sequence identity to any one of the APOBEC domains as described herein.

A recruiting domain can comprise at least about 80% sequence identity to any one of the GluR2 domains as described herein. In some cases, the recruiting domain can comprise at least about: 85%, 90%, 95%, 97%, 98%, or 99% sequence identity to any one of the GluR2 domains as described herein.

A recruiting domain can comprise at least about 80% sequence identity to any one of the Cast3 domains as described herein. In some cases, the recruiting domain can comprise at least about: 85%, 90%, 95%, 97%, 98%, or 99% sequence identity to any one of the Cas13 domains as described herein.

A nucleic acid sequence can encode for at least 1, 2, 3, 4, 5, RNA editing recruiting domains. A nucleic acid sequence can encode for at least 2 RNA editing recruiting domains, wherein one is an Alu domain. A nucleic acid sequence can encode for at least 2 RNA editing recruiting domains, wherein one is an APOBEC domain. A nucleic acid sequence can encode for at least 2 RNA editing recruiting domains, wherein one is a GluR2 domain. A nucleic acid sequence can encode for at least 2 RNA editing recruiting domains, wherein one is a Cas13 domain.

A recruiting domain can comprise one or more stem loop structures. A recruiting domain can comprise at least 2 stem loop structures. A recruiting domain can comprise at least 3 stem loop structures. A recruiting domain may not comprise a stem loop structure. A recruiting domain that comprises at least one stem loop structure can be an Alu domain, an APOBEC domain, a GluR2 domain, Cast3 domain, or any combination thereof.

At least a portion of a recruiting domain can be single stranded. In some cases, an Alu domain can be at least partially single stranded. In some cases, an APOBEC domain can be at least partially single stranded. In some cases, an GluR2 domain can be at least partially single stranded. In some cases, a Cas13 domain can be at least partially single stranded.

A recruiting domain can comprise a plurality of repeats. A recruiting domain can comprise a plurality of Alu repeats.

In some cases, a viral vector can comprise one or more RNA editing recruiting domains. In some cases, a viral vector can comprise more than one RNA editing recruiting domain. In some cases, a viral vector can comprise 2, 3, 4, 5 or more RNA editing recruiting domains. A nucleic acid sequence can encode for one or more RNA editing recruiting domains. A nucleic acid sequence can encode for more than one RNA editing recruiting domain. A nucleic acid sequence can encode for 2, 3, 4, 5 or more RNA editing recruiting domains. A nucleic acid sequence can encode for at least an Alu domain and a GluR2 domain. A nucleic acid sequence can encode for at least an Alu domain and a Cas13 domain. A nucleic acid sequence can encode for at least an Alu domain and an APOBEC domain. A nucleic acid sequence can encode for at least a GluR2 domain and an APOBEC domain. A nucleic acid sequence can encode for at least a GluR2 domain and an Cas13 domain. A nucleic acid sequence can encode for at least a Cas13 domain and an APOBEC domain.

A nucleic acid sequence can encode for a target RNA that can be complementary to at least a portion of a target RNA. It can be complementary to at least a portion of that target RNA. The portion that can be complementary can be from about 50 basepairs (bp) to about 200 bp in length. The portion that can be complementary can be from about 20 bp to about 100 bp in length. The portion that can be complementary can be from about 10 bp to about 50 bp in length. The portion that can be complementary can be from about 50 bp to about 300 bp in length. Modifying a length of the portion that is complementary can enhance efficiency of editing. In some cases, longer lengths of the portion can enhance efficiency of editing as compared to shorter lengths.

A nucleic acid sequence can encode for at least one RNA editing entity recruiting domain and a nucleic acid sequence encoding for an RNA that can be complementary to at least a portion of a target RNA and comprise a contiguous nucleic acid sequence of at least about 200 bp in length. The contiguous nucleic acid sequence can comprise a length from about 100 bp to about 300 bp in length. The contiguous nucleic acid sequence can comprise a length from about 150 bp to about 400 bp in length. The contiguous nucleic acid sequence can comprise a length from about 200 bp to about 500 bp in length. The contiguous nucleic acid sequence can comprise a length from about 50 bp to about 300 bp in length. Modifying a length of the contiguous nucleic acid sequence can enhance efficiency of editing. In some cases, longer lengths of the contiguous sequence can enhance efficiency of editing as compared to shorter lengths.

A nucleic acid can comprise a linker sequence, such as a linker sequence positioned between a targeting domain and a recruiting domain. In some cases, a nucleic acid can comprise a sequence such as 5'-X-(Y-X')n- L-Z-3', wherein X is complementary to the target RNA sequence downstream of the specific position, X' is complementary to the target RNA sequence upstream of the specific position, Y comprises one or more nucleotides which may not be complementary to the target RNA sequence, n can be an integer from 1 to 10, L can be a linker sequence comprising any number of nucleotides (including zero), and Z can be a sequence that is recognized by and binds to the RNA editing entity. L can also consist of a different chemical linkage, such as a (oligo)peptide linkage, or PEG linkage.

A nucleic acid can comprise between 20 and several hundred nucleotides. In some cases, longer targeting portions provide more specificity for the target site of the RNA sequence to be edited, less off-target effects due to unintentional (off-target) binding as well as more room to create secondary structures, such as stem-loop structures, cruciforms, toe hold structures, within the targeting portion itself, mismatches or wobble-bases (due to mismatches with one or more of the complementary base(s) in the targeted RNA sequence at or near the site to be edited), and so forth. In some cases, targeting portions can be complementary to the target RNA sequence over the entire length of the targeting portion except for the mismatch opposite the nucleotide to be edited, and optionally one or two wobble bases.

Nucleic acids can be modified using various chemistries and modifications. In some cases, regular internucleosidic linkages between nucleotides can be altered by mono- or di-thioation of the phosphodiester bonds to yield phosphorothioate esters or phosphorodithioate esters, respectively. Other modifications of the internucleosidic linkages can include amidation or peptide linkers. A ribose sugar can be modified by substitution of the 2'-O moiety with a lower alkyl (C1 -4, such as 2'- O-Me), alkenyl (C2-4), alkynyl (C2-4), methoxyethyl (2'-MOE), or other substituent. In some cases, substituents of the 2' OH group can comprise a methyl, methoxyethyl or 3,3'-dimethylallyl group. In some cases, locked nucleic acid sequences (LNAs), comprising a 2'-4' intramolecular bridge (such as a methylene bridge between the 2' oxygen and 4' carbon) linkage inside the ribose ring, can be applied. Purine nucleobases and/or pyrimidine nucleobases can be modified to alter their properties, for example by amination or deamination of the heterocyclic rings.

A viral vector can be an adeno-associated virus (AAV) vector. An AAV can be a recombinant AAV. An AAV can comprise an AAV1 serotype, an AAV2 serotype, an AAV3 serotype, an AAV4 serotype, an AAV5 serotype, an AAV6 serotype, an AAV7 serotype, an AAV8 serotype, an AAV9 serotype, a derivative of any of these, or any combination thereof. An AAV can be selected from the group consisting of: an AAV1 serotype, an AAV2 serotype, an AAV3 serotype, an AAV4 serotype, an AAV5 serotype, an AAV6 serotype, an AAV7 serotype, an AAV8 serotype, an AAV9 serotype, a derivative of any of these, and any combination thereof., A viral vector can be a modified viral vector. A viral vector can be modified to include a modified protein. In some cases, a viral vector can comprise a modified VP1 protein.

A nucleic acid sequence, that encodes for an RNA editing entity recruiting domain, a targeting domain, or a combination thereof, can comprise a structure (such as a secondary structure) that can be substantially a cruciform. A nucleic acid sequence can comprise at least two structures that can be substantially cruciforms. A recruiting domain can comprise a structure that can be substantially a cruciform. A recruiting domain can comprise at least two structures that can be substantially cruciforms. A secondary structure of a nucleic acid sequence (such as a portion encoding a recruiting domain) can be modified to enhance recruitment or binding of an ADAR. Modification of structure to enhance recruitment or binding of an ADAR can include forming cruciform structures.

An RNA editing entity recruiting domain can be positioned between at least two structures that can be substantially cruciforms. A targeting domain can be positioned between at least two structures that can be substantially cruciforms. An RNA editing entity recruiting domain can be positioned flanked by at least one structure that can be substantially a cruciform. A targeting domain can be positioned flanked by at least one structure that can be substantially a cruciform.

A cruciform structure can comprise a stem loop adjoining at least one pair of at least partially complementary strands of a cruciform structure. A cruciform structure can be substantially a cruciform. A cruciform structure can comprise less than substantially a cruciform, such as 3 of 4 stem loops, or 2 of 4 stem loops. One or more stem loops that can form a cruciform can comprise a different length. One or more stem loops that can form a cruciform can comprise a same length. One or more stem loops that can form a cruciform can comprise one or more mismatch bulges.

An RNA editing entity recruiting domain can comprise a structure that can be substantially a toehold. An RNA editing entity can comprise one or more mismatch bulges. An RNA editing entity may not comprise a mismatch bulge. An RNA editing entity recruiting domain can comprise a substantially toehold structure, a substantially cruciform structure, a substantially linear structure, a stem loop structure, a double stem loop structure, or a combination thereof.

A viral vector can comprise a nucleic acid sequence encoding for an RNA with a two dimensional shape. The two dimensional shape can convey superior recruitment or binding of ADAR as compared to an RNA with a different two dimensional shape. A sequence of a nucleic acid sequence that encodes for an RNA can be modified such that the RNA comprises a two dimensional shape that conveys superior recruitment or binding of ADAR. The two dimensional shape can be substantially a cruciform, a toehold, a stem loop, or any combination thereof. The two dimensional shape can comprise the substantially a cruciform. The two dimensional shape can comprise the toehold. The two dimensional shape can comprise the stem loop. The two dimensional shape can be linear.

An RNA encoded by a nucleic acid sequence can comprise a first domain and a second domain. A first domain can comprise a cruciform and a second domain can comprise a linear structure. The first and second domain can be directly or indirectly connected. The first domain can be a recruiting domain and the second domain can be a targeting domain. The RNA can comprise a third domain. The third domain can be directly or indirectly connected to the first or second domains. The third domain can be a recruiting domain. The third domain can comprise a cruciform structure.

An RNA encoded by a nucleic acid sequence can be a non-naturally occurring RNA. An RNA encoded by a nucleic acid sequence can comprise at least one base or at least one sugar comprises a chemical modification. An RNA can comprise two or more chemical modifications. A chemical modification can increase a stability of an RNA, such has a bioactive half-life of the RNA *in vivo.*

A nucleic acid can comprise one or more recruiting domains and one or more antisense domains. When the nucleic acid is contacted with an RNA editing entity and a target nucleic acid complementary to at least a portion of the antisense domain, it can modify at least one base pair of the target nucleic acid at an efficiency of at least about: 2x, 2.5x, 3x, 3.5x, 4x, 4.5x, 5x, 5.5x, or 6x greater than a comparable nucleic acid complexed with a Cas13b protein or an active fragment thereof, as determined by a sequence methods (such as Sanger method). The efficiency can be at least about 3x greater. The efficiency can be at least about 4x greater. The efficiency can be at least about 5x greater.

A nucleic acid can comprise one or more recruiting domains and one or more antisense domains. When the nucleic acid is contacted with an RNA editing entity and a target nucleic acid complementary to at least a portion of the antisense domain, it can modify at least one base pair of the target nucleic acid at an efficiency of at least about: 2x, 2.5x, 3x, 3.5x, 4x, 4.5x, 5x, 5.5x, or 6x greater than a comparable nucleic acid complexed with a GluR2 protein or an active fragment thereof, as determined by a sequence methods (such as Sanger method). The efficiency can be at least about 3x greater. The efficiency can be at least about 4x greater. The efficiency can be at least about 5x greater.

Nucleic acids as described herein can provide greater editing efficiencies than at least a portion of a native recruiting domain, such as a GluR2 domain. Nucleic acids can provide greater editing efficiencies than at least a portion of a modified recruiting domain, such as a modified GluR2 domain.

A target nucleic acid can comprise RNA. The RNA can be mRNA. The RNA can encode a protein or a portion thereof. A dysfunction of the protein or portion thereof can be implicated in a disease or condition. Administration of a composition, a vector, a nucleic acid, a non-naturally occurring RNA as described herein can treat, eliminate, cure, or reduce one or more symptoms of the disease or condition.

A disease or condition can comprise a neurodegenerative disease, a muscular disorder, a metabolic disorder, an ocular disorder, or any combination thereof. The disease or condition can comprise cystic fibrosis, albinism, alpha-1 -antitrypsin deficiency, Alzheimer disease, Amyotrophic lateral sclerosis, Asthma, β-thalassemia, Cadasil syndrome, Charcot-Marie-Tooth disease, Chronic Obstructive Pulmonary Disease (COPD), Distal Spinal Muscular Atrophy (DSMA), Duchenne/Becker muscular dystrophy, Dystrophic Epidermolysis bullosa, Epidermylosis bullosa, Fabry disease, Factor V Leiden associated disorders, Familial Adenomatous, Polyposis, Galactosemia, Gaucher's Disease, Glucose-6-phosphate dehydrogenase, Haemophilia, Hereditary Hematochromatosis, Hunter Syndrome, Huntington's disease, Hurler Syndrome, Inflammatory Bowel Disease (IBD), Inherited polyagglutination syndrome, Leber congenital amaurosis, Lesch-Nyhan syndrome, Lynch syndrome, Marfan syndrome, Mucopolysaccharidosis, Muscular Dystrophy, Myotonic dystrophy types I and II, neurofibromatosis, Niemann-Pick disease type A, B and C, NY-eso1 related cancer, Parkinson's disease, Peutz-Jeghers Syndrome, Phenylketonuria, Pompe's disease, Primary Ciliary Disease, Prothrombin mutation related disorders, such as the Prothrombin G20210A mutation, Pulmonary Hypertension, Retinitis Pigmentosa, Sandhoff Disease, Severe Combined Immune Deficiency Syndrome (SCID), Sickle Cell Anemia, Spinal Muscular Atrophy, Stargardt's Disease, Tay-Sachs Disease, Usher syndrome, X-linked immunodeficiency, various forms of cancer (*e.g.* BRCA1 and 2 linked breast cancer and ovarian cancer). The disease or condition can comprise a muscular dystrophy, an ornithine transcarbamylase deficiency, a retinitis pigmentosa, a breast cancer, an ovarian cancer, Alzheimer's disease, pain, Stargardt macular dystropy, Charcot-Marie-Tooth disease, Rett syndrome, or any combination thereof. Administration of a composition can be sufficient to: (a) decrease expression of a gene relative to an expression of the gene prior to administration; (b) edit at least one point mutation in a subject, such as a subject in need thereof; (c) edit at least one stop codon in the subject to produce a readthrough of a stop codon; (d) produce an exon skip in the subject, or (e) any combination thereof.

A pharmaceutical composition can comprise a first active ingredient. The first active ingredient can comprise a viral vector as described herein, a non-naturally occurring RNA as described herein, or a nucleic acid as described herein. The pharmaceutical composition can be formulated in unit dose form. The pharmaceutical composition can comprise a pharmaceutically acceptable excipient, diluent, or carrier. The pharmaceutical composition can comprise a second, third, or fourth active ingredient.

A composition described herein can compromise an excipient. An excipient can be added to a stem cell or can be co-isolated with the stem cell from its source. An excipient can comprise a cryo-preservative, such as DMSO, glycerol, polyvinylpyrrolidone (PVP), or any combination thereof. An excipient can comprise a cryo-preservative, such as a sucrose, a trehalose, a starch, a salt of any of these, a derivative of any of these, or any combination thereof. An excipient can comprise a pH agent (to minimize oxidation or degradation of a component of the composition), a stabilizing agent (to prevent modification or degradation of a component of the composition), a buffering agent (to enhance temperature stability), a solubilizing agent (to increase protein solubility), or any combination thereof. An excipient can comprise a surfactant, a sugar, an amino acid, an antioxidant, a salt, a non-ionic surfactant, a solubilizer, a trigylceride, an alcohol, or any combination thereof. An excipient can comprise sodium carbonate, acetate, citrate, phosphate, poly-ethylene glycol (PEG), human serum albumin (HSA), sorbitol, sucrose, trehalose, polysorbate 80, sodium phosphate, sucrose, disodium phosphate, mannitol, polysorbate 20, histidine, citrate, albumin, sodium hydroxide, glycine, sodium citrate, trehalose, arginine, sodium acetate, acetate, HCl, disodium edetate, lecithin, glycerine, xanthan rubber, soy isoflavones, polysorbate 80, ethyl alcohol, water, teprenone, or any combination thereof. An excipient can be an excipient described in the Handbook of Pharmaceutical Excipients, American Pharmaceutical Association (1986).

Non-limiting examples of suitable excipients can include a buffering agent, a preservative, a stabilizer, a binder, a compaction agent, a lubricant, a chelator, a dispersion enhancer, a disintegration agent, a flavoring agent, a sweetener, a coloring agent.

In some cases, an excipient can be a buffering agent. Non-limiting examples of suitable buffering agents can include sodium citrate, magnesium carbonate, magnesium bicarbonate, calcium carbonate, and calcium bicarbonate. As a buffering agent, sodium bicarbonate, potassium bicarbonate, magnesium hydroxide, magnesium lactate, magnesium glucomate, aluminium hydroxide, sodium citrate, sodium tartrate, sodium acetate, sodium carbonate, sodium polyphosphate, potassium polyphosphate, sodium pyrophosphate, potassium pyrophosphate, disodium hydrogen phosphate, dipotassium hydrogen phosphate, trisodium phosphate, tripotassium phosphate, potassium metaphosphate, magnesium oxide, magnesium hydroxide, magnesium carbonate, magnesium silicate, calcium acetate, calcium glycerophosphate, calcium chloride, calcium hydroxide and other calcium salts or combinations thereof can be used in a pharmaceutical formulation.

In some cases, an excipient can comprise a preservative. Non-limiting examples of suitable preservatives can include antioxidants, such as alpha-tocopherol and ascorbate, and antimicrobials, such as parabens, chlorobutanol, and phenol. Antioxidants can further include but not limited to EDTA, citric acid, ascorbic acid, butylated hydroxytoluene (BHT), butylated hydroxy anisole (BHA), sodium sulfite, p-amino benzoic acid, glutathione, propyl gallate, cysteine, methionine, ethanol and N- acetyl cysteine. In some instances a preservatives can include validamycin A, TL-3, sodium ortho vanadate, sodium fluoride, N-a-tosyl-Phe- chloromethylketone, N-a-tosyl-Lys-chloromethylketone, aprotinin, phenylmethylsulfonyl fluoride, diisopropylfluorophosphate, kinase inhibitor, phosphatase inhibitor, caspase inhibitor, granzyme inhibitor, cell adhesion inhibitor, cell division inhibitor, cell cycle inhibitor, lipid signaling inhibitor, protease inhibitor, reducing agent, alkylating agent, antimicrobial agent, oxidase inhibitor, or other inhibitor.

In some cases, a pharmaceutical formulation can comprise a binder as an excipient. Non-limiting examples of suitable binders can include starches, pregelatinized starches, gelatin, polyvinylpyrolidone, cellulose, methylcellulose, sodium carboxymethylcellulose, ethylcellulose, polyacrylamides, polyvinyloxoazolidone, polyvinylalcohols, C12-C18 fatty acid alcohol, polyethylene glycol, polyols, saccharides, oligosaccharides, and combinations thereof.

The binders that can be used in a pharmaceutical formulation can be selected from starches such as potato starch, corn starch, wheat starch; sugars such as sucrose, glucose, dextrose, lactose, maltodextrin; natural and synthetic gums; gelatine; cellulose derivatives such as microcrystalline cellulose, hydroxypropyl cellulose, hydroxyethyl cellulose, hydroxypropyl methyl cellulose, carboxymethyl cellulose, methyl cellulose, ethyl cellulose; polyvinylpyrrolidone (povidone); polyethylene glycol (PEG); waxes; calcium carbonate; calcium phosphate; alcohols such as sorbitol, xylitol, mannitol and water or a combination thereof.

In some cases, a pharmaceutical formulation can comprise a lubricant as an excipient. Non-limiting examples of suitable lubricants can include magnesium stearate, calcium stearate, zinc stearate, hydrogenated vegetable oils, sterotex, polyoxyethylene monostearate, talc, polyethyleneglycol, sodium benzoate, sodium lauryl sulfate, magnesium lauryl sulfate, and light mineral oil. The lubricants that can be used in a pharmaceutical formulation can be selected from metallic stearates (such as magnesium stearate, calcium stearate, aluminium stearate), fatty acid esters (such as sodium stearyl fumarate), fatty acids (such as stearic acid), fatty alcohols, glyceryl behenate, mineral oil, paraffins, hydrogenated vegetable oils, leucine, polyethylene glycols (PEG), metallic lauryl sulphates (such as sodium lauryl sulphate, magnesium lauryl sulphate), sodium chloride, sodium benzoate, sodium acetate and talc or a combination thereof.

In some cases, a pharmaceutical formulation can comprise a dispersion enhancer as an excipient. Non-limiting examples of suitable dispersants can include starch, alginic acid, polyvinylpyrrolidones, guar gum, kaolin, bentonite, purified wood cellulose, sodium starch glycolate, isoamorphous silicate, and microcrystalline cellulose as high HLB emulsifier surfactants.

In some cases, a pharmaceutical formulation can comprise a disintegrant as an excipient. In some cases, a disintegrant can be a non-effervescent disintegrant. Non-limiting examples of suitable non-effervescent disintegrants can include starches such as corn starch, potato starch, pregelatinized and modified starches thereof, sweeteners, clays, such as bentonite, micro-crystalline cellulose, alginates, sodium starch glycolate, gums such as agar, guar, locust bean, karaya, pecitin, and tragacanth. In some cases, a disintegrant can be an effervescent disintegrant. Non-limiting examples of suitable effervescent disintegrants can include sodium bicarbonate in combination with citric acid, and sodium bicarbonate in combination with tartaric acid.

In some cases, an excipient can comprise a flavoring agent. Flavoring agents incorporated into an outer layer can be chosen from synthetic flavor oils and flavoring aromatics; natural oils; extracts from plants, leaves, flowers, and fruits; and combinations thereof. In some cases, a flavoring agent can be selected from the group consisting of cinnamon oils; oil of wintergreen; peppermint oils; clover oil; hay oil; anise oil; eucalyptus; vanilla; citrus oil such as lemon oil, orange oil, grape and grapefruit oil; and fruit essences including apple, peach, pear, strawberry, raspberry, cherry, plum, pineapple, and apricot.

In some cases, an excipient can comprise a sweetener. Non-limiting examples of suitable sweeteners can include glucose (corn syrup), dextrose, invert sugar, fructose, and mixtures thereof (when not used as a carrier); saccharin and its various salts such as a sodium salt; dipeptide sweeteners such as aspartame; dihydrochalcone compounds, glycyrrhizin; Stevia Rebaudiana (Stevioside); chloro derivatives of sucrose such as sucralose; and sugar alcohols such as sorbitol, mannitol, sylitol, and the like.

In one aspect, this disclosure, the adRNA, helps recruit endogenous ADAR enzymes to a target mRNA and bring about site specific A-to-G editing. This has immense potential for gene therapy wherein the delivery of a single adRNA can potentially correct G-to-A point mutations. It also enables target RNA editing without having to overexpress RNA editing enzymes such the ADAR2. The disclosure demonstrates the applicability of this technology both *in vitro* and *in vivo.* This disclosure also demonstrates that by the creation of a long double stranded RNA, it is possible to recruit endogenous ADARs even in the absence of the ADAR recruiting domains. In addition, using these engineered adRNAs, it is possible to create multiple A-to-G edits in the mRNA in a target region. In one aspect, the system uses a U6 promoter (polIII) transcribed adRNAs as well as chemically synthesized adRNAs and there was shown to be efficient RNA editing. Thus, in one aspect, the constructs further comprise a promoter, such as a polII promoter, to transcribe adRNAs. Transcription from promoter such as a polIII promoter can improve target RNA editing efficiencies. Alu transcripts from a polIII promoter are preferentially edited. Also provided herein are engineered adRNA from the structure of Alu repeats that are targets for the endogenous ADARs.

The constructs of this disclosure can be used to localize adRNA to specific cellular compartments. For example, for nuclear localization, one can use adRNA-snRNA fusions. Similarly, by adding the N-terminal mitochondrial targeting sequence (MTS) it is possible to localize the adRNA to the mitochondria. Thus, in one aspect, the constructs further comprise the N-terminal mitochondrial targeting sequence (MTS). By the addition of an appropriate cis-acting zipcode, it is possible to localize adRNA into peroxisomes, endosomes and exosomes. Thus, in a further aspect, the constructs further comprise the appropriate cis-acting zipcode for localizing adRNA into peroxisomes, endosomes and exosomes. Localization of adRNA into endosomes can likely enable their transport across long distances in the case of neurons. Localization in exosomes can likely potentially help propagate adRNA to neighboring cells. Tethering moieties such as cholesterol to the adRNA can help in cellular uptake. Thus, in one aspect the constructs further comprise targeting moieties such as cholesterol.

In one aspect, the disclosure demonstrates that to create small molecule regulatable adRNAs, adRNA-aptamer are disclosed to be used in one aspect, to enable temporal control of RNA editing *e.g*. aptamers that bind flavin mononucleotide, guanine and other natural metabolites. Aptamers that bind sugars can also be used for this purpose.

In one aspect, the creation of a U1A-ADAR fusion is entirely of human origin. The N-terminal RNA recognition of motif of the splicesomal U1A protein binds to its cognate U1 hairpin II RNA with a dissociation constant of 63 nM.

The disclosure also provides constructs that further comprise a toehold.

The constructs of this disclosure can, in one aspect, be used in the absence of overexpression of the ADAR enzyme.

Thus, in certain aspects, the adRNA of this disclosure have certain components: a RNA targeting domain, from about 15 to about 200 base pairs in length (and ranges therebetween), which is complementary to the target RNA; 0-10 ADAR recruiting domains which can be derived from the GluR2 mRNA, Alu repeat elements or other RNA motifs that the ADAR binds to; and a cytosine mismatch required to direct the ADAR to the target adenosine which might be present anywhere in the targeting domain. When this adRNA binds to its target RNA, it recruits the ADAR enzyme to the target RNA. This ADAR enzyme now can catalyze the conversion of a target adenosine to inosine. For adRNAs of lengths over 50 base pairs, when expressed in HEK 293T and HeLa, the adRNAs cells can recruit ADARs even in the absence of the ADAR recruiting domain and enable significant levels of target RNA editing. A single adRNA can also be used to create multiple A-to-G edits in the target mRNA. In addition, by utilizing multiple adRNA it is possible to edit multiple different adRNA in the same cell. For example, in the *mdxmdx* mouse model of Duchenne muscular dystrophy, it is possible to not only correct the mutation in dystrophin, but also disrupt the mRNA sequences of genes coding for proteins involved
in nonsense mediated decay. Another application is the use of this technology to create loss of function, gain of function and dominant negative mutations and in one aspect, can be used for cancer screens, tumor progression as well as immunoediting studies.

### Engineered adRNA

Provided herein is an engineered ADAR1 or ADAR2 guide RNA ("adRNA") comprising, or alternatively consisting essentially of, or yet further consisting of: a sequence complementary to a target RNA. In one particular aspect, the engineered adRNA of this disclosure further comprises, or alternatively consists essentially of, or yet further consists of a sequence complementary to ornithine transcarbamylase.

In one aspect, the engineered adRNA of this disclosure further comprises, or alternatively consists essentially of, or yet further consists of an ADAR2 recruiting domain derived from GluR2 mRNA. In another aspect, the engineered adRNA of this disclosure further comprises, or alternatively consists essentially of, or yet further consists of ADAR1 recruiting domain derived from Alu repeats. In a further aspect, the engineered adRNA of this disclosure further comprises, or alternatively consists essentially of, or yet further consists of two MS2 hairpins flanking the sequence complementary to a target RNA. In some embodiments, the sequence complementary to a target RNA in the engineered adRNA of this disclosure comprises, or alternatively consists essentially of, or yet further consists of about 15 to 30 base pairs, or about 30 to 45 base pairs, or about 45 to 60 base pairs, or about 60 to 75 base pairs, or about 75 to 90 base pairs, or about 90 to 105 base pairs, or about 105 to 120 base pairs, or about 120 to 135 base pairs, or about 135 to 150 base pairs, or about 150 to 165 base pairs, or about 165 to 180 base pairs, or about 180 to 200 base pairs. In a further aspect, it is from about 40 to about 200, or about 50 to about 200, or from about 60 to about 200, or from about 70 to about 200, or from about 80 to about 200, or from about 90 to about 200, or from about 100 to about 200, base pairs.

Disclosed herein is an engineered adRNA comprising, or alternatively consisting essentially of, or yet further consisting of no ADAR recruiting domains, or about 1-2 ADAR recruiting domains, or about 2-3 ADAR recruiting domains, or about 3-4 ADAR recruiting domains, or about 4-5 ADAR recruiting domains, or about 5-6 ADAR recruiting domains, or about 6-7 ADAR recruiting domains, or about 7-8 ADAR recruiting domains, or about 8-9 ADAR recruiting domains, or about 9-10 ADAR recruiting domains. In some embodiments, the ADAR recruiting domains comprise, or alternatively consist essentially of, or yet further consist of GluR2 mRNA, Alu repeat elements or other RNA motifs to which ADAR binds. Also, provided herein is an engineered adRNA, wherein the ADAR2 recruiting domain of the engineered adRNA derived from GluR2 mRNA is located at the 5' end or the 3' end of the engineered adRNA. In some embodiments, the GluR2 mRNA is located at both the 5'end and the 3' end of the engineered adRNA.

In one aspect, the engineered adRNA of this disclosure, further comprises, or alternatively consists essentially of, or yet further consists of an editing inducer element. An "editing inducer element" can refer to a structure that is largely a double-stranded RNA, which is necessary for efficient RNA editing. Non-limiting examples of editing inducer elements are described in Daniel, C. et al. (2017) Genome Biol. 18, 195.

In one particular aspect, the engineered adRNA of this disclosure is encoded by a polynucleotide sequence selected from the group of sequences provided in **TABLE 1** or **FIG. 2****,** or an equivalent of each thereof.

Also disclosed herein is a complex comprising, or alternatively consisting essentially of, or yet further consisting of an engineered adRNA of this disclosure hybridized to a complementary polynucleotide under conditions of high stringency.

The disclosure also provides polypeptide and/or polynucleotide sequences for use in gene and protein editing techniques described below. It should be understood, although not always explicitly stated that the sequences provided herein can be used to provide the expression product as well as substantially identical sequences that produce a protein that has the same biological properties. These "biologically equivalent" or "biologically active" polypeptides are encoded by equivalent polynucleotides as described herein. They can possess at least 60%, or alternatively, at least 65%, or alternatively, at least 70%, or alternatively, at least 75%, or alternatively, at least 80%, or alternatively at least 85%, or alternatively at least 90%, or alternatively at least 95% or alternatively at least 98%, identical primary amino acid sequence to the reference polypeptide when compared using sequence identity methods run under default conditions. Specific polypeptide sequences are provided as examples of particular embodiments. Modifications to the sequences to amino acids with alternate amino acids that have similar charge. Additionally, an equivalent polynucleotide is one that hybridizes under stringent conditions to the reference polynucleotide or its complement or in reference to a polypeptide, a polypeptide encoded by a polynucleotide that hybridizes to the reference encoding polynucleotide under stringent conditions or its complementary strand. Alternatively, an equivalent polypeptide or protein is one that is expressed from an equivalent polynucleotide.

Also disclosed herein is an engineered adRNA-snRNA (small nuclear RNA) fusion. In one aspect, the engineered adRNA further comprises, or alternatively consists essentially of, or yet further consists of an N-terminal mitochondrial targeting sequence (MTS) to facilitate localization of the engineered adRNA to the mitochondria. In another aspect, provided herein is an engineered further comprising, or alternatively consisting essentially of, or yet further consisting of a cis-acting zipcode to facilitate localization of the engineered adRNA into peroxisomes, endosomes and exosomes. Localization of adRNA into endosomes can potentially enable their transport across long distances in the case of neurons. Localization in exosomes can potentially help propagate adRNA to neighboring cells. Tethering moieties such as cholesterol to the adRNA can help in cellular uptake.

Further provided herein is small molecule regulatable engineered adRNA. In one aspect, disclosed herein are engineered adRNA-aptamer fusions. Non-limiting examples of aptamers that can be used for this purpose include aptamers that bind flavin mononucleotide, guanine, other natural metabolites, or sugars. An "aptamer" can refer to a short single-stranded oligonucleotide capable of binding various molecules with high affinity and specificity. Non-limiting examples of aptamers are described in Lakhin, A. V. et al. (2013). Acta naturae, 5(4), 34-43.

Also disclosed herein is a U1A-ADAR fusion, entirely of human origin. The N-terminal RNA recognition of motif of the splicesomal U1A protein binds to its cognate U1 hairpin II RNA with a dissociation constant of 63 nM.

### Vectors and Recombinant Cells Expressing the Engineered adRNA

Provided herein is a vector comprising, or alternatively consisting essentially of, or yet further consisting of one or more of the isolated polynucleotide sequence encoding the engineered adRNA of this disclosure and optionally regulatory sequences operatively linked to the isolated polynucleotide. Non-limiting examples of a vector include a plasmid or a viral vector such as a retroviral vector, a lentiviral vector, an adenoviral vector, or an adeno-associated viral vector. The vectors can further comprise targeting sequences, zip codes or toeholds, as known in the art.

In one aspect, the regulatory sequences comprise, or alternatively consist essentially of, or yet further consist of a promoter, an enhancer element and/or a reporter. In some embodiments, the promoter is a human U6, a mouse U6 promoter, a CMV promoter, or a polIII promoter, or a polII promoter. In one aspect, the vector further comprises, or alternatively consists essentially of, or yet further consists of a detectable marker or a purification marker.

Further disclosed herein is a recombinant cell further comprising or alternatively consisting essentially of, or yet further consisting of the vector described above, wherein the engineered adRNA is recombinantly expressed.

### Compositions of the Engineered adRNA

Disclosed herein is a composition comprising, or alternatively consisting essentially of, or yet further consisting of a carrier and one or more of the engineered adRNA of this disclosure, the isolated polynucleotide encoding the engineered adRNA of this disclosure, the vector expressing the engineered adRNA of this disclosure, or the recombinant cell expressing the engineered adRNA of this disclosure. In one aspect, the carrier is a pharmaceutically acceptable carrier or a solid support. In a further aspect, the composition further comprises, or alternatively consists essentially of, or yet further consists of a chemotherapeutic agent or drug.

### Methods of Using the Engineered adRNAs

Provided herein is a method of modifying protein expression comprising, or alternatively consisting essentially of, or yet further consisting of contacting a polynucleotide encoding the protein, the expression of which is to be modified, with the engineered adRNA of this disclosure.

Also provided herein is a method of treating a disease or disorder associated with aberrant protein expression comprising, or alternatively consisting essentially of, or yet further consisting of administering to a subject in need of such treatment an effective amount of one or more of the engineered adRNA of this disclosure. In one particular aspect, provided herein is a method of treating Duchenne Muscular Dystrophy comprising, or alternatively consisting essentially of, or yet further consisting of administering to a subject in need of such treatment an effective amount of one or more of the engineered adRNA of this disclosure.

In the case of an *in vitro* application, in some embodiments the effective amount can depend on the size and nature of the application in question. It can also depend on the nature and sensitivity of the *in vitro* target and the methods in use. The skilled artisan can determine the effective amount based on these and other considerations. The effective amount can comprise one or more administrations of a composition depending on the embodiment.

The term "subject," "host," "individual," and "patient" are as used interchangeably herein to refer to animals, typically mammalian animals. Any suitable mammal can be treated by a method, cell or composition described herein. Non-limiting examples of mammals include humans, non-human primates (*e.g*., apes, gibbons, chimpanzees, orangutans, monkeys, macaques, and the like), domestic animals (*e.g*., dogs and cats), farm animals (*e.g*., horses, cows, goats, sheep, pigs) and experimental animals (*e.g*., mouse, rat, rabbit, guinea pig). In some embodiments a mammal is a human. A mammal can be any age or at any stage of development (*e.g*., an adult, teen, child, infant, or a mammal in utero). A mammal can be male or female. A mammal can be a pregnant female. In some embodiments a subject is a human. In some embodiments, a subject has or is suspected of having a cancer or neoplastic disorder. In other embodiments, a subject has or is suspected of having a disease or disorder associated with aberrant protein expression.

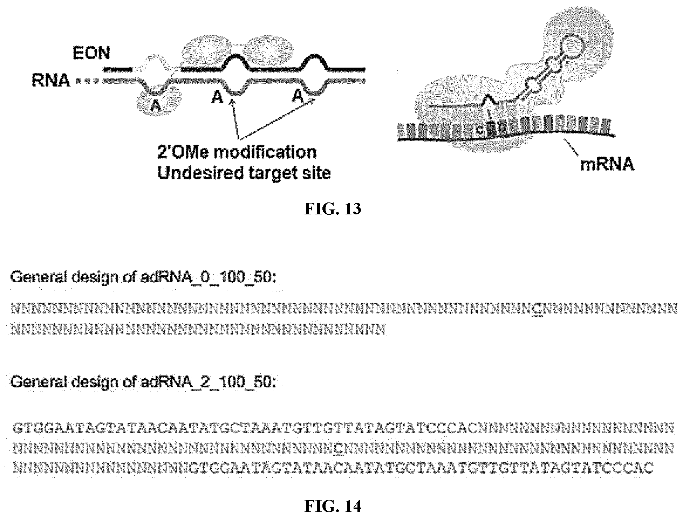

Referring to **FIG. 13****,** in contrast to antisense oligonucleotide (AON) designs (left side of **FIG. 13**) which can comprise short ssRNA (such as about 35 nt in length), exemplary constructs of the disclosure (right side of **FIG. 13**) can be long ssRNA having a length from about 60 bp to about 100 bp with superior target specificity and a total sequence length of from about 150 nt to about 250 nt in length. Constructs as described herein can include optimized and true hairpin structures - as opposed to mismatched bases that can be added to create hairpin-like RNA bulges, as shown on the left side of **FIG. 13** for the AON design. Advantages of true hairpin designs of the constructs include optimal and superior ADAR recruiting efficiency, resulting in higher on target editing yield - as compared to an AON construct. Hairpin designs can be completely independent from the mRNA target sequence and can be easily deployable for any new mRNA target. The target site for deamination can be unique, precise, and without need for undesired chemical modification, and without risk of undesired deamination at other sites. In contrast, AON designs utilizing a mismatch hairpin-like RNA bulge (such as show on the left side of **FIG. 13**), can be (a) limited efficacy in recruiting ADAR (not a true hairpin, structure can be too short); (b) prevented from reuse of sequence and bulges (need a unique design for each new target mRNA); (c) decreased specificity to a cell mRNA target site due to mismatches (increased risk of target damages), (d) required for 2'OMe modification in bulges to protect other Adenines from ADAR deamination activity (can cause undesired mutation at the wrong ribonucleotide).

### Recruitment of exogenous and endogenous ADARs via long-antisense-adRNAs

The CRISPR/Cas9 system is widely used in research but concerns over its *in vivo* applications exist. The two main concerns are the permanent edits that are made on the genome, and the immune response that can likely result from introducing a system that is bacterial in origin. As a result, RNA editing has gained interest as a potential solution for both challenges. The first challenge can be easily overcome by the application of RNA editing; RNAs transcribed from genes can be transient and edits will not permanently alter the cells. However, this can create a new problem in the form of decreased editing efficiencies, since many RNAs can be edited to achieve a phenotypic effect. Additionally, the second challenge of immune response may not be necessarily overcome. The ADAR family of enzymes that edit RNA are human in origin. ADAR1 in particular is expressed nearly ubiquitously in many cell types; there can be many potential therapeutic applications by harnessing its natural preference for RNA A-to-I (A-to-G) editing. This strategy also overcomes the significant hurdle of delivery, since a small guide RNA can be much simpler to transport than a large bulky enzyme. Therefore, the goal of the methods and compositions as described herein can include engineering a guide (referred to as adRNA) to recruit endogenous ADARs. ADARs can prefer to edit regions of double stranded RNA, particularly near sequences with secondary structures. Thus, engineering guides with increasing length of the antisense domain can be advantageous. The adRNA can have three components: (1) a RNA targeting domain, (such as from about 15 base pairs to about 200 base pairs in length), which can be complementary to the target RNA; (2) from about 0 to about 10 ADAR recruiting domains (which can be derived from the GluR2 mRNA, Alu repeat elements or other RNA motifs) to which the ADAR binds; and (3) a cytosine mismatch which can be required to direct the ADAR to the target adenosine which can be present anywhere in the targeting domain.

When this adRNA binds to its target RNA, it can recruit the ADAR enzyme to the target RNA. This ADAR enzyme can catalyze the conversion of a target adenosine to inosine. Interestingly, long adRNAs of lengths over 50 base pairs, when expressed in HEK 293T and HeLa cells can recruit ADARs even in the absence of the ADAR recruiting domain and can enable significant levels of target RNA editing. A single adRNA can also be used to create multiple A-to-G edits in the target mRNA. In addition, by utilizing multiple adRNA it can be possible to edit multiple different mRNA in the same cell. For example, in the *mdx* mouse model of Duchenne muscular dystrophy, it can be possible to not only correct the mutation in dystrophin, but also can disrupt the mRNA sequences of genes coding for proteins involved in nonsense mediated decay. Another application can be the use of this technology to create loss of function, gain of function, dominant negative mutations, or any combination thereof. This can be of paramount importance in cancer screens - tumor progression as well as immunoediting studies.

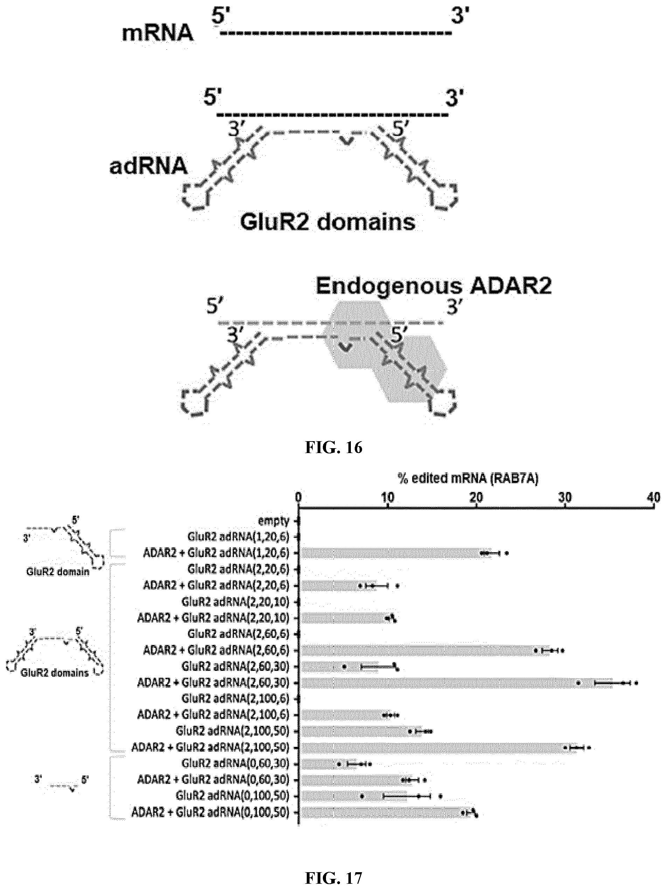

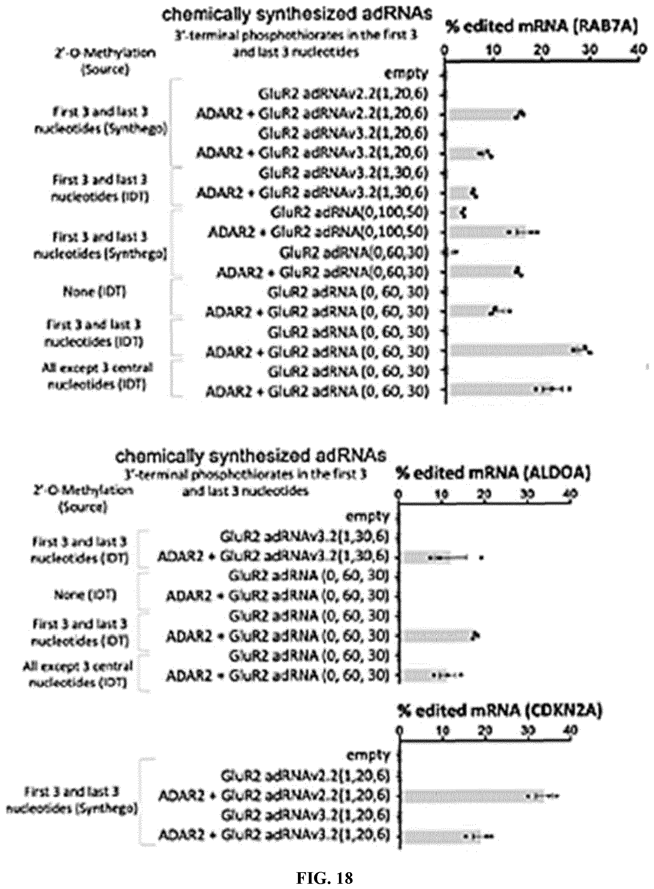

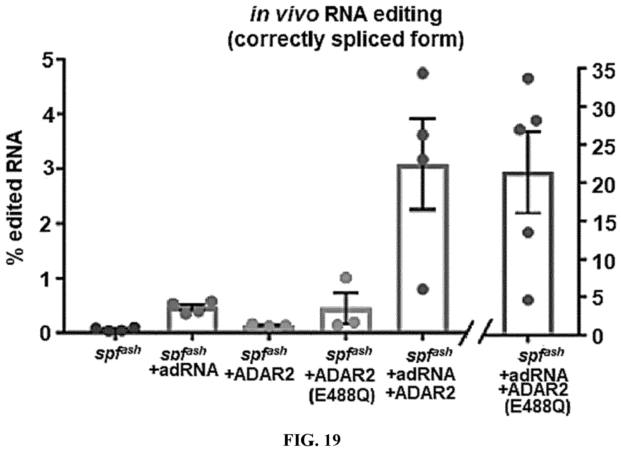

Referring to **FIG. 16****,** a schematic shows RNA editing via recruitment of endogenous ADARs in the presence of adRNA. These adRNA can be delivered either as chemically modified RNA or as U6 transcribed RNA. Referring to **FIG. 17****,** a U6 promoter transcribed adRNAs with progressively longer antisense domain lengths, in combination with zero, one or two GluR2 domains are evaluated for their ability to induce targeted RNA editing with or without exogenous ADAR2 expression. Values represent mean +/-SEM (n=3). Long adRNA can recruit endogenous ADARs for RNA editing. Referring to **FIG. 18****,** chemically synthesized adRNAs versions are tested against a panel of mRNAs with or without exogenous ADAR2 expression. The exact chemical modifications are stated in the figure along with the source of adRNA. Values represent mean +/- SEM (n=3). Referring to **FIG. 19****,** *in vivo* RNA correction efficiencies in the correctly spliced OTC mRNA in the livers of treated adult spf^{ash} mice (retro-orbital injections). RNA editing levels of 0.6% are seen in mice injected with U6 transcribed short adRNA.

### Recruitment of exogenous and endogenous ADARs via Alu-adRNAs

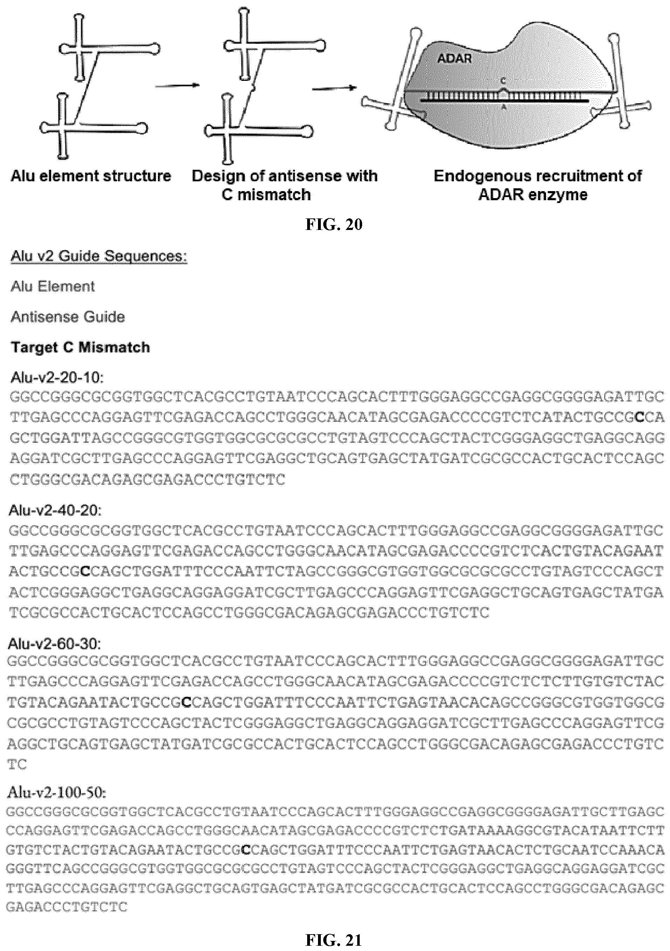

Alu genes are a transposable element in the genome and are a natural target of RNA editing by ADARs. An adRNA can be designed based on an Alu element structure to enable editing by endogenously recruited ADARs. Various positions on the Alu element structure can be tested where the native sequence can be replaced with the antisense sequence complementary to the target. Single stranded linker region can be selected, such as shown in **FIG. 20****.** Length of the antisense guide can be optimized, varying the length from about 20 to about 100 bases, targeting the RAB7A locus. Each guide can be designed to include one or more mismatches. A mismatch can be positioned between the cytosine of the antisense and the target adenosine base to be edited. This mismatch can be positioned in the middle of each antisense length. Editing efficiencies can be compared with an 100-50 antisense guide which can recruit both ADAR1 and ADAR2 and the GluR2 20-6 guide that can only recruits ADAR2.

Referring to **FIG. 20****,** a design of the Alu adRNA is shown. Left: a structure of an Alu element. Middle: a design incorporating a locus-specific antisense sequence with a C mismatch opposite the target A. Right: recruitment of the RNA editing enzyme ADAR to the target.

These guides are tested in 293FT cells by transfection with lipofectamine. Each guide is tested in cells overexpressing either ADAR1p110, ADAR1p150, ADAR2, or no enzyme overexpression to test the adRNA's ability to recruit endogenous ADARs. After 48 hours post-transfection, the cells are harvested, RNA is extracted, is converted to cDNA, and the RAB7A locus is amplified for Sanger sequencing. Editing efficiencies are calculated as a ratio of peak height. **FIG. 5** shows the results of the Alu guide length experiment.

Referring to **FIG. 5****,** the long Alu-v2-100-50 guide shows improved editing in cells where no ADAR enzyme is overexpressed. The overexpression of ADAR1p150 results in significantly higher editing rates for the Alu constructs while editing rates are similar for the linear 100-50 guide. ADAR1p150 can preferentially binds Z-RNA due to its extra ds-RNA binding domain than the shorter isoform, ADAR1p110, lacks. The Alu elements with their high GC content are known to form Z-DNA and Z-RNA, aiding in the recruitment of ADAR1p150.

### Split-ADAR2 deaminase domain (DD)

Overexpression of ADARs can lead to several transcriptome wide off-target edits. The ability to restrict the catalytic activity of the ADAR2 DD only to the target mRNA can reduce the number of off-targets. Creation of a split-ADAR2 DD can be one potential approach to reduce the number of off-targets. Split-protein reassembly or protein fragment complementation can be a widely used approach to study protein-protein interactions. Splitting the ADAR2 DD can be designed in such a way that each fragment of the split-ADAR2 DD can be catalytically inactive by itself. However, in the presence of the adRNA, the split halves can dimerize to form a catalytically active enzyme at the intended mRNA target.

Regions for splitting a protein can be identified by studying the crystal structure of the ADAR2 DD in complex with its naturally occurring substrates, understanding solvent accessibility scores, using predictive software(s), or any combination thereof. MS2-MCP systems and boxB-lambda N systems (that can efficiently recruit ADARs) can be utilized alone or in combination to recruit the N and C terminals of the split ADAR2 DD respectively. The adRNA can comprise one MS2 stem loop and one boxB hairpin along with an antisense domain complementary to the target. This can enable recruitment of the N and C terminals of the split ADAR2 DD at the target and thereby can constitute a catalytically active DD.

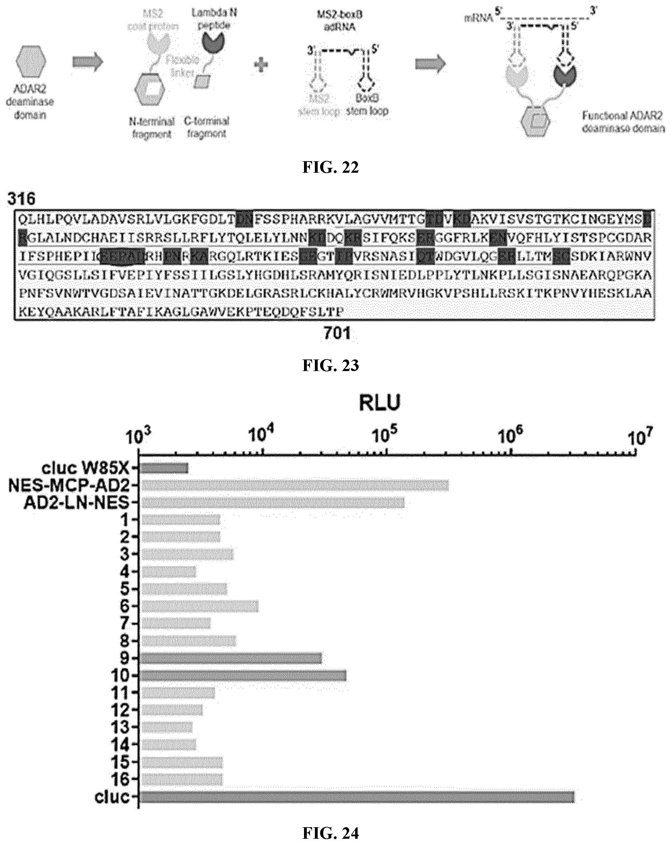

Referring to **FIG. 22** and **FIG. 23****,** a schematic of the split-ADAR2 DD system is shown and an exemplary sequence of the ADAR2 DD with sites for splitting highlighted.

Referring to **FIG. 24****,** pairs of fragments 1-16 can be assayed via a cypridina luciferase reporter (cluc W85X). Fragments 9 and 10 show the highest activity. The split positions corresponding to fragments 9 and 10 are circled in blue in **FIG. 23****.**

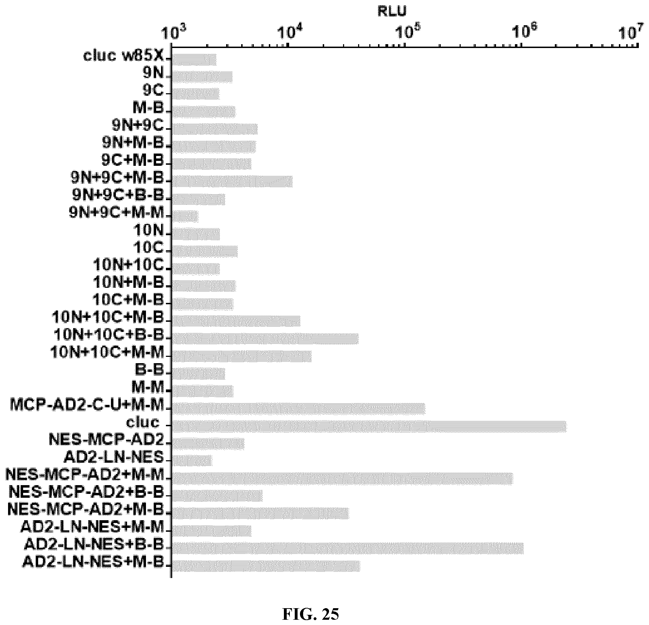

Referring to **FIG. 25****,** fragments 9 and 10 assayed against the Clue reporter. Further, a NES-MCP-AD2-C-U-variant can also be tested for A-G editing. N: N-terminal fragment, C: C-terminal fragment, M-M: MS2-MS2 adRNA, M-B: MS2-BoxB adRNA, B-B: BoxB-BoxB adRNA

Further completely humanized versions of these constructs can be created by harnessing human RNA binding proteins, such as (a) U1A or (b) its evolved variant TBP6.7 which has no known endogenous human hairpin targets or (c) the human histone stem loop binding protein (SLBP) or (d) the DNA binding domain of glucocorticoid receptor, or (e) any combination thereof. These proteins can be fused to the N and C terminal fragments of the ADAR2 to create a completely human and programmable RNA editing toolset that can edit adenosines with exquisite specificity. Further, chimeric RNA bearing two of the corresponding RNA hairpins can be utilized to recruit the ADAR2 fragments. Sequences of the RNA hairpins are provided herein.

A C-U RNA editing enzyme can be created by making one or more mutations (such as 16 mutations) in the ADAR2 deaminase domain. Even with one or more mutations, this editing enzyme can still show several transcriptome wide A-G and C¬U off-targets. To improve the specificity of this enzyme, it can be split at certain residues (such as those residues identified in previous screens) and thus develop a split ADAR system for C-U editing.

### Recruitment of exogenous and endogenous APOBECs for C-U editing

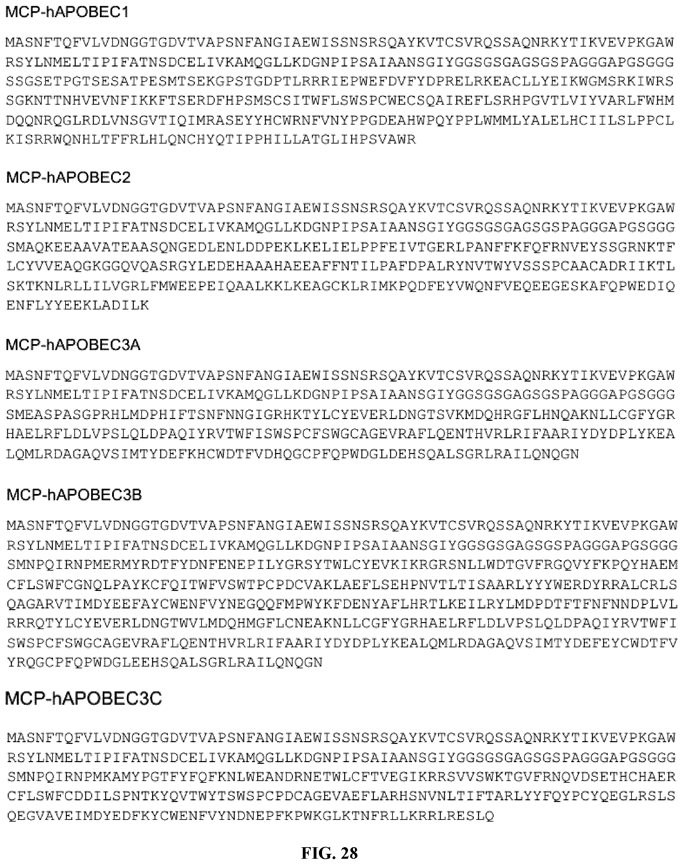

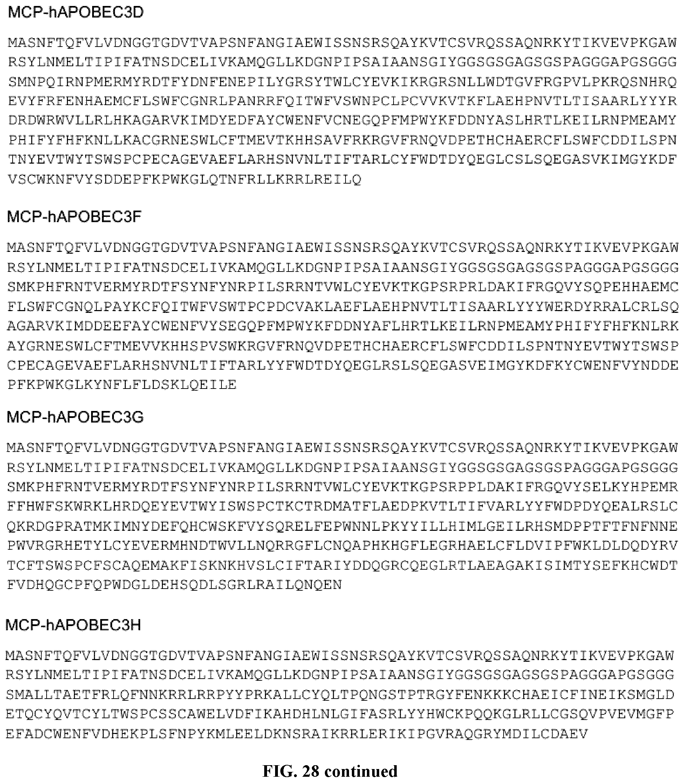

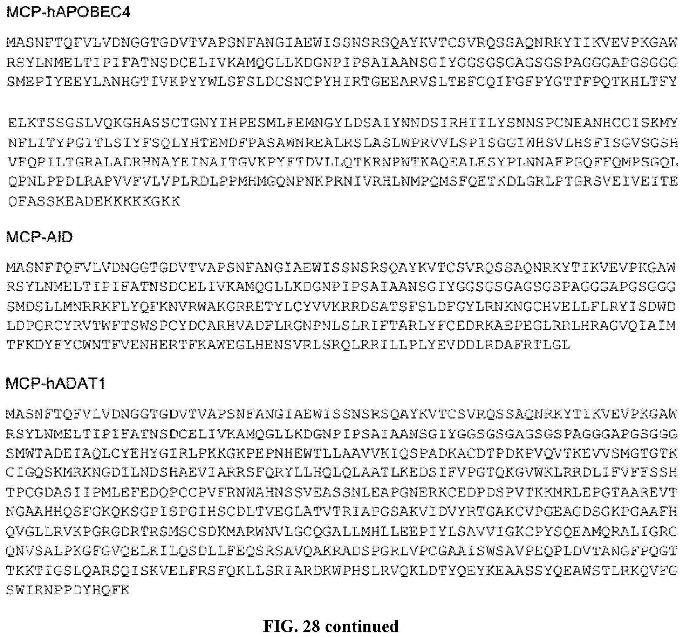

APOBECs (apolipoprotein B mRNA editing enzyme, catalytic polypeptide-like) are RNA editing enzymes that convert cytidines to uracil and create diversity at the mRNA level. These entities can be recruited for C-U editing of RNA by a guide RNA, such as an engineered APOBEC recruiting guide RNAs (apRNAs). In some cases, a fusion construct can be created comprising one or more APOBEC family members. For example, a fusion construct can comprise ADAT1 (Adenosine Deaminase TRNA Specific 1) and AID (Activation-induced cytidine deaminase) to the MCP (MS2 Coat Protein). Engineered MS2-apRNA can be utilized to recruit one or more MCP fusions. The protein sequences for the constructs as well as MS2-apRNA sequences are shown in **FIG. 28****-** **FIG. 30****.** A protein sequence for a fusion construct or a sequence utilized in any of the methods as described herein can comprise at least about: 70%, 75%, 80%, 85%, 90%, 95%, 97%, 98%, 99% sequence identity or more to at least a portion of any sequence of **FIG. 28****.**

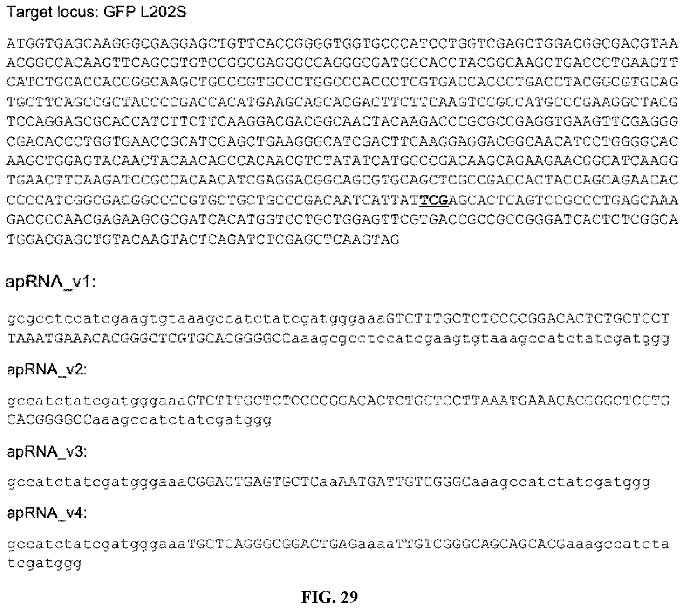

In some cases, methods can include constructs configured for recruitment of an APOBEC, such as APOBEC3A. Recruitment can be endogenous recruitment. Constructs configured for recruitment of APOBECs, can be designed by targeting preferences of primary sequence, secondary structure or a combination thereof. Designs for one or more apRNA can include those sequences or a portion thereof as show in **FIG. 29****.** In some cases, a sequence that can recruit an APOBEC, such as APOBEC3A or a sequence utilized in any of the methods as described herein can comprise at least about: 70%, 75%, 80%, 85%, 90%, 95%, 97%, 98%, 99% sequence identity or more to at least a portion of any sequence of **FIG. 29****.**

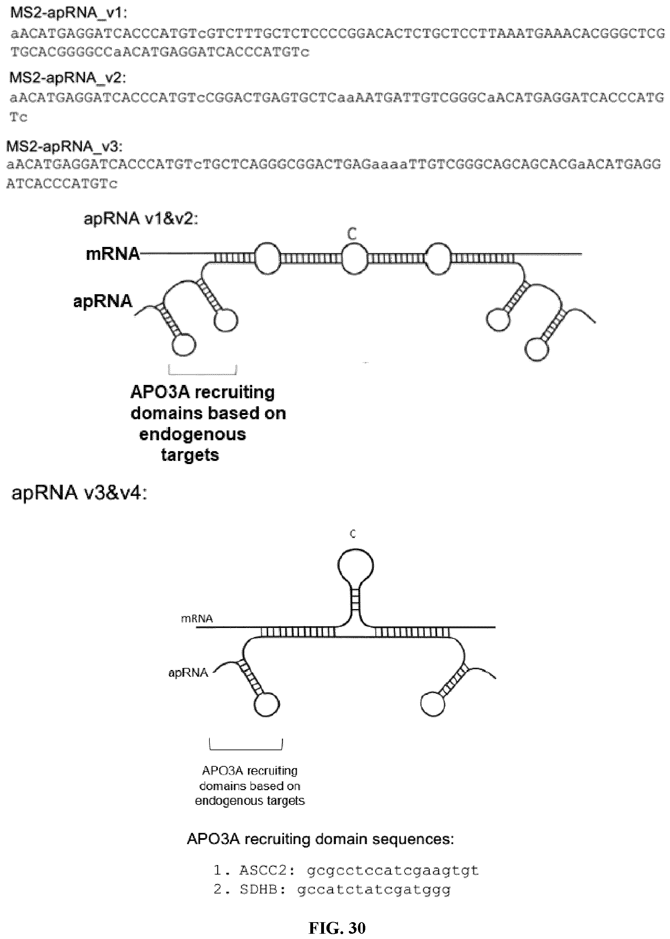

To recruit MCP-APOBEC3A, MS2-apRNA can be designed and their sequence can comprise any one or more of the sequences of **FIG. 30****.** In some cases, a sequence that can recruit MCP-APOBEC3A or a sequence utilized in any of the methods as described herein can comprise at least about: 70%, 75%, 80%, 85%, 90%, 95%, 97%, 98%, 99% sequence identity or more to at least a portion of any sequence of **FIG. 30****.**

### Luciferase Assay

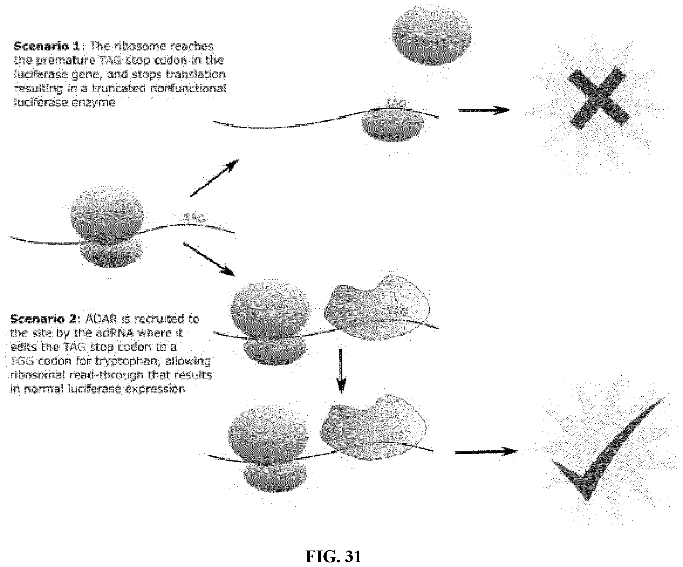

When the ADAR converts the target A in TAG to an I (read as a G) the ribosome can be able to fully translate the protein as diagrammed in **FIG. 31****.** Cells can be transfected and after 48 hours can be visualized with a luciferase reporter assay. The light readout from the cells can indicate restoration of luciferase activity.

Referring to **FIG. 31****,** a first scenario demonstrates an example in which a ribosome can reach a premature TAG stop codon in a luciferase gene, and can stop translation resulting in a truncated non-functional luciferase enzyme. In a second scenario, an ADAR can be recruited to a site by an adRNA where it can edit a TAG stop codon to a TGG codon for trytophan that can allow for ribosomal read-through that can result in normal luciferase expression. Such an system can permit evaluation of appropriate ADAR recruitment by an adRNA. When lucifierase expression is detected, ADAR recruitment by an adRNA can have occurred. When luciferase expression is not detected, ADAR recruitment may not have occurred.

### Kits

Also disclosed herein is a kit comprising, or alternatively consisting essentially of, or yet further consisting of the engineered adRNA of this disclosure, the isolated polynucleotide encoding the engineered adRNA of this disclosure, the vector expressing the engineered adRNA of this disclosure, the recombinant cell expressing the engineered adRNA of this disclosure, or the compositions disclosed herein and instructions for use. In one aspect, the instructions recite the methods of using the engineered adRNA disclosed herein.

A kit can comprise a vector. The vector can be packaged in a container. The kit can comprise a non-naturally occurring RNA. The non-naturally occurring RNA can be packaged in a container. The kit can comprise a syringe. A syringe can be the container in which the vector, nucleic acid, or non-naturally occurring RNA can be packaged. The kit can comprise a pharmaceutical composition as described herein. The kit can comprise instructions for administration to a subject in need thereof of a viral vector, a non-naturally occurring RNA, a pharmaceutical composition as described herein.

### EXAMPLES

The following examples are non-limiting and illustrative of procedures which can be used in various instances in carrying the disclosure into effect. Additionally, all reference disclosed herein are incorporated by reference in their entirety.

### Example 1

Not to be bound by theory, it is believed that ADARs, which may be found in mammals, can be recruited to catalyze therapeutic editing of point mutations. ADAR1 or ADAR2 can be recruited to the target RNA or potentially DNA via the use of engineered RNA scaffolds, engineered DNA scaffolds or DNA-RNA hybrid scaffolds. Tissues that can be potentially targeted using this approach include, but are not limited to, the central and peripheral nervous system, lungs, liver, gastrointestinal tract, pancreas, cardiac muscle, kidneys and skin.

An exemplary embodiment proposed herein is an engineered ADAR2 guide RNA (adRNA) that bears a 20-100 bp complementarity with the target RNA. This engineered adRNA also contains an ADAR2 recruiting domain from the GluR2 mRNA either at the 5' end or the 3' end, or both ends.

This was tested *in vivo* in the spf^{ash} mouse model of ornithine transcarbamylase deficiency. This model bears a G->A point mutation in the last nucleotide of exon 4. Upon delivery of only adRNA via AAVs, up to 1% correction of the point mutation in the absence of the overexpression of the ADAR enzymes was observed. The disclosure also shows this efficacy *in vitro* in HeLa cells that are known to express ADARs. RNA editing was observed in these cells upon delivery of only the adRNA.

Further, this efficiency can be applied to other recruiting domains. Accordingly, further aspects relate to an engineered single stranded ADAR2 guide DNA (adDNA) as well as adDNA-RNA hybrid with potential greater stability than adRNA. Methods of use of these enzymes as disclosed herein are further provided.

Not to be bound by theory, since the ADAR family of enzymes catalyze the hydrolytic deamination of adenosine to inosine, it is believed that these enzymes can be used to catalyze the hydrolytic deamination of cytosine to thymine by mutating three specific residues of the ADAR2 - V351, E396 and C451 that interact with the target adenosine. Thus, methods of providing this catalytic potential is provided by mutation of these sites.

All possible amino acid substitutions at the three residues mentioned have been created and are being screened to test the hypothesis.

In addition experiments are being performed to explore the roles of other amino acids such as S486 that might enable elimination of the ADARs intrinsic preference for a UAG editing site.

In order to engineer C->T edits, the roles of hAPOBEC1 and rAPOBEC 1 along with the overexpression of the Apobec1 complementation factor (ACF) are determined. In addition, a MCP-(h/r)APOBEC1-ACF fusion protein is further provided herein.

Also provided to the current adenine base editing approach to Cas9 (or Cpf1)-ADAR deaminase domain fusions (ADAR1, ADAR2 and their catalytically active mutants E1008Q and E488Q), compositions targeting the ssDNA displaced strand by current base editors are further provided herein. To accomplish such, the gRNA bound strand with a A-C bulge, ideally in the first 10bp close to the 5' end of the gRNA is targeted.

Additional embodiments are exemplified in the appended documents, incorporated herein by reference.

### Example 2

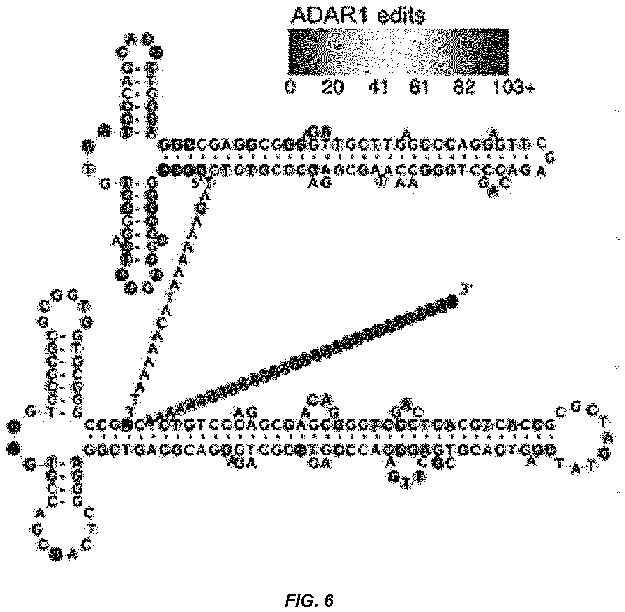

Referring to **FIG. 6****,** Alu elements can be a primary target of endogenous ADAR based RNA editing. Therefore, for the purpose of programmable RNA editing, the goal is to design an ADAR recruiting RNA (adRNA) based on the Alu elements as these can potentially enable efficient recruitment of endogenous ADARs. The Alu-adRNA is expressed from a human U6 promoter and the linker sequence between the Alu repeats is replaced by antisense domains of a variety of lengths, targeting the RAB7A transcript. Each antisense domain has a mismatched nucleotide in the middle of the antisense region, a cytosine across from the target adenosine.

The Alu-adRNA are tested out *in vitro* by transfection of 293FT cells, either along with ADAR1p110, ADAR1p150, ADAR2, or without an overexpressed enzyme, to demonstrate recruitment of endogenous ADARs. Cells are harvested 48 hours post transfection, RNA is extracted and converted to cDNA via the use of either random hexamers or oligo-dT primers. The RAB7A locus is then amplified and sent for Sanger sequencing. Editing efficiencies are calculated as the ratio of Sanger peak heights G/(A + G).

### Example 3

### Vector design and construction

Zero, one or two copies of the GluR2 adRNAs were cloned into an AAV vector containing a human U6 and mouse U6 promoter along with a CMV promoter driving the expression of GFP or the full length human ADAR2 enzyme or its hyperactive mutant ADAR2 (E488Q). Similarly, one or two copies of the MS2 adRNAs were cloned into an AAV vectors bearing the MCP-ADAR1 or MCP-ADAR2 deaminase domain fusions and their hyperactive mutants. To construct the GFP reporters - GFP-Amber, GFP-Ochre and GFP-Opal, three gene blocks were synthesized with `TAG', `TAA' and 'TGA' respectively replacing the Y39 residue of the wild type GFP and were cloned downstream of a CAG promoter. To construct the OTC and DMD reporters, 200 bp fragments of the spf^{ash} OTC and *mdx* DMD transcript bearing the target adenosine(s) to be edited were cloned downstream of the CAG promoter.

### Mammalian cell culture and transfection

All HEK 293T cells were grown in Dulbecco's Modified Eagle Medium supplemented with 10% FBS and 1% Antibiotic-Antimycotic (Thermo Fisher) in an incubator at 37 °C and 5% CO2 atmosphere. All *in vitro* transfection experiments were carried out in HEK 293T cells using the commercial transfection reagent Lipofectamine 2000 (Thermo Fisher). All *in vitro* RNA editing experiments involving a reporter were carried out in 24 well plates using 400ng of reporter plasmid and 800ng of the adRNA+enzyme plasmid. All *in vitro* RNA editing experiments targeting an endogenous transcript were carried out in 24 well plates using 800ng of the adRNA/Enzyme plasmid. dCas13b-ADAR2DDE488Q based RNA editing experiments were carried out using 800ng of the enzyme plasmid (Addgene #103864) as well 800 ng of the gRNA plasmid. Cells were transfected at 25-30% confluence and harvested 60 hours post transfection for quantification of editing. Chemically synthesized adRNAs (synthesized via IDT or Synthego) were transfected using Lipofectamine 3000 (Thermo Fisher) at an amount of 20 pmol/well.

### Production of AAV vectors

AAV8 particles were produced using HEK 293T cells via the triple transfection method and purified via an iodixanol gradient. Confluency at transfection was about 80%. Two hours prior to transfection, DMEM supplemented with 10% FBS was added to the HEK 293T cells. Each virus was produced in 5 x 15 cm plates, where each plate was transfected with 7.5 ug of pXR-8, 7.5 of ug recombinant transfer vector, 7.5 ug of pHelper vector using PEI (1ug/uL linear PEI in 1xDPBS pH 4.5, using HCl) at a PEI:DNA mass ratio of 4:1. The mixture was incubated for 10 minutes at RT and then applied dropwise onto the cell media. The virus was harvested after 72 hours and purified using an iodixanol density gradient ultracentrifugation method. The virus was then dialyzed with 1 x PBS (pH 7.2) supplemented with 50 mM NaCl and 0.0001% of Pluronic F68 (Thermo Fisher) using 50kDA filters (Millipore), to a final volume of ~1 mL and quantified by qPCR using primers specific to the ITR region, against a standard (ATCC VR-1616).
AAV-ITR-F: 5'-CGGCCTCAGTGAGCGA-3' (SEQ ID NO: 149) and
AAV-ITR-R: 5'-GGAACCCCTAGTGATGGAGTT-3' (SEQ ID NO: 150).

### Example 4 - in vivo RNA editing of point mutations via RNA-guided adenosine deaminases.

A system for sequence-specific RNA base editing via Adenosine Deaminases acting on RNA (ADAR) enzymes with associated ADAR guide RNAs (adRNAs) was designed. The system was systematically engineered to harness ADARs, and comprehensively evaluated its specificity and activity *in vitro* and *in vivo* via two mouse models of human disease. In some cases, this platform can enable tunable and reversible engineering of RNAs for diverse applications.

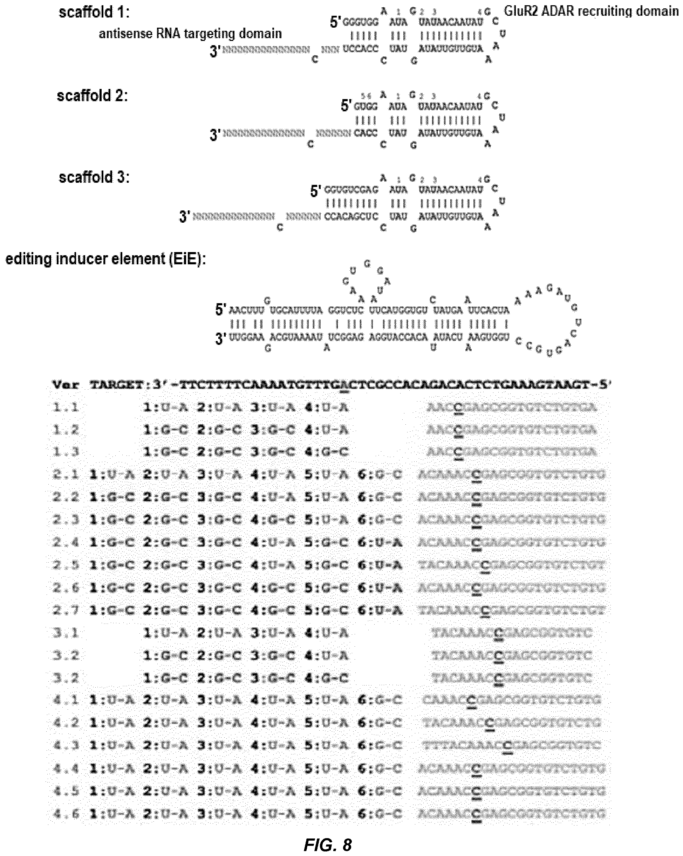

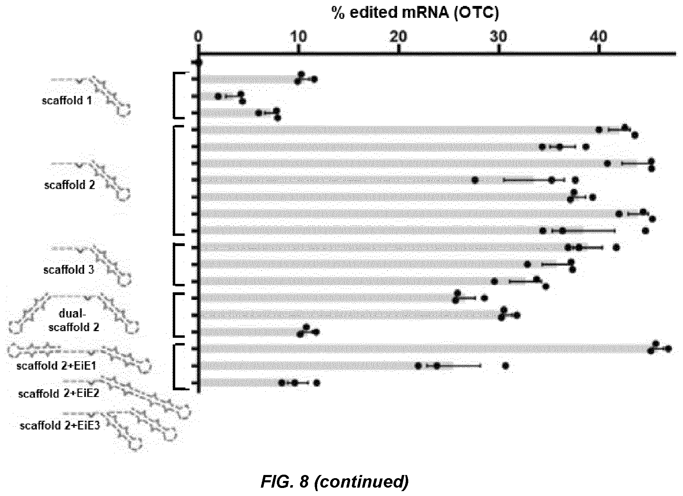

Adenosine to inosine RNA editing, a post-transcriptional RNA modification, is catalyzed by Adenosine Deaminases acting on RNA (ADAR) enzymes. Inosine is a deaminated form of adenosine that is biochemically recognized as guanine. Recently, multiple studies have demonstrated ADAR mediated targeted RNA editing. Building on these, two orthogonal toolsets were engineered for sequence-specific programmable RNA base editing *in vitro* and *in vivo.* Specifically, a system for targeted RNA editing via ADAR1/2 with associated ADAR guide RNAs (adRNAs) was utilized (**FIG. 32A**). The adRNAs comprise in part a programmable antisense region that is complementary to the target RNA sequence with a mismatched cytidine opposite the target adenosine. Additionally, they bear in one version, zero, one, or two ADAR-recruiting domains engineered from the naturally occurring ADAR substrate GluR2 pre-mRNA (referred hereon as GluR2 adRNA); and in a second format, two MS2 hairpins flanking the antisense region (referred hereon as MS2 adRNA). The GluR2 adRNA was systematically optimized to enhance recruitment of exogenous and/or endogenous ADARs by evaluating multiple scaffold variants, including mutagenized scaffolds based on G-C versus A-U pairing, addition of editing inducer elements, and antisense domain length and mis-match position modifications (**FIG. 8****,** **FIG. 34A-C**). The latter MS2 adRNA version was in turn optimized to harness synthetic proteins comprising the deaminase domains (DD) of ADAR1 or ADAR2 fused to the MS2 Coat Protein (MCP), via systematic antisense domain length and mis-match position modifications, coupled with use of hyper-active versions of the deaminase domains, and versions bearing nuclear localization (NLS) versus export (NES) signals (**FIG. 32B****,** **FIG. 35A****, 41B**).

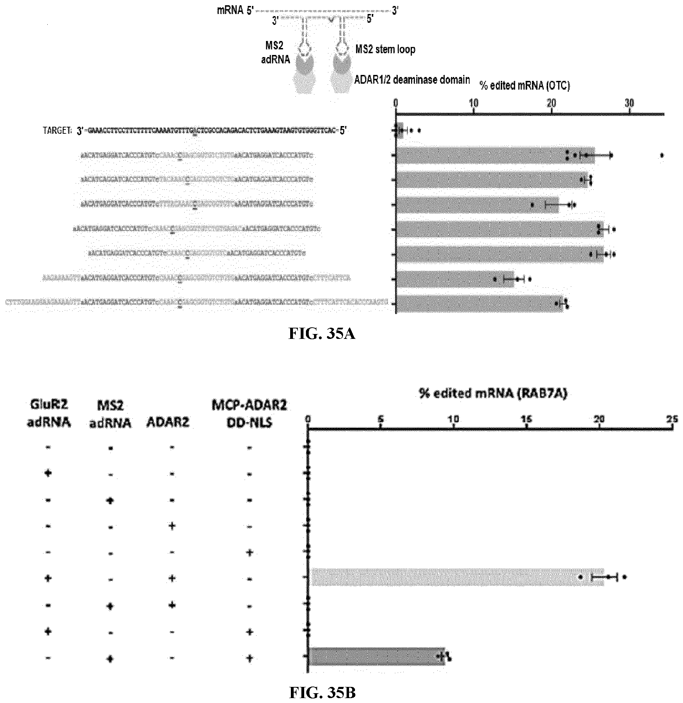

The activity of the above two systems were comprehensively evaluated and benchmarked with the recently developed RNA editing system based on Cas13b. These *in vitro* experiments revealed that: (1) the engineered constructs were active in their ability to effect targeted RNA editing with yields comparable to the Cas13b based system (**FIG. 32B****,** **FIG. 36A****, Tables 2, 3**), and U6 transcribed adRNAs and chemically synthesized adRNAs were both effective formats (**FIG. 36B**); (2) adRNAs bearing long antisense domains, both with and without GluR2 domains, suffice to recruit exogenously expressed ADARs, and to a degree endogenous ADARs too to enable efficient RNA editing (**FIG. 32B****,** **FIG. 34B****, 40C, 42C**); (3) the constructs based on the MS2 adRNAs and corresponding MCP-ADAR1/2 fusions showed the highest and most robust activity, including across a large panel of endogenous genes chosen across a spectrum of different expression levels (**FIG. 32B****,** **FIG. 36C**); (4) use of a NES and/or hyper-active deaminase domains in the MCP-ADAR1/2 fusions consistently yielded higher RNA editing yields at the target adenosine, but also led to a higher propensity of editing at non-targeted adenosines in the flanking sequences (**FIG**. **32B****,** **FIG. 37A**). To further validate this, a similar promiscuity ensued from deletion of the native NLS domain in ADAR2 (Δ1-138) (**FIG. 37B** - **FIG. 37D**); and 5) these two toolsets were operationally orthogonal: specifically, the editing efficiency of the MCP-ADAR2 deaminase domain fusion with a co-expressed MS2 adRNA or GluR2 adRNA was evaluated and displayed on-target editing only via the former. Conversely, full-length ADAR2 was observed to be recruited by the GluR2 adRNA and not the MS2 adRNAs (**FIG. 35B**).

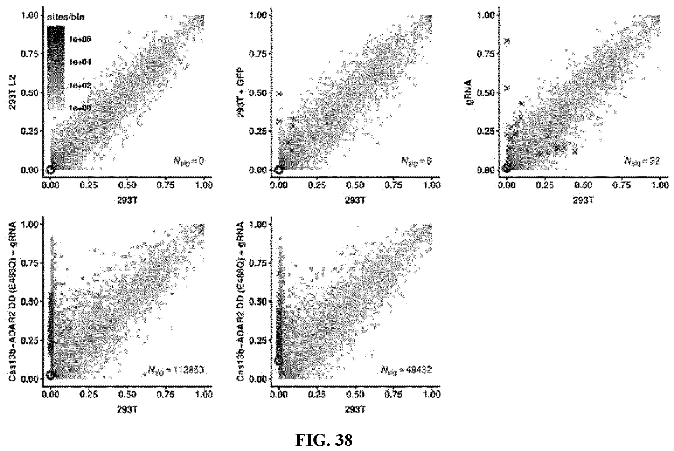

Having demonstrated robust activity of this toolset, its specificity profiles were investigated via analysis of the transcriptome-wide off-target A->G editing effected by this system (**FIG. 32C**). To this end, HEK 293T cells were transfected with each construct and analyzed by RNA-seq. Untransfected cells were included as controls. From each sample, ~40 million uniquely aligned sequencing reads were collected. Fisher's exact test was used to quantify significant changes in A->G editing yields, relative to untransfected cells, at each reference adenosine site having sufficient read coverage. The number of sites with at least one A->G editing event detected in any of the samples was computed. Of these, the number of sites with statistically significant A->G edits, at a false discovery rate (FDR) of 1%, and with fold change of at least 1.1, was found to vary over a wide range, from lowest for the MCP-ADAR2 DD-NLS construct, to highest for the MCP-ADAR1 DD (E1008Q)-NES (**FIG. 38** - **FIG. 41****, Tables 4, 5**). To investigate the distribution of editing yields, violin plots were generated considering the A-sites whose editing yields changed significantly in at least one sample (**FIG. 32**). Taken together, the RNA-seq experiments revealed that transcriptome-wide off-target edits were: 1) less prevalent in MCP-ADAR constructs with NLS than constructs with NES; 2) less prevalent in MCP-ADAR2 constructs than MCP-ADAR1 constructs; 3) less prevalent in the wild-type MCP-ADAR constructs than the E>Q hyperactive mutants (**FIG. 42A****, Table 5**); and 4) the off-targets were primarily due to ADAR overexpression and use of adRNAs alone resulted in least number of off-targets (**FIG. 42B**).

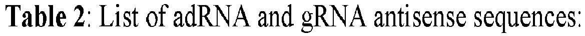

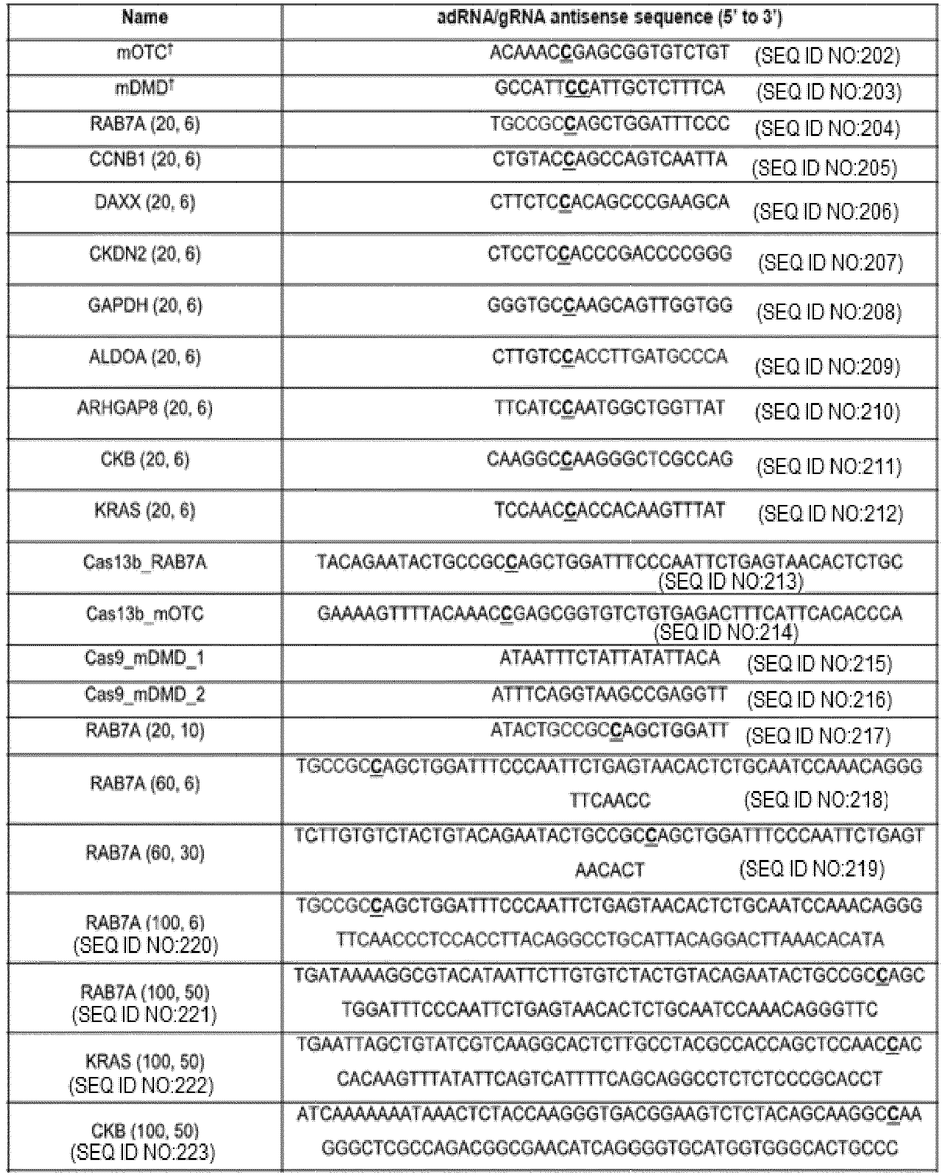

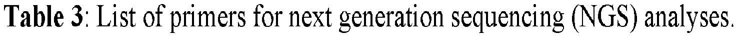

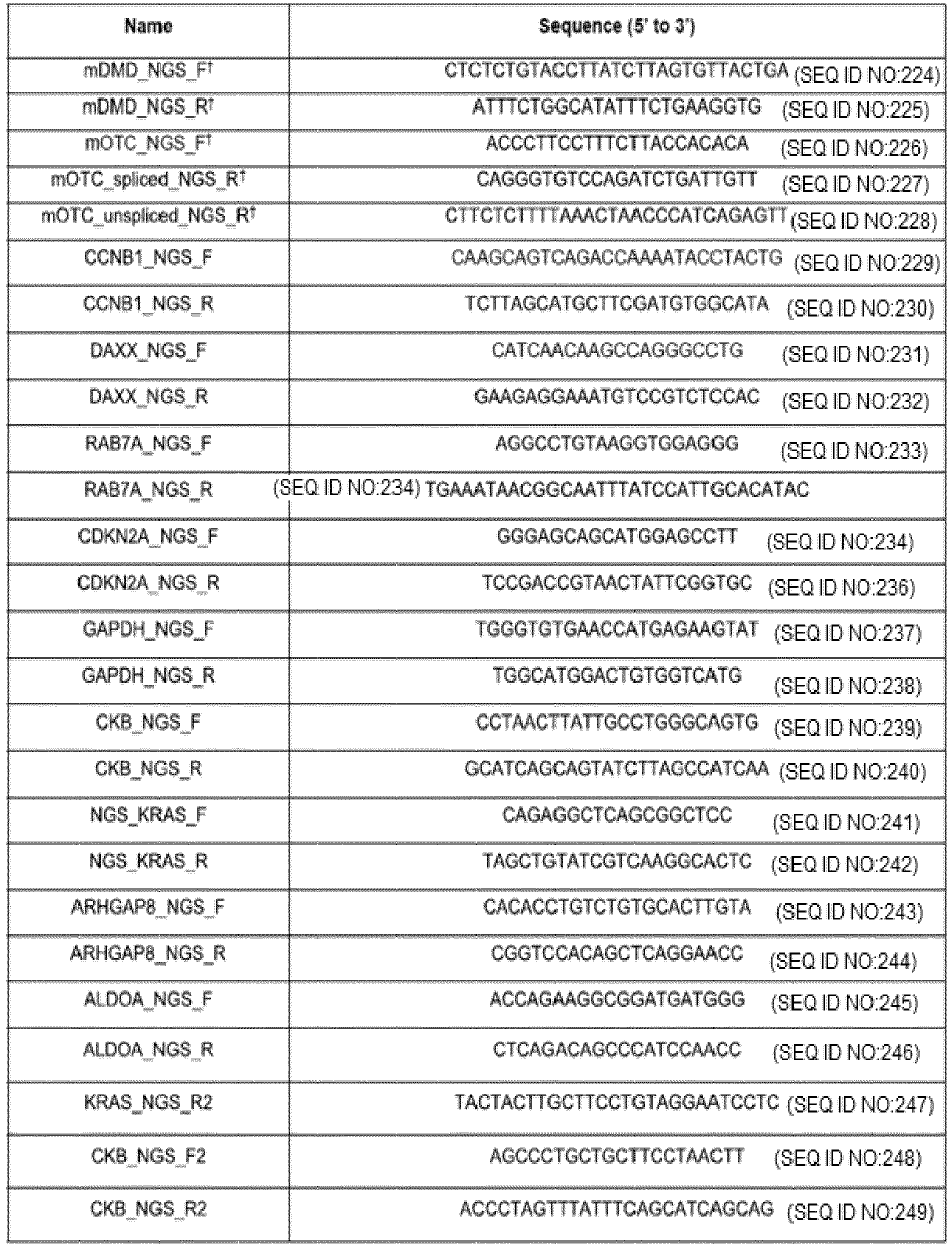

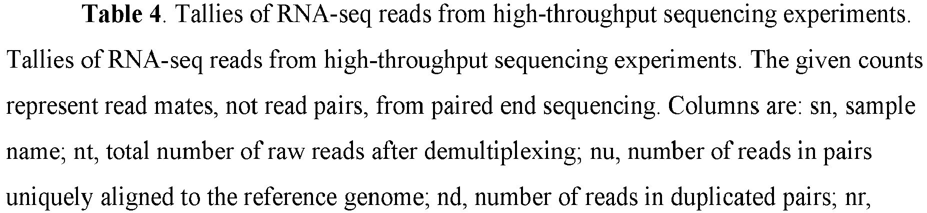

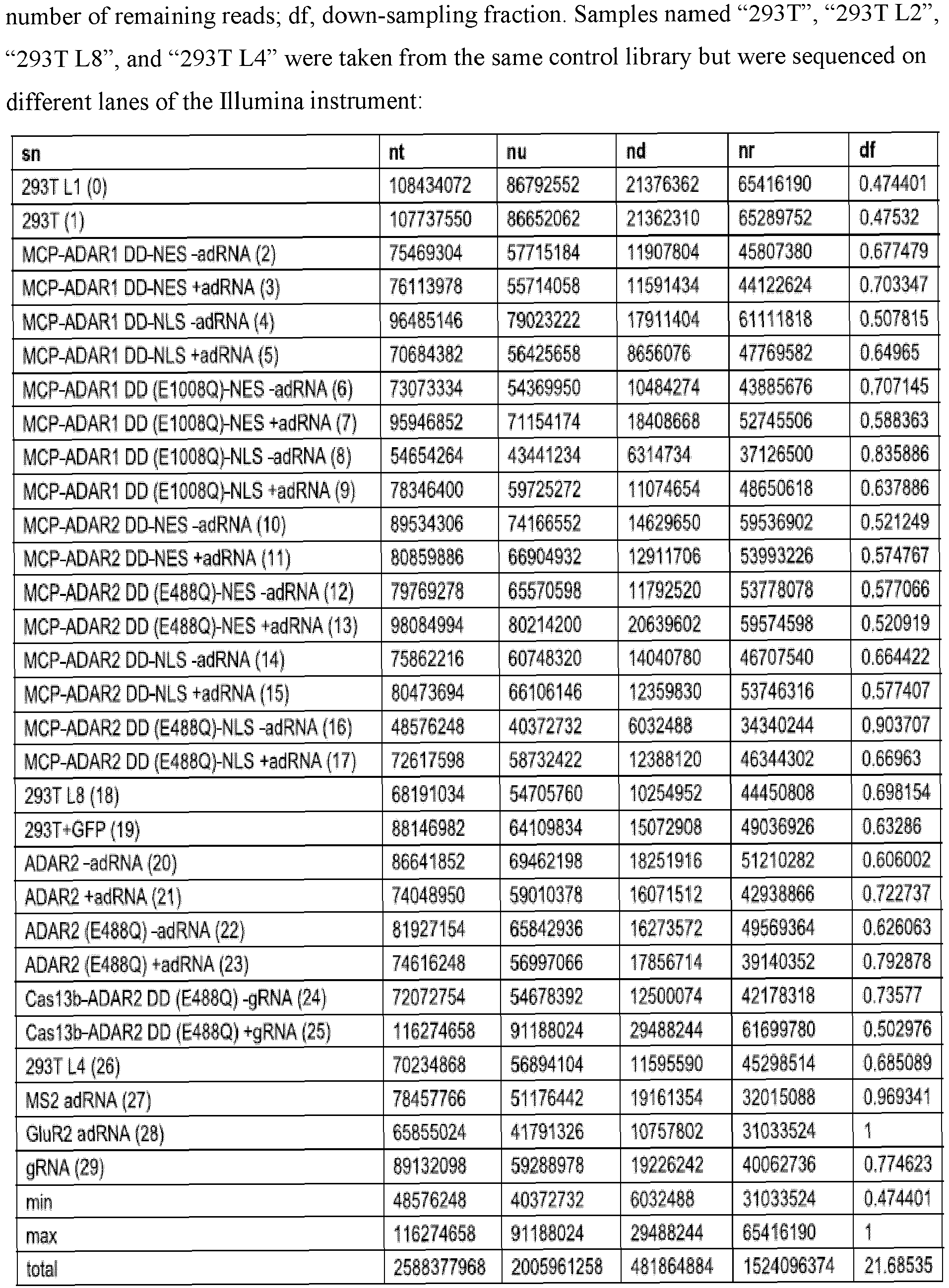

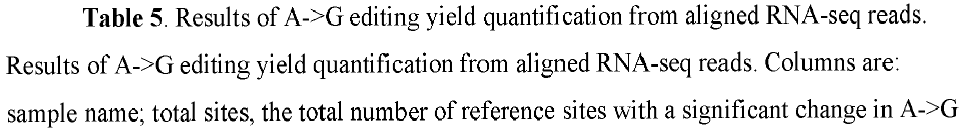

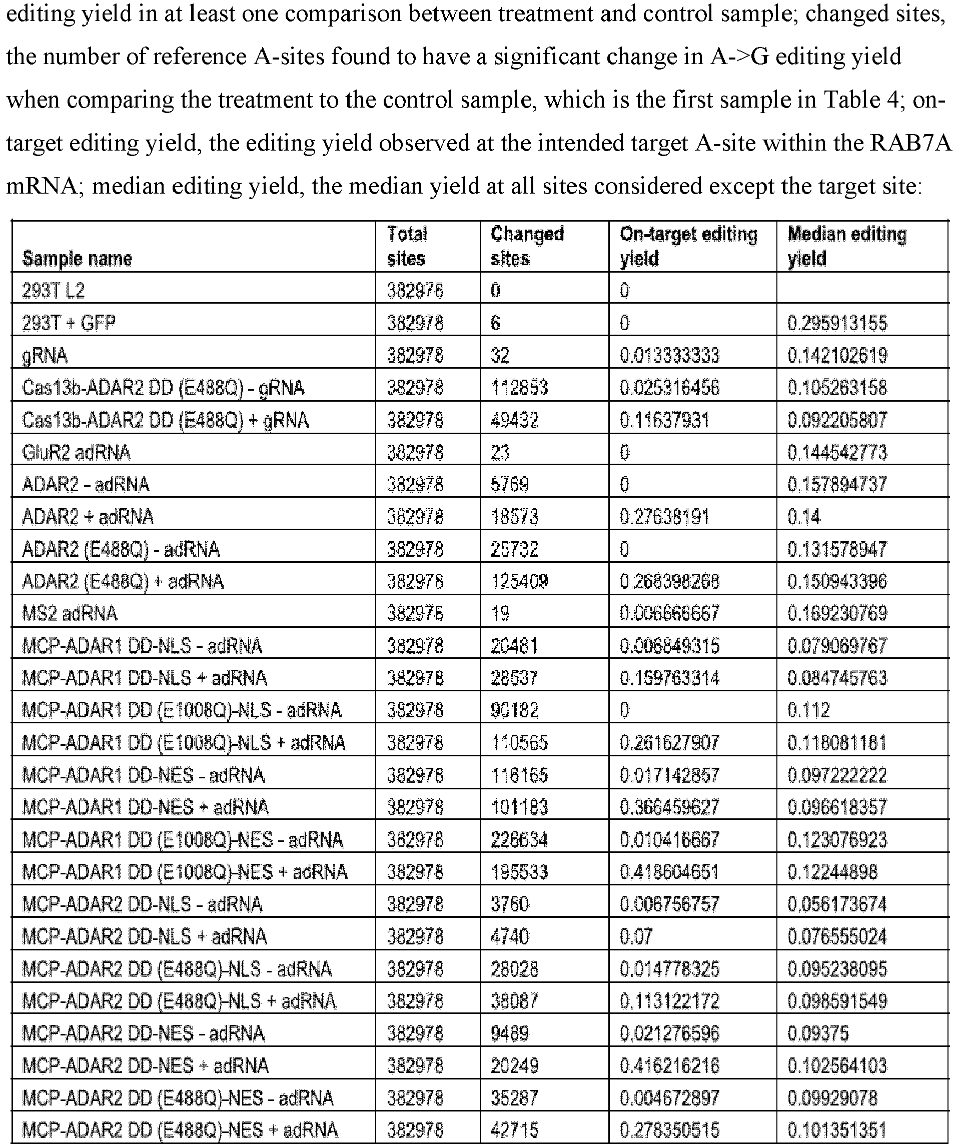

Following these *in vitro* studies, the system was evaluated for *in vivo* RNA targeting in gene therapy applications, utilizing the adRNA cum exogenous ADAR expression construct versions, as those consistently enabled the highest *in vitro* RNA editing yields. The *mdx* mouse model for Duchenne muscular dystrophy (DMD) was first evaluated, which bears an ochre stop site in exon 23 of the dystrophin gene. This choice was additionally motivated by the fact that nonsense mutations in general may be responsible for nearly 11% of described gene lesions causing inheritable human disease, and close to 20% of disease-associated single base substitutions that affect the coding regions of genes. Thus, validation of an RNA editing strategy here can have broad therapeutic application. Towards this, the RNA editing of stop codons was first optimized *in vitro* (**FIG. 43**). Notably, it was observed that addition of a second copy of the adRNA significantly improved the targeting efficiencies (**FIG. 43C**), and thus in the *in vivo* studies a dual-adRNA delivery approach was utilized. The constructs were then packaged into AAV8, and injected 2E+12 vector genomes (vg)/muscle into the tibialis anterior (TA) or gastrocnemius of *mdx* mice. To further benchmark the approach, the *mdx* mice were concurrently targeted via CRISPR-Cas9 based excision of exon 23 (**FIG. 33A**). Four or eight weeks post injection, TA and gastrocnemius muscles were collected from *mdx* mice, wild type mice, mice treated with adRNA targeting and non-targeting controls, and CRISPR-Cas9. Immunofluorescence staining revealed clear restoration of dystrophin expression via targeted RNA editing (**FIG. 33B****,** **FIG. 44A**). In addition, nNOS activity was also restored at the sarcolemma (**FIG. 33B****,** **FIG. 44A**). RNA editing yields (TAA->TGG/TAG/TGA) of up to 3.6%, and TAA->TGG up to 2.4% were observed in treated mice (**FIG. 33C****,** **FIG. 43E**). Western blots of the treated muscles confirmed the immunofluorescence observations, demonstrating 1-2.5% protein restoration. (**FIG. 44B**). As benchmark, muscles injected with vectors bearing CRISPR-Cas9 also expectedly led to restoration of dystrophin expression in a subset of the muscle cells (**FIG. 33B**), with Western blots of the treated muscles confirming up to 10% protein restoration. (**FIG. 44C**).

To further confirm the efficacy of this approach, ADAR mediated RNA editing was next evaluated in an independent mouse model of human disease, the male sparse fur ash (spf^{ash}) mouse model of ornithine transcarbamylase (OTC) deficiency. The spf^{ash} mice harbor a G->A point mutation in the last nucleotide of the fourth exon of the OTC gene, which leads to OTC mRNA deficiency and production of a mutant protein. Recent studies have demonstrated the use of CRISPR-Cas9 and homologous recombination based strategies for robust correction of this mutation in neonatal mice. To test the effectiveness of the system in editing the point mutation in spf^{ash} OTC mRNA (**FIG. 33D**), the constructs were evaluated *in vitro* (**FIG. 45A**). The constructs were packaged into AAV8, which has high liver tropism, and injected 2.5E+12 vg/mouse in 10-12 week old spf^{ash} mice. Three to four weeks post injection, liver samples were collected from spf^{ash} mice, wild-type litter mates, and spf^{ash} mice treated with the ADAR2 targeting and non-targeting vectors and evaluated corresponding editing efficiency via NGS. Notably, upon delivery of the adRNA and the ADAR2, 0.8-4.7% edited mRNA was observed amongst the correctly spliced OTC mRNA, and interestingly adRNA alone resulted in low but significant RNA editing yields (**FIG. 33E**). Moreover, upon the delivery of the hyper-active ADAR2 mutant (E488Q), a high edited fraction (4.6-33.8%) was observed in the correctly spliced OTC mRNA (**FIG. 33e****,** **FIG. 45B**), 4.6-8.2% in the OTC pre-mRNA (**FIG. 45C**), and confirmed a reduction in the incorrectly spliced product (**FIG. 45D**). Western blots of the treated liver samples confirmed partial (2.5-5%) restoration of OTC protein (**FIG. 45E**).

Taken together, the results establish the utility of RNA-guided ADARs for *in vivo* RNA editing of point mutations. In some cases, sequence preferences of the ADAR enzymes, RNA folding, intrinsic half-life, localization, translation machinery, and resident RNA binding proteins can potentially impact accessibility and editability of target sites in the RNA, and can be important design parameters to consider for enabling efficacious targeting. For instance, in the *mdx* model, ADAR based RNA editing approaches can have to compete with nonsense mediated decay of mutant dystrophin mRNA, and also the requirement for effecting two A->I substitutions in the context of non-ideal flanking nucleotides to eliminate the premature stop codon and potential impact on RNA stability and function. Furthermore, in the spf^{ash} model, the need to target the transient OTC pre-mRNA can entail rapid target engagement and editing. Further progress can also be needed addressing important limitations of the system such as the off-targets induced by intrinsic enzyme-RNA binding, processivity, promiscuity, stimulation of the interferon response by the delivery modalities themselves (such as lipid, nanoparticles or viral) leading in turn to increased endogenous ADAR expression, potential of adRNAs to induce RNAi, and also off-target hybridization of the antisense domain of the adRNA which can potentially have deleterious effects. In this regard, the studies revealed toxicity in mice systemically injected with the hyperactive ADAR mutants (**FIG. 46**). These studies can be critical to aid systematic improvement of the specificity and safety of this approach. Another important consideration while considering RNA targeting for gene therapy, especially via the use of non-integrating vectors, can be the necessity for periodic re-administration of the effector constructs, owing to the limited half-life of edited mRNAs and effectors. In this regard, compared to the CRISPR based RNA editing approaches, the RNA-guided ADAR strategy can be directly human therapeutics relevant, as versions of the same solely utilize effector RNAs and human proteins. Additionally, as ADARs are widely expressed, for instance, ADAR1 across most human tissues and ADAR2 in particular in the lung and brain, endogenous recruitment of these via adRNAs bearing long-antisense domains (as demonstrated in **FIG. 32****,** **FIG. 33E** and **FIG. 34****,** **FIG. 36**) presents a very attractive strategy for efficacious RNA editing. With progressive improvements, this toolset can have broad implications for diverse basic science and therapeutic applications.

**FIG. 32****: Engineering programmable RNA editing and characterizing specificity profiles: (A)** Schematics of RNA editing via constructs utilizing the full length ADAR2 and an engineered adRNA derived from the GluR2 transcript, or MS2 Coat Protein (MCP) fusions to the ADAR1/2 deaminase domains and the corresponding MS2 hairpin bearing adRNA. **(B)** Comparison of RNA editing efficiency of the endogenous RAB7A transcript by different RNA editing constructs quantified by Sanger sequencing (efficiency calculated as a ratio of Sanger peak heights G/(A+G)). Experiments were carried out in HEK 293T cells. Values represent mean +/- SEM (n=3). (C) Violin plots representing distributions of A->G editing yields observed at reference sites where at least one treatment sample was found to have a significant change (Fisher's exact test, FDR = 1%) in editing yield relative to the control sample. Blue circles indicate editing yields at the target A-site within the RAB7A transcript. Black dots represent median off-target editing yields. To better visualize the shapes of the distributions, their maximum extent along the y-axis was equalized across plots, and were truncated at 60% yield.

**FIG. 33****: *In vivo* RNA editing in mouse models of human disease: (A)** Schematic of the DNA and RNA targeting approaches to restore dystrophin expression in the *mdx* mouse model of Duchenne Muscular Dystrophy: (i) a dual gRNA-CRISPR based approach leading to in frame excision of exon 23 and (ii) ADAR2 and MCP-ADAR1 based editing of the ochre codon. **(B)** Immunofluorescence staining for dystrophin in the TA muscle shows partial restoration of expression in treated samples (intra-muscular injections of AAV8-ADAR2, AAV8-ADAR2 (E488Q), and AAV8-CRISPR). Partial restoration of nNOS localization is also seen in treated samples (scale bar: 250µm). **(C)** *In vivo* TAA->TGG/TAG/TGA RNA editing efficiencies in corresponding treated adult *mdx* mice. Values represent mean +/- SEM (n=4, 3, 7, 3, 3, 10, 3, 4 independent TA muscles respectively). **(D)** Schematic of the OTC locus in the spf^{ash} mouse model of Ornithine Transcarbamylase deficiency which have a G->A point mutation at a donor splice site in the last nucleotide of exon 4, and approach for correction of mutant OTC mRNA via ADAR2 mediated RNA editing. **(E)** *In vivo* RNA correction efficiencies in the correctly spliced OTC mRNA in the livers of treated adult spf^{ash} mice (retro-orbital injections of AAV8-ADAR2 and AAV8-ADAR2 (E488Q)). Values represent mean +/- SEM (n=4, 4, 3, 3, 4, 5 independent animals respectively).

### Vector design and construction

One or two copies of the adRNAs were cloned into an AAV vector containing a human U6 and mouse U6 promoter along with a CMV promoter driving the expression of the enzyme. To construct the GFP reporters - GFP-Amber, GFP-Ochre and GFP-Opal, three gene blocks were synthesized with `TAG', `TAA' and 'TGA' respectively replacing the Y39 residue of the wild type GFP and were cloned downstream of a CAG promoter. To construct the OTC and DMD reporters, 200 bp fragments of the spf^{ash} OTC and *mdx* DMD transcript bearing the target adenosine(s) to be edited were cloned downstream of the CAG promoter.

### Mammalian cell culture and transfection

All HEK 293T cells were grown in Dulbecco's Modified Eagle Medium supplemented with 10% FBS and 1% Antibiotic-Antimycotic (Thermo Fisher) in an incubator at 37 °C and 5% CO₂ atmosphere. All *in vitro* transfection experiments were carried out in HEK 293T cells using the commercial transfection reagent Lipofectamine 2000 (Thermo Fisher). All *in vitro* RNA editing experiments involving a reporter were carried out in 24 well plates using 400ng of reporter plasmid and 800ng of the adRNA+enzyme plasmid. All *in vitro* RNA editing experiments targeting an endogenous transcript were carried out in 24 well plates using 800ng of the adRNA/Enzyme plasmid. dCas13b-ADAR2*_{DD}*E488Q based RNA editing experiments were carried out using 800ng of the enzyme plasmid (Addgene #103864) as well 800 ng of the gRNA plasmid. Cells were transfected at 25-30% confluence and harvested 60 hours post transfection for quantification of editing. Chemically synthesized adRNAs (synthesized via IDT or Synthego) were transfected using Lipofectamine 3000 (Thermo Fisher) at an amount of 20 pmol/well.

### Production of AAV vectors

AAV8 particles were produced using HEK 293T cells via the triple transfection method and purified via an iodixanol gradient. Confluency at transfection was about 80%. Two hours prior to transfection, DMEM supplemented with 10% FBS was added to the HEK 293T cells. Each virus was produced in 5 x 15 cm plates, where each plate was transfected with 7.5 ug of pXR-8, 7.5 of ug recombinant transfer vector, 7.5 ug of pHelper vector using PEI (1ug/uL linear PEI in 1xDPBS pH 4.5, using HCl) at a PEI:DNA mass ratio of 4:1. The mixture was incubated for 10 minutes at RT and then applied dropwise onto the cell media. The virus was harvested after 72 hours and purified using an iodixanol density gradient ultracentrifugation method. The virus was then dialyzed with 1 x PBS (pH 7.2) supplemented with 50 mM NaCl and 0.0001% of Pluronic F68 (Thermo Fisher) using 50kDA filters (Millipore), to a final volume of ~1 mL and quantified by qPCR using primers specific to the ITR region, against a standard (ATCC VR-1616).
AAV-ITR-F: 5'-CGGCCTCAGTGAGCGA-3' (SEQ ID NO: 149) and
AAV-ITR-R: 5'-GGAACCCCTAGTGATGGAGTT-3' (SEQ ID NO: 150).

### RNA isolation and Next Generation Sequencing library preparation

RNA from animal tissue was extracted using the RNeasy Plus Universal Mini Kit (Qiagen), according to the manufacturer's protocol. RNA from cells was extracted using the RNeasy Mini Kit (Qiagen). cDNA was synthesized from 500ng RNA using the Protoscript II First Strand cDNA synthesis Kit (NEB). Next generation sequencing libraries were prepared as follows. Briefly, 1ul of cDNA prepared above was amplified by PCR with primers that amplify about 150 bp surrounding the sites of interest using KAPA Hifi HotStart PCR Mix (Kapa Biosystems). PCR products were purified (Qiagen PCR Purification Kit/ Gel Extraction Kit) to eliminate byproducts. Libraries were constructed with NEBNext Multiplex Oligos for Illumina kit (NEB). 10 ng of input DNA was amplified with indexing primers. Samples were then pooled and loaded on an Illumina Miseq (150bp single-end run) or Hiseq (100bp paired-end run). Data analysis was performed using CRISPResso (Pinello, L. *et al.* 2016). A minimum of 100,000 reads were analyzed for all *in vivo* experiments. RNA-seq libraries were prepared from 300ng of RNA, using the NEBNext Poly(A) mRNA magnetic isolation module and NEBNext Ultra RNA Library Prep Kit for Illumina. Samples were pooled and loaded on an Illumina Hiseq (100bp paired-end run).

### Quantification of OTC mRNA editing yields in the spf^{ash} mice

The spf^{ash} mice bear three forms of OTC RNA: the pre-mRNA, the correctly spliced mRNA and an incorrectly spliced, elongated mRNA formed due to the use of a cryptic splice site 48 base pairs into intron 4. Let the total number of the correctly spliced mRNA be X, incorrectly spliced variant be Y and the pre-mRNA be Z. Xe, Ye and Ze denote the A->G edited mRNA in the three forms. The mRNA editing yield ideally can be calculated as (Xe+Ye+Ze)/(X+Y+Z). However, since it is not possible to amplify the spliced and pre-mRNA variants using the same primers, **FIG. 34E** shows the fraction of edited transcripts in the correctly spliced mRNA (Xe/X) which will in turn be translated to produce the OTC protein. In addition, **FIG. 48C** shows the fraction of edited transcripts in the pre-mRNA (Ze/Z). This fraction, upon correct splicing will contribute to formation of OTC protein. Finally, the incorrectly spliced mRNA results in the production of a protein elongated by 16 amino acids which is selectively degraded. In **FIG. 48D,** bands corresponding to X and Y are shown.

### Animal experiments

All animal procedures were performed in accordance with protocols approved by the Institutional Animal Care and Use Committee (IACUC) of the University of California, San Diego. Mice were acquired from Jackson labs. AAVs were injected into the gastrocnemius or TA muscle of *mdx* mice (C57BL/10ScSn-*Dmd^{mdx}*/J) using 2E+12 vg/muscle. AAVs were injected into spf^{ash} mice (B6EiC3Sn a/*A-Otc^{spf-ash}*/J) via retro-orbital injections using 2.5E+12 vg/mouse. Mice that appeared to have a rough hair coat, moved slowly and appeared slightly hunched were termed as sick mice and euthanized.

### Immunofluorescence

Harvested gastrocnemius or TA muscles were placed in molds containing OCT compound (VWR) and flash frozen in liquid nitrogen. 10 µm sections were cut onto pretreated histological slides. Slides were fixed using 4% Paraformaldehyde. Dystrophin and nNOS were detected with rabbit polyclonal antibodies against the C-terminal domain of dystrophin (1:200, Abcam 15277) and N-terminal domain of nNOS (1:100, Immunostar 24431) respectively, followed by a donkey anti-rabbit Alexa 546 secondary antibody (1:400, Thermo Fisher).

### Western Blots

Muscle biopsies from *mdx* mice and liver biopsies from spf^{ash} mice were fragmented in RIPA buffer (Sigma) with a proteinase inhibitor cocktail (Roche) and incubated for 1 hour on ice with intermittent vortexing. Samples were centrifuged at 15500xg for 30 min at 4°C and the supernatant was isolated and quantified with a Pierce Coomassie Plus (Bradford) assay kit (Thermo Fisher). Protein isolate was mixed with 4x Laemmli Loading buffer (Biorad) and 2-Mercaptoethanol (Biorad) and boiled at 100°C for 10 min. 100 µg total protein from muscle biopsies or 60 ug from liver biopsies was loaded into each well of a4-15% Mini Protean TGX gel (Biorad) with Tris-Glycine-SDS buffer (Biorad) and electrophoresed for 60 min at 100 V. Protein from muscle biopsies was transferred to nitrocellulose membranes overnight at 34V while that from liver biopsies was transferred at 65V for 1hour 30 minutes in a 1X tris-glycine transfer buffer containing 10% methanol and 0.1% SDS at 4°C. The blot was blocked for 1 hour in 5% milk-TBST. Blots were probed with rabbit anti-dystrophin (1:200, Abcam 15277), rabbit anti-GAPDH (1:4000, Cell Signaling 2118S), rabbit anti-OTC (1:800, Abcam 203859) and mouse anti-ADAR2 (1:150, Santa Cruz Biotechnology 73409) overnight at 4°C in 5% milk-TBST. Blots were washed with TBST and then incubated with anti-rabbit or anti-mouse horseradish peroxidase-conjugated secondary antibodies (Cell Signaling) for 1 hour in 5% milk-TBST. After washing with TBST, blots were visualized using SuperSignal West Femto Chemiluminescent Substyeild (Thermo Fisher) and X-Ray films.

### Statistics and Reproducibility

*In vitro* experiments: *In vitro* experiments were carried out once with a minimum of 3 independent replicates. *In vivo* experiments: For the *mdx* mouse model, ADAR2 and MCP-ADAR1 (E1008Q) NLS based experiments were carried out twice. Both rounds of experiments yielded consistent RNA editing efficiencies, dystrophin immunofluorescence and dystrophin restoration as seen by western blots. ADAR2 (E488Q) and CRISPR-Cas9 based experiments were carried out once. For the spf^{ash} mouse model, experiments were carried out twice, based on the availability of mice. RNA editing efficiencies of the OTC transcript, both the spliced and pre-mRNA were consistent in both rounds of experiments. RT-PCR and Western blots were carried out on animals in experimental set 1.

### 1. Quantification of RNA A->G editing

### (a) RNA-seq read alignment

RNA-seq read pairs with 100 bases per read mate were aligned to the GRCh38 reference genome using STAR aligner version 2.6.0c (Dobin A et al 2013). The genome index was built using primary assembly annotations from GENCODE release 28 (GRCh38.p12). Default parameters were used to run STAR, except for the following relevant settings: readMapNumber=-1, alignSJoverhangMin=5, align SJDB overhangmin= 1, alignEndsType=EndToEnd, outFilterMismatchNmax=10, outFilterMultimapNmax=1, outSAMunmapped=None, outSAMmultNmax=1. The reads of the resulting uniquely aligned pairs were sorted by genomic coordinate using samtools sort (Li H. et al 2009). Duplicated read pairs were marked using samtools markdup and were removed from subsequent analysis. Tallies of total, aligned, duplicated, and remaining reads (not pairs) are reported for each sample in **Table 4.**

### (b) Selection of reference sites for quantification of editing yields

The assessment of sites with significant changes in A-to-G editing yields (see below) is sensitive to the number of uniquely aligned reads available for each sample. To minimize potential biases when comparing different samples in terms of significantly edited sites, the uniquely aligned reads for each HEK293T sample were down-sampled using samtools view with option -s and the down-sampling fractions reported in **Table 4.** These fractions were calculated by dividing the smallest number of uniquely aligned reads among samples by the number of uniquely aligned reads available for the sample being down-sampled. Down-sampling was not performed on the reads of the control sample, the first in **Table 4.** The down-sampled reads where then processed using samtools mpileup. The output of this tool was parsed to extract the counts of each base found in the aligned reads at each A-site and T-site in the GRCh38 reference genome sequence. Insertions and deletions were ignored. Reference sites with read coverage less than 10 were omitted from downstream analysis. The number of remaining reference A- and T-sites with read coverage of at least 10 varied by -15% across the samples listed in **Table 4.** Without down-sampling, such number was found to vary by ~50%. From the reference A- and T-sites with read coverage of at least 10, a final list of total sites (A-sites and T-sites) was selected by choosing those sites that were common to all samples and for which at least one G or C was observed at a reference A- or T-site, respectively, in the aligned reads of at least one sample. The other sites, those not common to all samples or with zero observed editing events in all samples, were discarded.

### (c) Assessment of significant changes in A-to-G editins yields

To uncover significant changes in A-to-G editing yields, several pairs of control and treatment samples were considered. For each pair, the control sample was the first sample listed in in **Table 4,** while the treatment sample was one of the samples shown in **FIG. 32****.** For each pair of compared samples, and for each reference A-site selected as described above, a Fisher exact test was carried out using a 2x2 contingency matrix C with entries defined as follows: C_{1,1} = count of bases other than G observed in the control sample, C_{2,1} = count of G bases observed in the control sample, C_{1,2} = count of bases other than G observed in the test sample, C_{2,2} = count of G bases observed in the test sample. A similar contingency matrix was used for each selected reference T-site, except that G was replaced with C in the above definitions. The p-values calculated for all selected reference sites and for a given comparison of samples were adjusted for multiple testing using the Benjamini-Hochberg method. A-sites and T-sites with adjusted p-values less than a false discovery rate (FDR) of 1% and with a fold change of at least 1.1 in editing yield were deemed to have a significant change in A-to-G editing yield on forward and reverse transcripts, respectively. The counts of these sites for each comparison of samples are shown as *N*_{sig} in **FIG. 38** - **FIG. 42,** and are reported under the column "changed sites" in **Table 5.** The total number of reference sites with a significant change in A-to-G editing yield was computed. The editing yields at these sites were used to construct the distributions shown in **FIG. 32****.** The on-target A-to-G editing yields shown as blue circles in **FIG. 32** and **FIG. 38** - **FIG. 42** were estimated for each sample as C_{2,2} / (C_{1,2} + C_{2,2}) using counts observed at the intended target A-site in the RAB7A transcript. These values are reported under the column "editing yield" in **Table 5.** The 1-based genomic coordinate of the intended target A-site was found to be chr3:128814202 by submitting the following sequence to BLAT after selecting reference assembly hg38:
AGCGGCAGTATTCTGTACAGTAGACACAAGAATTATGTACGCCTTTTATCA (SEQ ID NO:151).

**FIG. 8** - Engineering GluR2 adRNAs: scaffold domain engineering. Sequence information of adRNA scaffolds: ADAR recruiting domain, antisense RNA targeting domain and the cytosine mismatch highlighted. Base pairs mutated to create stabilized scaffolds are numbered and highlighted in red, and the editing inducer element motif is shown in green. Quantification of editing efficiency of thus generated scaffolds for the OTC reporter transcript quantified by Sanger sequencing is shown. Values represent mean +/- SEM (n=3). Experiments were carried out in HEK 293T cells.

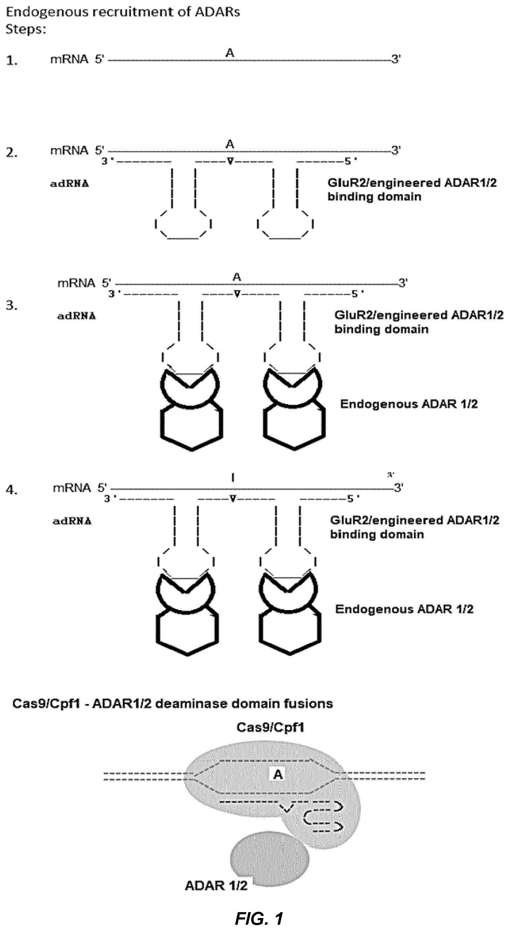

**FIG. 34** - Engineering GluR2 adRNAs: antisense domain engineering. (a) Optimization of adRNA antisense region using adRNA scaffold 2: length and distance from the ADAR2 recruiting region were systematically varied. Values represent mean +/- SEM (n=3). (b) U6 promoter transcribed adRNAs with progressively longer antisense domain lengths, in combination with zero, one or two GluR2 domains were evaluated for their ability to induce targeted RNA editing with or without exogenous ADAR2 expression. Values represent mean +/- SEM (n=3). A portion of this data is reused in Fig. 1b. All the above experiments were carried out in HEK 293T cells. (c) Experimental confirmation of expression of endogenous ADAR1 and ADAR2 (relative to GAPDH) in HEK 293T and HeLa cell lines. Observed levels were similar to those documented in The Human Protein Atlas (see world-wide-web at proteinatlas.org)

**FIG. 35** - Engineering MS2 adRNAs. (a) Systematic evaluation of antisense RNA targeting domain of the MS2 adRNA. Values represent mean +/- SEM (n=3). (b) On-target RNA editing by MCP-ADAR2 DD-NLS requires co-expression of the MS2 adRNA. Values represent mean +/- SEM (n=3). Experiments were carried out in HEK 293T cells.

**FIG. 36** - Analysis of RNA editing yields across a panel of targets. (**A**) Comparison of RNA editing efficiency of the OTC reporter transcript by GluR2 adRNA and MS2 adRNA guided RNA editing constructs as well as the Cas13b based REPAIR construct. Values represent mean +/- SEM (n=6 for reporter and Cas13b based constructs, n=3 for other constructs). (**B**) Chemically synthesized adRNAs versions were tested against a panel of mRNAs with or without exogenous ADAR2 expression. The exact chemical modifications are stated in the figure along with the source of adRNA. Values represent mean +/- SEM (n=3). (**C**) Analysis of RNA editing yields across a spectrum of endogenous targets chosen to cover a range of expression levels. U6 transcribed long adRNAs with none or two GluR2 domains were also evaluated against multiple endogenous mRNA targets with or without exogenous ADAR2 expression. Editing is observed at tested loci even in the absence of exogenous ADAR2 expression. Values represent mean +/- SEM (n=3). Experiments were carried out in HEK 293T cells.

**FIG. 37** - ADAR2 variants and their impact on editing and specificity. (**A**) Comparison of on target RNA editing and editing in flanking adenosines of the RAB7A transcript by GluR2 adRNA and MS2 adRNA guided RNA editing constructs as well as the Cas13b based REPAIR construct. Mean (n=3) editing yields are depicted. Experiments were carried out in in HEK 293T cells and editing efficiency was calculated as a ratio of Sanger peak heights G/(A+G). (**B**) ADAR2 (E488Q) exhibits higher efficiency than the ADAR2 in the *in vitro* editing of the spf^{ash} OTC reporter transcript (p=0.037, unpaired t-test, two-tailed); values represent mean +/- SEM (n=3), and (**C**) *mdx* DMD reporter transcript (p=0.048, p=0.012 respectively, unpaired t-test, two-tailed); values represent mean +/- SEM (n=3). (d) Comparison of the editing efficiency and specificity profiles of the ADAR2, ADAR2 (E488Q) and the ADAR2 (Δ1-138) for the OTC reporter transcript (upper panel) and endogenous RAB7A transcript (lower panel). Heatmap indicates the A->G edits in the vicinity of the target (red arrow). Values represent mean +/- SEM (n=3). Experiments were carried out in HEK 293T cells and editing efficiency was calculated as a ratio of Sanger peak heights G/(A+G).

**FIG. 38** - Transcriptome scale specificity profiles of RNA editing approaches (Cas13b-ADAR REPAIR +/- gRNA). 2D histograms comparing the transcriptome-wide A->G editing yields observed with each Cas13b-ADAR2 construct (y-axis) to the yields observed with the control sample (x-axis). Each histogram represents the same set of 8,729,464 reference sites, where read coverage was at least 10 and at least one putative editing event was detected in at least one sample. Bins highlighted in red contain sites with significant changes in A->G editing yields when comparing treatment to control sample. Red crosses in each plot indicate the 100 sites with the smallest adjusted p-values. Blue circles indicate the intended target A-site within the RAB7A transcript. Large counts in bins near the lower-left corner likely correspond not only to low editing yields in both test and control samples, but also to sequencing errors and alignment errors. Large counts in bins near the upper-right corner of each plot likely correspond to homozygous single nucleotide polymorphisms (SNPs), as well as other differences between the reference genome and the genome of the HEK293T cell line used in the experiments.

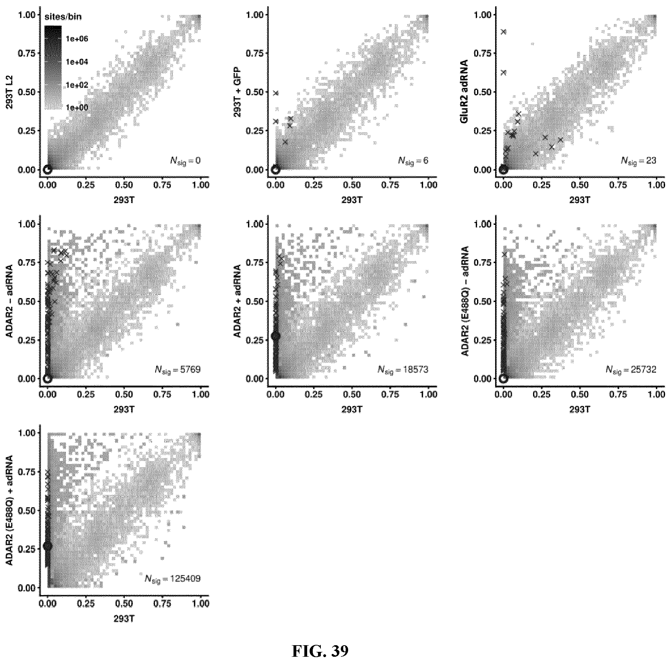

**FIG. 39** - Transcriptome scale specificity profiles of RNA editing approaches (ADAR2 +/- adRNA). The version used for these studies is GluR2 adRNA(1,20,6). 2D histograms comparing the transcriptome-wide A->G editing yields observed with each ADAR construct (y-axis) to the yields observed with the control sample (x-axis). More details are provided in **FIG. 38****.**

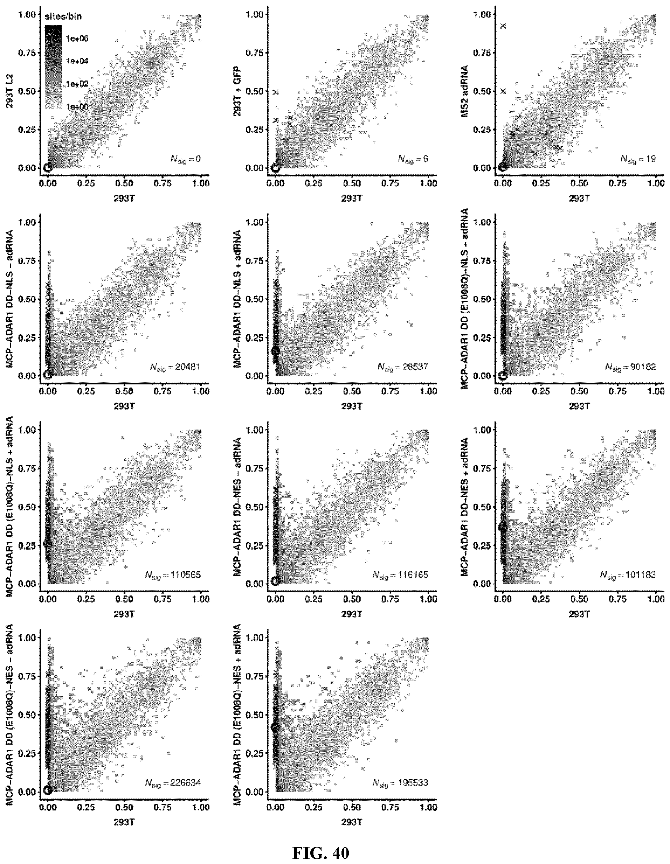

**FIG. 40** - Transcriptome scale specificity profiles of RNA editing approaches (MCP-ADAR1 DD +/- adRNA). 2D histograms comparing the transcriptome-wide A->G editing yields observed with each ADAR construct (y-axis) to the yields observed with the control sample (x-axis). More details are provided in **FIG. 38****.**

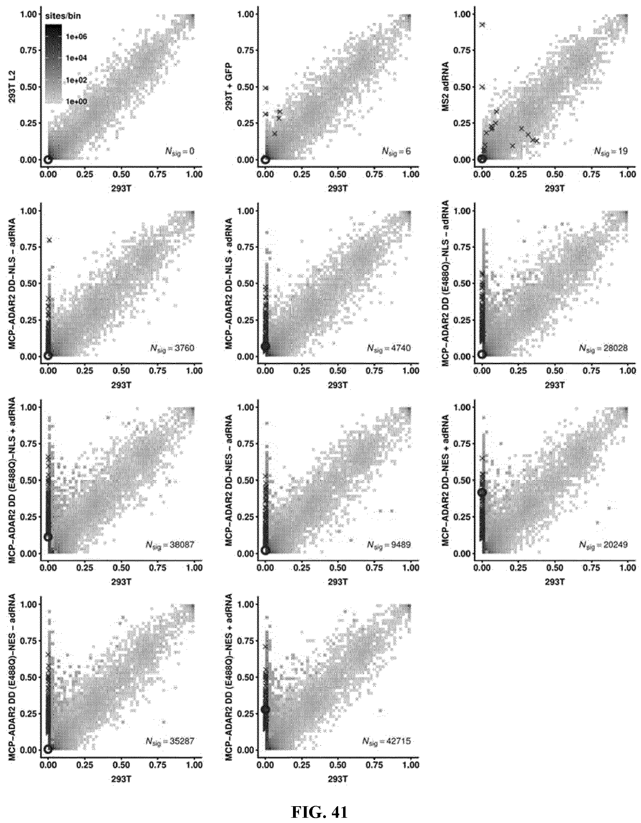

**FIG. 41** - Transcriptome scale specificity profiles of RNA editing approaches (MCP-ADAR2 DD +/- adRNA). 2D histograms comparing the transcriptome-wide A->G editing yields observed with each ADAR construct (y-axis) to the yields observed with the control sample (x-axis). More details are provided in **FIG. 38****.**

**FIG. 42** - Variation of transcriptome scale editing specificity with construct features. (**A**) Each point in the box plots corresponds to the fraction of edited sites for one of the MCP-ADAR constructs listed in **FIG. 32****.** The fraction of edited sites for each construct was calculated by dividing the number of reference sites with significant changes in A-to-G editing yield (see Table 3) by the total number 8,729,464 of reference sites considered. Construct features indicated on the horizontal axes were compared using the Mann-Whitney U test, yielding p-values of 0.16 for NLS vs. NES, 0.0070 for ADAR1 vs. ADAR2, 0.72 for "- adRNA" vs. "+ adRNA", and 0.038 for "ADAR WT" vs. "ADAR E>Q" (n=8 for all conditions). (**B**) 2D histograms comparing the transcriptome-wide A->G editing yields observed with each construct (y-axis) to the yields observed with the control sample (x-axis). More details are provided in **FIG. 38****.** Inset shows violin plots representing distributions of A->G editing yields observed at reference sites where at least one treatment sample was found to have a significant change (Fisher's exact test, FDR = 1%) in editing yield relative to the control sample. Blue circles indicate editing yields at the target A-site within the RAB7A transcript. To better visualize the shapes of the distributions, their maximum extent along the y-axis was equalized across plots, and were truncated at 60% yield. Samples here correspond to 293Ts transfected with long antisense domain bearing adRNAs that can enable RNA editing via exogenous and/or endogenous ADAR recruitment.

**FIG. 43** - Optimization and evaluation of dystrophin RNA editing experiments *in vitro* and *in vivo* in *mdx* mice. (**A**) Schematic of RNA editing utilizing the full length ADAR2 along with an engineered adRNA or a reverse oriented adRNA (radRNA); (ii) RNA editing efficiencies of amber and ochre stop codons, in one-step and two-steps. Experiments were carried out in HEK 293T cells. Values represent mean +/- SEM (n=3). (**B**) RNA editing of ochre codons requires two cytosine mismatches in the antisense RNA targeting domains of adRNA or radRNA to restore GFP expression. Experiments were carried out in HEK 293T cells. Values represent mean +/- SEM (n=3). (**C**) Schematic of the AAV vectors utilized for *in vivo* delivery of adRNA and ADAR2, and *in vitro* optimization of RNA editing of amber and ochre stop codons in the presence of one or two copies of the adRNA, delivered via an AAV vector (p=0.0003, p=0.0001, p=0.0015 respectively, unpaired t-test, two-tailed). Experiments were carried out in HEK 293T cells. Values represent mean +/- SEM (n=3 for reporters, n=6 for other conditions). (**D**) Representative Sanger sequencing plot showing editing of the ochre stop codon (TAA->TGG) in the *mdx* DMD reporter transcript (quantified by NGS). Experiments were carried out in HEK 293T cells (n=3). (**E**) Representative example of *in vivo* RNA editing analyses of treated *mdx* mice (quantified using NGS).

**FIG. 44** - Immunofluorescence and Western blot analyses of *in vivo* dystrophin RNA editing experiments in *mdx* mice. **(A)** Immunofluorescence staining for dystrophin in the TA muscle shows partial restoration of expression in treated samples (intra-muscular injections of AAV8-ADAR2, AAV8-ADAR2 (E488Q), AAV8-MCP-ADAR1 (E1008Q) NLS). Partial restoration of nNOS is localization also seen in treated samples (scale bar: 250µm). **(B)** Western blots showing partial recovery of dystrophin expression (1-2.5%) in TA muscles of *mdx* mice injected with both components of the editing machinery, the enzyme and adRNA, and stable ADAR2 expression in injected TA muscles up to 8 weeks post injections. **(C)** Western blot showing partial restoration of dystrophin expression (10%) using AAV8-CRISPR.

**FIG. 45** - Optimization and evaluation of OTC RNA editing experiments *in vitro* and *in vivo* in spf^{ash} mice. (**A**) Representative Sanger sequencing plot showing correction of the point mutation in the spf^{ash} OTC reporter transcript (quantified using NGS). Experiments were carried out in HEK 293T cells (n=3). (**B**) Representative example of *in vivo* RNA editing analyses of treated spf^{ash} mice showing correction of the point mutation in the correctly spliced OTC mRNA (quantified using NGS). (**C**) *In vivo* RNA correction efficiencies in the OTC pre-mRNA in the livers of treated adult spf^{ash} mice (retro-orbital injections of AAV8-ADAR2 and AAV8-ADAR2 (E488Q). Values represent mean +/- SEM (n=4, 4, 3, 3, 4, 5 independent animals respectively). (**D**) PCR products showing the correctly and incorrectly spliced OTC mRNA. The incorrectly spliced mRNA is elongated by 48 base pairs. Fraction of incorrectly spliced mRNA is reduced in mice treated with adRNA+ADAR2 (E488Q). (**E**) Western blot for OTC shows partial restoration (2.5%-5%) of expression in treated adult spf^{ash} mice and stable ADAR2 (E488Q) expression three weeks post injections.

**FIG. 46** - Toxicity analyses of *in vivo* RNA editing experiments. Summary of animal experiments documenting the route of AAV administration, construct delivered, and health of injected mice 3 weeks post injections.

### Example 5

Disclosed herein are the results of experiments in an *mdx* mouse model of Duchenne muscular dystrophy using an E100Q mutant of ADAR1 (comprised in MCP-ADAR1 (E100Q)). In some cases, this mutant can improve editing yields *in vivo* compared to ADAR2 and an E488Q mutant of ADAR2. Further disclosed herein is an application of the ADAR system in a manner to alter splicing patterns by editing a splice acceptor site or branch point in an intron, thereby resulting in exon skipping. In addition, methods of using APOBECs are contemplated and exemplified. Demonstrated herein are examples in which the creation of local structure of alipoprotein B mRNA that ACF-APOBEC complex binds to, at an mRNA of interest, can enable C → T RNA editing. Further disclosed herein are method of utilizing ADAR enzymes for programmable editing of both RNA and DNA.

To utilize the ADAR editing system, with or without exogenous ADAR1 or ADAR2, adRNAs can be generated. The examples provided specific adRNAs of interest for a given target. Further, provided herein are exemplary chemically synthetized adRNAs with 2'-O-methyl 3' phosphothiorate modifications in the first and last 3, 6, 9, or 12 nucleotides, and/or 2'-O-methyl modifications throughout the antisense region except the 3 nucleotides centered around the mis-match site (underlined below), coupled with targeting moieties such as GalNc or cholesterol or cell-penetrating peptides can be used to engineer targeted RNA editing in cells or *in vivo* (especially in liver, lung and brain) with or without exogenous ADAR1/2 over-expression.

### General Design:

| adRNA22:      GTGGAAgAGgAgAACAATATGCTAAATGTTGTTcTcGTcTCCC
ACNNNNNNCNNNNNNNNNNNNNNN (SEQ ID NO:152)

### adRNA32:

GGTGTCGAGAAgAGgAgAACAATATGCTAAATGTTGTTcTcGTcTCCTCGACACCNN
NNNNCNNNNNNNNNNNNNNN (SEQ ID NO:153)

### specific examples:

adRNA22_RAB7A: GTGGAAgAGgAgAACAATATGCTAAATGTTGTTcTcGTcT
CCCACTGCCGCCAGCTGGATTTCCC (SEQ ID NO:154)

adRNA32_RAB7A: GGTGTCGAGAAgAGgAgAACAATATGCTAAATGTTGTTc
TcGTcTCCTCGACACCTGCCGCCAGCTGGATTTCCC (SEQ ID NO:155)

### adRNA22_CKDN2A:

GTGGAAgAGgAgAACAATATGCTAAATGTTGTTcTcGTcTCCCACCTCCTCCACCCG
ACCCCGGG (SEQ ID NO:156)

### adRNA32_CKDN2A:

GGTGTCGAGAAgAGgAgAACAATATGCTAAATGTTGTTcTcGTcTCCTCGACACCCT
CCTCCACCCGACCCCGGG (SEQ ID NO:157)

The examples provided herein below describe a multiplicity of steps taken by to further elucidate the embodiments disclosed herein, such as:
(1) delivering MCP-ADAR constructs with MS2-adRNA targeting the premature stop codon in the dystrophin transcript of *mdx* mice;
(2) testing out exon skipping via creation of point mutations in the *mdx* mouse model of Duchenne muscular dystrophy, with adRNAs delivered along with ADAR2/ADAR2(E488Q) or MS-adRNAs delivered with MCP-ADAR1/ADAR (E1008Q) or ms-adRNAs delivered with MCP-ADAR2/ADAR2 (E488Q) - utilized to create A~>G substitutions at the splice site;
(3) testing editing of splice site mutations in the OTC transcript of spc-ash mice;
(4) testing out editing of a mutant splice site to alter splicing patterns in the spf-ash mouse model of ornithine transcarbamylase deficiency;
(5) targeting the mouse SCN9A transcript to engineer insensitivity to pain by creating A→G substitutions at the splice sites to knockdown the SCN9A transcript;
(6) engineering APOBEC gRNAs to create the local structure of the alipoprotein B mRNA at a target mRNA of interest; and
(7) testing out ADARs in editing RNA and DNA by utilizing RNA, DNA, and RNA-DNA hybrids to recruit the ADAR.

In the examples, it was observed that enhanced RNA yields and protein expression were achieved via the use of MS2-adRNAs and MCP-ADAR1 (E1008Q) while editing the premature stop codon in a dystrophin transcript of *mdx* mice. Upon delivery of the ADAR2 (E488Q) with an adRNA, a reduction in incorrectly spliced OTC mRNA in an spf-ash mouse model was also confirmed.

The disclosure thus describes a system that can enable site-specific A->G editing of RNA. Such an approach can be used to edit splice acceptor sites and branch points to alter splicing. This can also enable exon skipping. Further embodiments contemplate use of A->G editing of DNA. In addition, the disclosure provides the potential for C->T edits in RNA via the use of APOBEC1 expressed along with ACF1.

The disclosure describes the first site-specific RNA editing *in vivo.* In fact, utilization of MCP-ADAR1 (E1008Q) demonstrates higher editing efficiencies than prior constructs in the *mdx* mouse model of muscular dystrophy. Further test, both *in vitro* and *in vivo,* examiner (1) RNA editing for altering splicing, as well as (2) efficacy of C->T editing via APOBECs along with the overexpression of ACF and (3) ADARs for editing DNA. 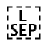

Compared to other ADAR2 systems (*e.g*. Stafforst, Zhang, and Rosenthal labs) and Cas13d based inhibition of splicing, the present ADAR system is unique.

As described herein, the ADAR system comprise, or alternatively consist essentially of, or yet further consist of: a RNA targeting domain complementary to the target RNA and one or more ADAR recruiting domains that enable recruitment of ADARs. Upon introduction of these components, the ADAR enzyme can catalyze the conversion of a target adenosine to inosine and thereby repair point mutations. The disclosure describes the use of ADAR2 or MCP-ADAR1/2-NLS or their hyperactive mutants to create a A->G edits at splice acceptor sites and/or branch point in introns to engineer exon skipping. Skipping symmetric exons results in formation of a truncated protein. This strategy can be used to skip exon 23 in the dystrophin transcript of the *mdx* mouse model of DMD which bears a premature stop codon in this exon. Skipping exon 23 results in the translation of a truncated dystrophin protein which is functional. In addition, skipping asymmetric exons leads to frameshift mutations. Thus, by skipping essential exons or asymmetric exons it can be possible to engineer gene knockdowns. In addition, editing of the start codon ATG and Kozak/Shine-Dalgarno sequences, can also help alter translation efficiencies and result in knockdown of genes. Exon skipping strategy can be used to target the SCN9A transcript to engineer insensitivity to pain. The local structure of the apolipoprotein B mRNA that the ACF-APOBEC complex binds to, at the mRNA target of interest, can be created for carefully positioning the C to be edited at a position analogous to the naturally occurring site in the apolipoprotein B mRNA. This is achieved by overexpression of a pair of adRNAs to create an apolipoprotein B mRNA like structure that can be edited by overexpression of MCP-ACF1 and APOBEC 1.

### Example 6 - Exon skipping via creation of splice acceptor and/or branch point mutations

The disclosure demonstrates the use of ADAR2 or MCP-ADAR1/2-NLS or their hyperactive mutants to create a A->G edits at splice acceptor sites and/or branch point in introns to engineer exon skipping. Skipping symmetric exons results in formation of a truncated protein. In the present example, this strategy can be used to skip exon 23 in the dystrophin transcript of the *mdx* mouse model of DMD which bears a premature stop codon in this exon. Skipping exon 23 results in the translation of a truncated dystrophin protein which is functional. In addition, skipping asymmetric exons leads to frameshift mutations. Thus, by skipping essential exons or asymmetric exons it can be possible to engineer gene knockdowns. In addition, editing of the start codon ATG and Kozak/Shine-Dalgarno sequences, helps alter translation efficiencies and result in knockdown of genes. This strategy can be used to target the SCN9A transcript to engineer insensitivity to pain.

Exemplary sequences for achieving this end are provided herein below.

### SEQUENCE SET 1: Exemplary adRNA Sequences

adRNA sequences for exon 23 skipping in the dystrophin transcript in *mdx* mice (SEQ ID NO:158-159)

| | |
|---|---|
| adDM D-I22-E23 | GTGGAATAGTATAACAATATGCTAAATGTTGTTATAGTATCCCACG AGCCCCAAAATTAAATAGA |
| adDM D-E23-I23 | GTGGAATAGTATAACAATATGCTAAATGTTGTTATAGTATCCCACT TACCCGAAATTTCGAAGT |

MS2-adRNA sequences for exon 23 skipping in the dystrophin transcript in *mdx* mice (SEQ ID NO:160-161)

| | |
|---|---|
| MS2-DMD-I22-E23 | aACATGAGGATCACCCATGTcGAGCCCCAAAATTAAATAGAaACAT GAGGATCACCCATGTc |
| MS2-DMD-E23-I23 | aACATGAGGATCACCCATGTcTTACCCGAAATTTTCGAAGTaACATG AGGATCACCCATGTc |

MS2-adRNA sequence for editing the premature stop codon in *mdx* mice (SEQ ID 162)

| | |
|---|---|
| MS2_D MD | aACATGAGGATCACCCATGTcCCATTCCATTGCTCTTTCAAaACATG AGGATCACCCATGTc |

adRNA sequences for exon skipping in the SCN9A transcript in mice (SEQ ID NO:163-167)

| | |
|---|---|
| adRNA_SCN9A_ I8_E9_F | GTGGAATAGTATAACAATATGCTAAATGTTGTTATAGTATC CCACACTGCCCACAGATGAACAAG |
| adRNA_SCN9A_ I4_E5_F | GTGGAATAGTATAACAATATGCTAAATGTTGTTATAGTATC CCACTGTACCCGAAGGAGAGAATA |
| adRNA_SCN9A_ I2_E3_F | GTGGAATAGTATAACAATATGCTAAATGTTGTTATAGTATC CCACAAAGTCCGAGGAGGAAAAAG |
| adRNA_SCN9A_ I5_E6_F | GTGGAATAGTATAACAATATGCTAAATGTTGTTATAGTATC CCACAATACCCGTAGGATTAAATC |
| adRNA_SCN9A_ I13_E14_F | GTGGAATAGTATAACAATATGCTAAATGTTGTTATAGTATC CCACAAGTTCCGGAAAACAAACAA |

MS2-adRNA sequences for exon skipping in the SCN9A transcript in mice (SEQ ID NO: 168-172)

| | |
|---|---|
| MS2_adRNA_SCN9 A_I8_E9_F | aACATGAGGATCACCCATGTcACTGCCCACAGATGAACAA GaACATGAGGATCACCCATGTc |
| MS2_adRNA_SCN9 A_I4_E5_F | aACATGAGGATCACCCATGTcTGTACCCGAAGGAGAGAAT AaACATGAGGATCACCCATGTc |
| MS2_adRNA_SCN9 A_I2_E3_F | aACATGAGGATCACCCATGTcAAAGTCCGAGGAGGAAAA AGaACATGAGGATCACCCATGTc |
| MS2_adRNA_SCN9 A_I5_E6_F | aACATGAGGATCACCCATGTcAATACCCGTAGGATTAAAT CaACATGAGGATCACCCATGTc |
| MS2_adRNA_SCN9 A_I13_E14_F | aACATGAGGATCACCCATGTcAAGTTCCGGAAAACAAACA AaACATGAGGATCACCCATGTc |

### Example 7 - C→ T editing via APOBECs

The local structure of the apolipoprotein B mRNA that the ACF-APOBEC complex binds to, at the mRNA target of interest, can be created for carefully positioning the C to be edited at a position analogous to the naturally occurring site in the apolipoprotein B mRNA. This is achieved by overexpression of a pair of gRNAs to create a apolipoprotein B mRNA like structure that can be edited by overexpression of one or more of the following combinations:
1. MCP-ACF1 and APOBEC1
2. MCP-ACF1 and MCP-APOBEC1
3. MCP-linker-ACF-linker-APOBEC1
4. MCP-APOBEC1 and ACF1

### SEQUENCE SET 2: Exemplary C^T Editing Sequences

MS2-adRNA sequences for C-T editing (SEQ ID NO: 173-174)

| | |
|---|---|
| MS2-gRN A-1 | acatatatgataNNNNNNNNNNaACATGAGGATCACCCATGTcNNNNNNNNNNNNNNNN NNNNNNNNNNN |
| MS2-gRN A-2 | NNNNNNNNNNNNNNNNNNNNNaACATGAGGATCACCCATGTcNNNNNN NNNNttgatcagtatatta |

### Example 8 - Creation of point mutations relevant for cancers

Several genes involved in cancer pathways harbor single amino acid substitution. Creation of dominant negative mutants, constitutionally active mutants and catalytically inactive mutants is possible by creating A->G substitutions in the mRNA sequences of these genes. Some of these genes include KRAS, HRAS, JAK2, GSK3β, β-catenin, SmoM2, Caspase3, Caspase 8, TGF-β, p53.

### Example 9 - Editing DNA and both strands of DNA/RNA hybrids

Since ADARs have been shown to edit double stranded RNA as well as both strands of a DNA-RNA hybrid, it is possible to recruit ADARs via single stranded DNA or DNA-RNA hybrids to edit both DNA and RNA. This can be used to modify the current adenine base editing approach to Cas9 (or Cpf1)-ADAR-deaminase domain fusions (ADAR1, ADAR2 and their catalytically active mutants (E1008Q) and E488Q), and instead of targeting the ssDNA displaced strand by current base editors, the gRNA bound strand with a A-C bulge can be targeted, ideally in the first 10bp close to the 5' end of the gRNA.

### Example 10 - Ornithine transcarbamylase deficiency

ADAR2 (E488Q) along with an adRNA was delivered in spf^{ash} mice. In addition to the correctly spliced mRNA, spf^{ash} mice harbor an incorrectly spliced, elongated mRNA variant which is formed due to the use of a cryptic splice site, 48 base pairs into exon 4. Upon delivery of the ADAR2 (E488Q) with an adRNA, a reduction in the incorrectly spliced product was confirmed. Highly efficient RNA editing yields of up to 33.9% in the spliced mRNA was also observed. In the pre-mRNA yields of up to 8% were observed. Protein restoration of 2.5-5%, within 3 weeks of injections was also observed. This demonstrates the utility of RNA editing in the correction of splice site mutations and altering splicing patterns. (**FIG. 50A-D)**

### Example 11 - Duchenne muscular dystrophy

MCP-ADAR1(E1008Q) was tested with MS2-adRNA in *mdx* mice. Enhanced RNA editing yields and restoration of dystrophin expression over the ADAR2 and ADAR2 (E488Q) enzymes were observed. **(****FIG. 51A-B)**

### Equivalents

Unless otherwise defined, all technical and scientific terms used herein have the same meaning as commonly understood by one of ordinary skill in the art to which this technology belongs.

The technology illustratively described herein can suitably be practiced in the absence of any element or elements, limitation or limitations, not specifically disclosed herein. Thus, for example, the terms "comprising," "including," "containing," *etc.* shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the technology claimed.

Thus, it should be understood that the materials, methods, and examples provided here are representative of preferred aspects, are exemplary, and are not intended as limitations on the scope of the technology.

The technology has been described broadly and generically herein. Each of the narrower species and sub-generic groupings falling within the generic disclosure also form part of the technology. This includes the generic description of the technology with a proviso or negative limitation removing any subject matter from the genus, regardless of whether or not the excised material is specifically recited herein.

In addition, where features or aspects of the technology are described in terms of Markush groups, those skilled in the art will recognize that the technology is also thereby described in terms of any individual member or subgroup of members of the Markush group.

All publications, patent applications, patents, and other references mentioned herein are expressly incorporated by reference in their entirety, to the same extent as if each were incorporated by reference individually. In case of conflict, the specification, including definitions, will control.

### Specific Embodiments

A number of compositions, methods and systems are disclosed herein. Specific exemplary embodiments of these compositions, methods and systems are disclosed below.

### PART 1

*Embodiment 1.* An engineered ADAR1 or ADAR2 guide RNA ("adRNA") comprising: a sequence complementary to a target RNA.

*Embodiment 2.* The engineered adRNA of embodiment 1, further comprising an ADAR2 recruiting domain derived from GluR2 mRNA.

*Embodiment 3.* The engineered adRNA of embodiment 1, further comprising two MS2 hairpins flanking the sequence complementary to a target RNA.

*Embodiment 4.* The engineered adRNA of any one of embodiments 1-3, wherein the sequence complementary to the target RNA comprises between about 20 to 100 base pairs.

*Embodiment 5.* The engineered adRNA of embodiment 2 or 4, wherein the ADAR2 recruiting domain derived from GluR2 mRNA is located at the 5' end or the 3' end of the engineered adRNA.

*Embodiment 6*. The engineered adRNA of embodiment 5, comprising a GluR2 mRNA at both the 5'end and the 3' end of the engineered adRNA.

*Embodiment 7*. The engineered adRNA of embodiment 5 or 6, further comprising an editing inducer element.

*Embodiment 8.* The engineered adRNA of any of the preceding embodiments, wherein the target RNA is ornithine transcarbamylase.

*Embodiment 9.* An engineered ADAR2 guide RNA ("adRNA") encoded by a polynucleotide sequence selected from the group of sequences provided in **TABLE 1** or **FIG.** 2, or an equivalent of each thereof.

*Embodiment 10.* An isolated polynucleotide encoding the engineered adRNA of any one of embodiments 1-9, or an equivalent of each thereof.

*Embodiment 11.* A vector comprising one or more of the isolated polynucleotide of embodiment 10 or the polynucleotide sequence encoding the engineered adRNA of embodiment 9 and optionally regulatory sequences operatively linked to the isolated polynucleotide.

*Embodiment 12.* The vector of embodiment 11, wherein the regulatory sequences comprise a promoter, an enhancer element and/or a reporter.

*Embodiment 13.* The vector of embodiment 12, wherein the promoter is a human U6, a mouse U6 promoter or a CMV promoter.

*Embodiment 14.* The vector of any one of embodiments 11-13, further comprising a detectable marker or a purification marker.

*Embodiment 15.* The vector of embodiment 14, wherein the vector is a plasmid or a viral vector.

*Embodiment 16.* The vector of embodiment 15, wherein the vector is selected from a group consisting of a retroviral vector, a lentiviral vector, an adenoviral vector, and an adeno-associated viral vector.

*Embodiment 17.* A recombinant cell further comprising the vector of any one of embodiments 11-16, wherein the engineered adRNA is recombinantly expressed.

*Embodiment 18.* A composition comprising a carrier and one or more of the engineered adRNA of any one of embodiments 1-9, the isolated polynucleotide of embodiment 10, the vector of any one of embodiments 11-16 or the recombinant cell of embodiment 17.

*Embodiment 19.* The composition of embodiment 18, further comprising a chemotherapeutic agent or drug.

*Embodiment 20.* The composition of embodiment 18 or 19, wherein the carrier is a pharmaceutically acceptable carrier or a solid support.

*Embodiment 21.* A method of modifying protein expression comprising contacting a polynucleotide encoding the protein with the engineered adRNA of any one of embodiments 1-9.

*Embodiment 22.* The method of embodiment 21, wherein the contacting is *in vitro* or *in vivo.*

*Embodiment 23.* A method of treating a disease or disorder associated with aberrant protein expression comprising administering to a subject in need of such treatment an effective amount of one or more of the engineered adRNA of any one of embodiments 1-9.

*Embodiment 24.* The method of embodiment 23, wherein the disease or disorder is Duchenne Muscular Dystrophy.

*Embodiment 25.* The method of embodiment 23 or 24, wherein the subject is an animal.

*Embodiment 26.* The method of embodiment 23 or 24, wherein the animal is a mammal.

*Embodiment 27.* Use of an effective amount of one or more of the engineered adRNA of any one of embodiments 1-9 for treating a disease or disorder associated with aberrant protein expression.

*Embodiment 28.* The use of embodiment 27, wherein the disease or disorder is Duchenne Muscular Dystrophy.

*Embodiment 29.* A kit comprising the engineered adRNA of any one of embodiments 1-9, the isolated polynucleotide of embodiment 10, the vector of any one of embodiments 11-16, the recombinant cell of embodiment 17, or the composition of any one of embodiments 18-20 and instructions for use.

*Embodiment 30.* The kit of embodiment 19, wherein the instructions recite the method of any one of embodiments 21-26.

*Embodiment 31.* A complex comprising an adRNA of any one of embodiments 1-9, hybridized to a complementary polynucleotide under conditions of high stringency.

### PART 2

*Embodiment 1.* An engineered ADAR2 guide RNA ("adRNA") comprising: a sequence complementary to a target RNA, and an ADAR2 recruiting domain derived from GluR2 mRNA.

*Embodiment 2.* The engineered adRNA of embodiment 1, wherein the sequence complementary to the target RNA comprises between about 20 to 100 base pairs.

*Embodiment 3.* The engineered adRNA of embodiment 1 or embodiment 2, wherein the ADAR2 recruiting domain derived from GluR2 mRNA is located at the 5' end or the 3' end of the engineered adRNA.

*Embodiment 4.* The engineered adRNA of embodiment 3, comprising a GluR2 mRNA at both the 5' end and the 3' end of the engineered adRNA.

*Embodiment 5.* The engineered adRNA of any of the preceding embodiments, wherein the target RNA is ornithine transcarbamylase.

*Embodiment 6.* An engineered ADAR2 guide RNA ("adRNA") encoded by a sequence selected from the group of sequences provided in **TABLE 1** or **FIG. 2****.**

*Embodiment 7.* A method of modifying protein expression comprising contacting a polynucleotide encoding the protein with the engineered adRNA of any one of embodiments 1-6.

*Embodiment 8.* The method of embodiment 7, wherein the contacting is *in vitro* or *in vivo.*

*Embodiment 9.* A method of treating a disease or disorder associated with aberrant protein expression comprising administering to a subject in need of such treatment an effective amount of one or more of the engineered adRNA of any one of embodiments 1 to 6.

*Embodiment 10.* The method of embodiment 9, wherein the subject is an animal.

*Embodiment 11.* The method of embodiment 9, wherein the animal is a mammal.

*Embodiment 12.* Use of an effective amount of one or more of the engineered adRNA of any one of embodiments 1 to 6 for treating a disease or disorder associated with aberrant protein expression.

*Embodiment 13.* A kit comprising the engineered adRNA of any one of embodiments 1 to 6 and instructions for use.

*Embodiment 14.* The kit of embodiment 13, wherein the instructions recite the method of embodiment 7 or embodiment 9.

*Embodiment 15.* A composition comprising the AdRNA of any one of embodiments 1-6, and a carrier.

*Embodiment 16.* The composition of embodiment 15, wherein the carrier is a pharmaceutically acceptable carrier.

*Embodiment 17.* A complex comprising an AdRNA of any one of embodiments 1-6, hybridized to a complementary polynucleotide under conditions of high stringency.

### PART 3

*Embodiment 1.* An ADAR system for exon skipping comprising an adRNA targeting a splice acceptor and/or a branch point in an intron and, optionally, an ADAR enzyme.

*Embodiment 2.* The ADAR system of claim 1, wherein the ADAR enzyme is ADAR1, ADAR2, or a mutant or variant each thereof.

*Embodiment 3.* The ADAR system of claim 2, wherein the mutant or variant is selected from ADAR1 (E1008Q) and ADAR2 (E488Q).

*Embodiment 4.* The ADAR system of any one of claims 1 to 3, wherein the intron is comprised in a gene selected from dystrophin, SCN9A, or ornithine transcarbamylase.

*Embodiment 5.* The ADAR system of any one of claims 1 to 4, wherein the adRNA is selected from **SEQUENCE SET 1.**

*Embodiment 6.* A method of treating a disease, disorder, or condition characterized by aberrant gene expression comprising administering the ADAR system of any one of claims 1 to 5.

*Embodiment 7.* The method of claim 6, wherein the disease, disorder, or condition is selected from Duchenne muscular dystrophy or ornithine transcarbamylase deficiency.

*Embodiment 8.* The method of claim 6 or claim 7, wherein the disease, disorder, or condition is associated with pain.

*Embodiment 9.* An APOBEC system for cytosine to thymine editing comprising a pair of gRNA that creates an alipoprotein B mRNA like structure and, optionally, an APOBEC enzyme.

*Embodiment 10.* The APOBEC system of claim 9, wherein the pair of gRNA is the pair of sequences provided in **SEQUENCE SET 2.**

### REFERENCES

Montiel-Gonzalez, M. F., Vallecillo-Viejo, I., Yudowski, G. A. & Rosenthal, J. J. C. Correction of mutations within the cystic fibrosis transmembrane conductance regulator by site-directed RNA editing. Proc. Natl. Acad. Sci. U. S. A. 110, 18285-90 (2013).
Wettengel, J., Reautschnig, P., Geisler, S., Kahle, P. J. & Stafforst, T. Harnessing human ADAR2 for RNA repair - Recoding a PINK1 mutation rescues mitophagy. Nucleic Acids Res. 45, 2797-2808 (2017).
Fukuda, M. et al. Construction of a guide-RNA for site-directed RNA mutagenesis utilising intracellular A-to-I RNA editing. Sci. Rep. 7, 41478 (2017).
Heep, M., Mach, P., Reautschnig, P., Wettengel, J. & Stafforst, T. Applying Human ADAR1p110 and ADAR1p150 for Site-Directed RNA Editing-G/C Substitution Stabilizes GuideRNAs against Editing. Genes (Basel). 8, 34 (2017).
Vallecillo-Viejo, I. C., Liscovitch-Brauer, N., Montiel-Gonzalez, M. F., Eisenberg, E. & Rosenthal, J. J. C. Abundant off-target edits from site-directed RNA editing can be reduced by nuclear localization of the editing enzyme. RNA Biol. 15, 104-114 (2018).
Sinnamon, J. R. et al. Site-directed RNA repair of endogenous Mecp2 RNA in neurons. Proc. Natl. Acad. Sci. U. S. A. 114, E9395-E9402 (2017).
Cox, D. B. T. et al. RNA editing with CRISPR-Cas13. Science eaaq0180 (2017). doi:10.1126/science.aaq0180
Montiel-González, M. F., Vallecillo-Viejo, I. C. & Rosenthal, J. J. C. An efficient system for selectively altering genetic information within mRNAs. Nucleic Acids Res. 44, gkw738 (2016).
Hanswillemenke, A., Kuzdere, T., Vogel, P., Jeely, G. & Stafforst, T. Site-Directed RNA Editing in vivo Can Be Triggered by the Light- Driven Assembly of an Artificial Riboprotein.doi:10.1021/jacs.5b10216
Woolf, T. M., Chase, J. M. & Stinchcomb, D. T. Toward the therapeutic editing of mutated RNA sequences. Biochemistry 92, 8298-8302 (1995).
Stafforst, T. & Schneider, M. F. An RNA-Deaminase Conjugate Selectively Repairs Point Mutations. Angew. Chemie Int. Ed. 51, 11166-11169 (2012).
Schneider, M. F., Wettengel, J., Hoffmann, P. C. & Stafforst, T. Optimal guideRNAs for re-directing deaminase activity of hADAR1 and hADAR2 in trans. Nucleic Acids Res. 42, e87 (2014).
Azad, M. T. A., Bhakta, S. & Tsukahara, T. Site-directed RNA editing by adenosine deaminase acting on RNA for correction of the genetic code in gene therapy. Gene Ther. 24, 779-786 (2017).
Vogel, P. et al. Efficient and precise editing of endogenous transcripts with SNAP-tagged ADARs. Nat. Methods 15, 535-538 (2018).
Smalley, E. First AAV gene therapy poised for landmark approval. Nat. Biotechnol. 2017 3511 (2017).
Calcedo, R. & Wilson, J. M. AAV Natural Infection Induces Broad Cross-Neutralizing Antibody Responses to Multiple AAV Serotypes in Chimpanzees. Hum. Gene Ther. Clin. Dev. 27, 79-82 (2016).
Harbison, C. E. et al. Examining the cross-reactivity and neutralization mechanisms of a panel of mabs against adeno-associated virus serotypes 1 and 5. J. Gen. Virol. 93, (2012).
Riviere, C., Danos, O. & Douar, A. M. Long-term expression and repeated administration of AAV type 1, 2 and 5 vectors in skeletal muscle of immunocompetent adult mice. Gene Ther. 13, 1300-1308 (2006).
Mays, L. E. & Wilson, J. M. The Complex and Evolving Story of T cell Activation to AAV Vector-encoded Transgene Products. Mol. Ther. 19, 16-27 (2011).
Chen, J., Wu, Q., Yang, P., Hsu, H. C. & Mountz, J. D. Determination of specific CD4 and CD8 T cell epitopes after AAV2- and AAV8-hF.IX gene therapy. Mol Ther 13, 260-269 (2006).
Ertl, H. C. J. & High, K. A. Impact of AAV Capsid-Specific T-Cell Responses on Design and Outcome of Clinical Gene Transfer Trials with Recombinant Adeno-Associated Viral Vectors: An Evolving Controversy. Hum. Gene Ther. 28, 328-337 (2017).
Hinderer, C. et al. Severe toxicity in nonhuman primates and piglets following high-dose intravenous administration of an AAV vector expressing human SMN. Hum. Gene Ther. hum.2018.015 (2018). doi:10.1089/hum.2018.015
Mingozzi, F. et al. Prevalence and pharmacological modulation of humoral immunity to AAV vectors in gene transfer to synovial tissue. Gene Ther 20, 417-424 (2013).
Basner-Tschakarjan, E., Bijjiga, E. & Martino, A. T. Pre-clinical assessment of immune responses to adeno-associated virus (AAV) vectors. Frontiers in Immunology 5, (2014).
Harding, F. A., Stickler, M. M., Razo, J. & DuBridge, R. B. The immunogenicity of humanized and fully human antibodies: Residual immunogenicity resides in the CDR regions. MAbs 2, 256-265 (2010).
De Groot, a S., Knopp, P. M. & Martin, W. De-immunization of therapeutic proteins by T-cell epitope modification. Dev. Biol. (Basel). 122, 171-194 (2005).
Ferdosi, S. R. et al. Multifunctional CRISPR/Cas9 with engineered immunosilenced human T cell epitopes. bioRxiv (2018).
Veronese, F. M. & Mero, A. The impact of PEGylation on biological therapies. BioDrugs 22, 315-329 (2008).
Armstrong, J. K. et al. Antibody against poly(ethylene glycol) adversely affects PEG-asparaginase therapy in acute lymphoblastic leukemia patients. Cancer 110, 103-111 (2007).
Kurosaki, T., Kometani, K. & Ise, W. Memory B cells. Nat. Rev. Immunol. 15, 149-159 (2015).
Zabel, F. et al. Distinct T helper cell dependence of memory B-cell proliferation versus plasma cell differentiation. Immunology 150, 329-342 (2017).
Chylinski, K., Makarova, K. S., Charpentier, E. & Koonin, E. V. Classification and evolution of type II CRISPR-Cas systems. Nucleic Acids Research 42, 6091-6105 (2014).
Burstein, D. et al. New CRISPR-Cas systems from uncultivated microbes. Nat. Publ. Gr. 542, (2017).
Makarova, K. S., Zhang, F. & Koonin, E. V. SnapShot: Class 2 CRISPR-Cas Systems. Cell 168, 328-328.e1 (2017).
Ellis, B. L. et al. A survey of ex vivo/in vitro transduction efficiency of mammalian primary cells and cell lines with Nine natural adeno-associated virus (AAV1-9) and one engineered adeno-associated virus serotype. Virol. J. 10, 74 (2013).
Andreatta, M. & Nielsen, M. Gapped sequence alignment using artificial neural networks: application to the MHC class I system. Bioinformatics 32, 511-517 (2015).
Andreatta, M. et al. Accurate pan-specific prediction of peptide-MHC class II binding affinity with improved binding core identification. Immunogenetics 67, 641-650 (2015).
Truong, D.-J. J. et al. Development of an intein-mediated split-Cas9 system for gene therapy. Nucleic Acids Res. 43, 6450-6458 (2015).
Moreno, A. M. et al. In Situ Gene Therapy via AAV-CRISPR-Cas9-Mediated Targeted Gene Regulation. Mol. Ther. 0, (2018).
Benveniste, O. et al. Prevalence of Serum IgG and Neutralizing Factors Against Adeno-Associated Virus (AAV) Types 1,2,5,6,8, and 9 in the Healthy Population: Implications for Gene Therapy Using AAV Vectors. Hum. Gene Ther. 21, 704-712 (2010).
Hsu, P. D., Lander, E. S. & Zhang, F. Development and applications of CRISPR-Cas9 for genome engineering. Cell 157, 1262-1278 (2014).
Ran, F. A. et al. In vivo genome editing using Staphylococcus aureus Cas9. Nature 520, 186-190 (2015).
Clemente, T., Dominguez, M. R., Vieira, N. J., Rodrigues, M. M. & Amarante-Mendes, G. P. In vivo assessment of specific cytotoxic T lymphocyte killing. Methods 61, 105-109 (2013).
Weinmann, J. & Grimm, D. Next-generation AAV vectors for clinical use: an ever-accelerating race. Virus Genes 53, 707-713 (2017).
Gabriel, N. et al. Bioengineering of AAV2 Capsid at Specific Serine, Threonine, or Lysine Residues Improves Its Transduction Efficiency in vitro and in vivo. Hum. Gene Ther. Methods 24, 80-93 (2013).
Zhong, L. et al. Next generation of adeno-associated virus 2 vectors: Point mutations in tyrosines lead to high-efficiency transduction at lower doses. Proc. Natl. Acad. Sci. 105, 7827-7832 (2008).
Shen, S. et al. Engraftment of a Galactose Receptor Footprint onto Adeno-associated Viral Capsids Improves Transduction Efficiency. J. Biol. Chem. 288, 28814-28823 (2013).
Zinn, E. et al. In Silico Reconstruction of the Viral Evolutionary Lineage Yields a Potent Gene Therapy Vector. Cell Rep. 12, 1056-1068 (2017).
Deverman, B. E. et al. Cre-dependent selection yields AAV variants for widespread gene transfer to the adult brain. Nat. Biotechnol. 34, 204-209 (2016).
Smith, J. K. & Agbandje-McKenna, M. Creating an arsenal of Adeno-associated virus (AAV) gene delivery stealth vehicles. PLOS Pathog. 14, e1006929 (2018).
Bartel, M., Schaffer, D. & Büning, H. Enhancing the clinical potential of AAV vectors by capsid engineering to evade pre-existing immunity. Front. Microbiol. 2, 204 (2011).
Hui, D. J. et al. AAV capsid CD8+ T-cell epitopes are highly conserved across AAV serotypes. Mol. Ther. - Methods Clin. Dev. 2, 15029 (2015).
Gao, G., Vandenberghe, L. & Wilson, J. New Recombinant Serotypes of AAV Vectors. Curr. Gene Ther. 5, 285-297 (2005).
Grieger, J. C., Choi, V. W. & Samulski, R. J. Production and characterization of adeno-associated viral vectors. Nat. Protoc. 1, 1412-1428 (2006).
Mingozzi, F. & Büning, H. Adeno-Associated Viral Vectors at the Frontier between Tolerance and Immunity. Front. Immunol. 6, 120 (2015).
Mingozzi, F. & High, K. A. Therapeutic in vivo gene transfer for genetic disease using AAV: progress and challenges. Nat. Rev. Genet. 12, 341-355 (2011).
Wagner, D. L. et al. High prevalence of S. pyogenes Cas9-specific T cell sensitization within the adult human population - A balanced effector/regulatory T cell response. bioRxiv 295139 (2018). doi:10.1101/295139
Gernoux, G., Wilson, J. M. & Mueller, C. Regulatory and Exhausted T Cell Responses to AAV Capsid. Hum. Gene Ther. 28, 338-349 (2017).
Zhu, J., Huang, X. & Yang, Y. The TLR9-MyD88 pathway is critical for adaptive immune responses to adeno-associated virus gene therapy vectors in mice. J. Clin. Invest. 119, 2388-2398 (2009).
Rogers, G. L. & Herzog, R. W. TLR9 and Dendritic Cells Are Required for CD8+ T Cell Responses to the AAV Capsid. Blood 124, (2014).
Faust, S. M. et al. CpG-depleted adeno-associated virus vectors evade immune detection. J. Clin. Invest. 123, 2994-3001 (2013).
Hirsch, M. & Wu, J. 319. Rationally Designed AAV Inverted Terminal Repeats Enhance Gene Targeting. Mol. Ther. 24, S129 (2016).
Léger, A. et al. Adeno-Associated Viral Vector-Mediated Transgene Expression Is Independent of DNA Methylation in Primate Liver and Skeletal Muscle. PLoS One 6, e20881 (2011).
Deaton, A. M. & Bird, A. CpG islands and the regulation of transcription. Genes Dev. 25, 1010-22 (2011).
Shao, W. et al. Double-stranded RNA innate immune response activation from long-term adeno-associated virus vector transduction. JCI Insight 3, (2018).
Peisley, A. et al. Cooperative assembly and dynamic disassembly ofMDA5 filaments for viral dsRNA recognition. Proc. Natl. Acad. Sci. U. S. A. 108, 21010-5 (2011).
Borel, F., Kay, M. A. & Mueller, C. Recombinant AAV as a platform for translating the therapeutic potential of RNA interference. Mol. Ther. 22, 692-701 (2014).
Xie, J., Burt, D. R. & Gao, G. Adeno-associated virus-mediated microRNA delivery and therapeutics. Semin. Liver Dis. 35, 81-8 (2015).
Li, J., Kim, S. G. & Blenis, J. Rapamycin: one drug, many effects. Cell Metab. 19, 373-9 (2014).
Meliani, A. et al. Antigen-selective modulation of AAV immunogenicity with tolerogenic rapamycin nanoparticles enables successful vector re-administration. Nat. Commun. 9, 4098 (2018).
Battaglia, M. et al. Rapamycin promotes expansion of functional CD4+CD25+FOXP3+ regulatory T cells of both healthy subjects and type 1 diabetic patients. J. Immunol. 177, 8338-47 (2006).
Lee, E. J., Guenther, C. M. & Suh, J. Adeno-associated virus (AAV) vectors: Rational design strategies for capsid engineering. Curr. Opin. Biomed. Eng. 7, 58-63 (2018).
Xie, Q. et al. The atomic structure of adeno-associated virus (AAV-2), a vector for human gene therapy. Proc. Natl. Acad. Sci. U. S. A. 99, 10405-10 (2002).
Walters, R. W. et al. Structure of Adeno-Associated Virus Serotype 5. J. Virol. 78, 3361-3371 (2004).
Kotterman, M. A., Chalberg, T. W. & Schaffer, D. V. Viral Vectors for Gene Therapy: Translational and Clinical Outlook. Annu. Rev. Biomed. Eng. 17, 63-89 (2015).
Grimm, D. et al. In vitro and In vivo Gene Therapy Vector Evolution via Multispecies Interbreeding and Retargeting of Adeno-Associated Viruses. J. Virol. 82, 5887-5911 (2008).
Chew, W. L. et al. A multifunctional AAV-CRISPR-Cas9 and its host response. Nat. Methods 13, 868-874 (2016).
Katrekar, D. et al. In vivo RNA editing of point mutations via RNA-guided adenosine deaminases. Nat. Methods (2019). doi:10.1038/s41592-019-0323-0
Kim, D. D. Y. et al. Widespread RNA editing of embedded alu elements in the human transcriptome. Genome Res. 14, 1719-25 (2004).
Herbert, A. Z-DNA and Z-RNA in human disease. Commun. Biol. 2, 7 (2019).
Koeris, M., Funke, L., Shrestha, J., Rich, A. & Maas, S. Modulation of ADAR1 editing activity by Z-RNA in vitro. Nucleic Acids Res. 33, 5362-70 (2005).
Yang, J.-H. et al. Intracellular localization of differentially regulated RNA-specific adenosine deaminase isoforms in inflammation. J. Biol. Chem. 278, 45833-42 (2003).
Abudayyeh, O. O. et al. A cytosine deaminase for programmable single-base RNA editing. Science 365, 382-386 (2019).
Point Mutation - Definition, Types and Examples | Biology Dictionary.
Lueck, J. D. et al. Engineered transfer RNAs for suppression of premature termination codons. Nat. Commun. 10, 822 (2019).
Jonsson, T. et al. A mutation in APP protects against Alzheimer's disease and age-related cognitive decline. Nature 488, 96-99 (2012).
Sun, J. & Roy, S. The physical approximation of APP and BACE-1: A key event in alzheimer's disease pathogenesis. Dev. Neurobiol. 78, 340-347 (2018).
Sun, J. et al. CRISPR/Cas9 editing of APP C-terminus attenuates β-cleavage and promotes α-cleavage. Nat. Commun. 10, 53 (2019).
Melcher, T. et al. A mammalian RNA editing enzyme. Nature 379, 460-464 (1996).
Montiel-Gonzalez, M. F., Vallecillo-Viejo, I., Yudowski, G. A. & Rosenthal, J. J. C. Correction of mutations within the cystic fibrosis transmembrane conductance regulator by site-directed RNA editing. Proc. Natl. Acad. Sci. U. S. A. 110, 18285-90 (2013).
Wettengel, J., Reautschnig, P., Geisler, S., Kahle, P. J. & Stafforst, T. Harnessing human ADAR2 for RNA repair - Recoding a PINK1 mutation rescues mitophagy. Nucleic Acids Res. 45, 2797-2808 (2017).
Fukuda, M. et al. Construction of a guide-RNA for site-directed RNA mutagenesis utilising intracellular A-to-I RNA editing. Sci. Rep. 7, 41478 (2017).
Vallecillo-Viejo, I. C., Liscovitch-Brauer, N., Montiel-Gonzalez, M. F., Eisenberg, E. & Rosenthal, J. J. C. Abundant off-target edits from site-directed RNA editing can be reduced by nuclear localization of the editing enzyme. RNA Biol. 15, 104-114 (2018).
Sinnamon, J. R. et al. Site-directed RNA repair of endogenous Mecp2 RNA in neurons. Proc. Natl. Acad. Sci. U. S. A. 114, E9395-E9402 (2017).
Cox, D. B. T. et al. RNA editing with CRISPR-Cas13. Science 358, 1019-1027 (2017).
Azad, M. T. A., Bhakta, S. & Tsukahara, T. Site-directed RNA editing by adenosine deaminase acting on RNA for correction of the genetic code in gene therapy. Gene Ther. 24, 779-786 (2017).
Vogel, P. et al. Efficient and precise editing of endogenous transcripts with SNAP-tagged ADARs. Nat. Methods 15, 535-538 (2018).
Daniel, C., Widmark, A., Rigardt, D. & Öhman, M. Editing inducer elements increases A-to-I editing efficiency in the mammalian transcriptome. Genome Biol. 18, 195 (2017).
Woolf, T.M., Chase J.M., & Stinchcomn D.T. Toward the therapeutic editing of mutated RNA sequences. Proc. Natl. Acad. Sci. USA 92, 8298-8302 (1995).
Chung, H. et al. Human ADAR1 Prevents Endogenous RNA from Triggering Translational Shutdown. Cell 172, 811-824.e14 (2018).
Desterro, J. M. P. et al. Dynamic association of RNA-editing enzymes with the nucleolus. J. Cell Sci. 116, 1805-1818 (2003).
Mort, M., Ivanov, D., Cooper, D. N. & Chuzhanova, N. A. A meta-analysis of nonsense mutations causing human genetic disease. Hum. Mutat. 29, 1037-1047 (2008).
Nelson, C. E. et al. In vivo genome editing improves muscle function in a mouse model of Duchenne muscular dystrophy. Science 351, 403-7 (2016).
Tabebordbar, M. et al. In vivo gene editing in dystrophic mouse muscle and muscle stem cells. Science (80-. ). 351, 407-411 (2016).
Long, C. et al. Postnatal genome editing partially restores dystrophin expression in a mouse model of muscular dystrophy. Science (80-. ). 351, 400-403 (2016).
Hodges, P. E. & Rosenberg, L. E. The spfash mouse: a missense mutation in the ornithine transcarbamylase gene also causes aberrant mRNA splicing. Proc. Natl. Acad. Sci. U. S. A. 86, 4142-4146 (1989).
Yang, Y. et al. A dual AAV system enables the Cas9-mediated correction of a metabolic liver disease in newborn mice. Nat. Biotechnol. 34, 334-338 (2016).
Chen, L. et al. Recoding RNA editing of AZIN1 predisposes to hepatocellular carcinoma. Nat. Med. 19, 209-216 (2013).
Mann CJ, Honeyman K, Cheng AJ, et al. Antisense-induced exon skipping and synthesis of dystrophin in the mdx mouse. Proceedings of the National Academy of Sciences of the United States of America. 2001;98(1):42-47.
S. Konermann, P. Lotfy, N. J. Brideau, J. Oki, M. N. Shokhirev, P. D. Hsu, Transcriptome Engineering with RNA-Targeting Type VI-D CRISPR Effectors. Cell 173, 665-676.el4 (2018).

### EMBODIMENTS

Embodiment 1. A vector that comprises a nucleic acid with a polynucleotide sequence encoding at least one RNA editing entity recruiting domain, wherein:
(a) the polynucleotide sequence encoding the at least one RNA editing entity recruiting domain lacks a secondary structure comprising a stem-loop, or
(b) the polynucleotide sequence encoding the at least one RNA editing entity recruiting domain comprises at least about 80% sequence identity to at least one sequence selected from: an Alu domain encoding sequence, an Apolipoprotein B mRNA Editing Catalytic Polypeptide-like (APOBEC) recruiting domain encoding sequence, and combination thereof.

Embodiment 2. The vector of Embodiment 0, wherein the polynucleotide sequence encoding the at least one RNA editing entity recruiting domain comprises at least about 80% sequence identity to the Alu domain sequence.

Embodiment 3. The vector of Embodiment 0, wherein the polynucleotide sequence encoding the at least one RNA editing entity recruiting domain comprises at least about 80% sequence identity to the APOBEC recruiting domain encoding sequence.

Embodiment 4. The vector of Embodiment 0, wherein the vector is a viral vector.

Embodiment 5. The vector of Embodiment 0, wherein the vector is a liposome.

Embodiment 6. The vector of Embodiment 0, wherein the vector is a nanoparticle.

Embodiment 7. The vector of Embodiment 0, wherein the at least one RNA editing entity recruiting domain is configured to recruit an ADAR protein.

Embodiment 8. The vector of Embodiment 0, wherein the ADAR protein is an ADAR1, ADAR2, or ADAR3 protein.

Embodiment 9. The vector of Embodiment 0 or 0, wherein the ADAR protein is a human ADAR protein.

Embodiment 10. The vector of Embodiment 0 or 0, wherein the ADAR protein is a recombinant ADAR protein.

Embodiment 11. The vector of Embodiment 0 or 0, wherein the ADAR protein is a modified ADAR protein.

Embodiment 12. The vector of Embodiment 0, wherein the at least one RNA editing entity recruiting domain is configured to recruit an APOBEC protein.

Embodiment 13. The vector of Embodiment 0, wherein the APOBEC protein is an APOBEC1, APOBEC2, APOBEC3A, APOBEC3B, APOBEC3C, APOBEC3E, APOBEC3F, APOBEC3G, APOBEC3H, or APOBEC4 protein.

Embodiment 14. The vector of Embodiment 0 or 0, wherein the ADAR protein is a human ADAR protein.

Embodiment 15. The vector of Embodiment 0 or 0, wherein the ADAR protein is a recombinant ADAR protein.

Embodiment 16. The vector of Embodiment 0 or 0, wherein the ADAR protein is a modified ADAR protein.

Embodiment 17. The vector of Embodiment 0, wherein the at least one RNA editing entity recruiting domain lacks a secondary structure comprising a stem-loop.

Embodiment 18. The vector of Embodiment 0, wherein the polynucleotide sequence encodes for at least two RNA editing recruiting domains.

Embodiment 19. The vector of Embodiment 18, wherein at least one of the at least two RNA editing recruiting domains is an Alu domain.

Embodiment 20. The vector of Embodiment 19, wherein the Alu domain sequence forms a secondary structure that comprises at least one stem-loop.

Embodiment 21. The vector of Embodiment 19, wherein the Alu domain encoding sequence comprises a plurality of Alu repeats.

Embodiment 22. The vector of Embodiment 19, wherein the Alu domain encoding sequence is at least partially single stranded.

Embodiment 23. The vector of Embodiment 18, wherein at least one of the at least two RNA editing recruiting domains is an APOBEC recruiting domain.

Embodiment 24. The vector of Embodiment 18, wherein at least one of the at least two RNA editing recruiting domain encoding sequences comprises at least about 80% sequence identity to a GluR2 domain encoding sequence.

Embodiment 25. The vector of Embodiment 0, wherein at least one of the at least two RNA editing recruiting domains is a GluR2 domain.

Embodiment 26. The vector of Embodiment 18, wherein at least one of the at least two RNA editing recruiting domains is a Cas13 domain.

Embodiment 27. The vector of Embodiment 19 or 23, wherein the at least two RNA editing recruiting domains are the Alu domain and the APOBEC recruiting domain.

Embodiment 28. The vector of any one of Embodiments 1-27, that further comprises a nucleic acid encoding for an RNA that is complementary to at least a portion of a target RNA.

Embodiment 29. The vector of Embodiment 28, wherein the nucleic acid encoding for the RNA that is complementary to at least the portion of the target RNA is from about 10 base pairs (bp) to about 1000 bp in length.

Embodiment 30. The vector of Embodiment 28, wherein the nucleic acid encoding the at least one RNA editing entity recruiting domain and the nucleic acid encoding for the RNA that is complementary to at least the portion of the target RNA comprises a contiguous nucleic acid of at least about 200 bp in length.

Embodiment 31. The vector of any one of Embodiments 0-0, wherein the nucleic acid is chemically synthesized.

Embodiment 32. The vector of any one of Embodiments 0-0, wherein the nucleic acid is genetically encoded.

Embodiment 33. The vector of any one of Embodiments 0-0, wherein the vector comprises DNA.

Embodiment 34. The vector of Embodiment 33, wherein the DNA is double stranded.

Embodiment 35. The vector of Embodiment 33, wherein the DNA is single stranded.

Embodiment 36. The vector of any one of Embodiments 0-0, wherein the vector comprises RNA.

Embodiment 37. The vector of any one of Embodiments 0-0, wherein the RNA comprises a base modification.

Embodiment 38. The vector of any one of Embodiments 0-0, wherein the vector is an adeno-associated virus (AAV) vector.

Embodiment 39. The vector of Embodiment 38, wherein the AAV is a recombinant AAV (rAAV).

Embodiment 40. The vector of Embodiment 38, wherein the AAV is selected from the group consisting of an AAV1 serotype, an AAV2 serotype, an AAV3 serotype, an AAV4 serotype, an AAV5 serotype, an AAV6 serotype, an AAV7 serotype, an AAV8 serotype, an AAV9 serotype, a derivative of any these, and a combination of any of these.

Embodiment 41. The vector of Embodiment 38, wherein the AAV is the AAV5 serotype or a derivative thereof.

Embodiment 42. The vector of Embodiment 0, comprising the derivative of the AAV, wherein the derivative of the AAV comprises a modified VP1 protein.

Embodiment 43. The vector of Embodiment 3, wherein the APOBEC recruiting domain is selected from the group consisting of: an APOBEC1 recruiting domain, an APOBEC2 recruiting domain, an APOBEC3A recruiting domain, an APOBEC3B recruiting domain, an APOBEC3C recruiting domain, an APOBEC3E recruiting domain, an APOBEC3F recruiting domain, an APOBEC3G recruiting domain, an APOBEC3H recruiting domain, an APOBEC4 recruiting domain, and any combination thereof.

Embodiment 44. The vector of any one of Embodiments 1-43, wherein the at least one RNA editing entity recruiting domain recruits at least two RNA editing entities, and wherein at least one of the at least two polynucleotide sequences encoding for the RNA editing entities comprises at least about 80% identity to an APOBEC protein encoding sequence.

Embodiment 45. The vector of any one of Embodiments 1-43, wherein the at least one RNA editing entity recruiting domain recruits at least two RNA editing entities, and wherein at least one of the at least two polynucleotide sequences encoding for the RNA editing entities comprises at least about 80% identity to an ADAR protein encoding sequence.

Embodiment 46. The vector of any one of Embodiments 0-0, wherein the RNA recruiting domain encoded by the nucleic acid comprises at least one stem loop.

Embodiment 47. The vector of any one of Embodiments 1-46, wherein the polynucleotide sequence encoding the at least one RNA editing entity recruiting domain comprises a secondary structure that is substantially a cruciform.

Embodiment 48. The vector of any one of Embodiments 1-46, wherein the polynucleotide sequence encoding the at least one RNA editing entity recruiting domain comprises at least two secondary structures that are substantially cruciforms.

Embodiment 49. The vector of Embodiment 48, wherein the polynucleotide sequence encoding the at least one RNA editing entity recruiting domain is positioned between a polynucleotide sequence that forms the at least two secondary structures that are substantially cruciforms.

Embodiment 50. The vector of any one of Embodiments 47-49, wherein the cruciform secondary structure comprises a stem-loop adjoining at least one pair of at least partially complementary strands of the cruciform secondary structure.

Embodiment 51. The vector of any one of Embodiments 1-46, wherein the polynucleotide sequence encoding the at least one RNA editing recruiting domain comprises a secondary structure that is substantially a toehold.

Embodiment 52. A vector comprising a nucleic acid encoding for RNA with a two dimensional shape that is substantially a cruciform, wherein the RNA comprises at least one sequence encoding an RNA editing entity recruiting domain.

Embodiment 53. The vector of Embodiment 0, further comprising a nucleic acid encoding for RNA with at least one targeting domain encoding sequence that is complementary to at least a portion of a target RNA sequence.

Embodiment 54. The vector of Embodiment 0, wherein the nucleic acid encoding for the RNA with the at least one targeting domain that is complementary to at least the portion of the target RNA sequence further comprises a substantially linear two dimensional structure.

Embodiment 55. A non-naturally occurring RNA encoded by the vector of any one of Embodiments 0-0.

Embodiment 56. A non-naturally occurring RNA comprising a first domain sequence comprising a two dimensional shape that is substantially a cruciform and a second domain sequence that has a substantially linear two dimensional structure connected to the first domain sequence, wherein the first domain sequence encodes for an RNA editing entity recruiting domain and the second domain sequence encodes for a targeting domain, wherein the second domain sequence is complementary to at least a portion of a target RNA.

Embodiment 57. The non-naturally occurring RNA of Embodiment 0, further comprising a third domain sequence attached to the second domain sequence.

Embodiment 58. The non-naturally occurring RNA of Embodiment 0, wherein the third domain sequence comprises an RNA editing entity recruiting domain encoding sequence that forms a secondary structure having a two dimensional shape that is substantially a cruciform.

Embodiment 59. The non-naturally occurring RNA of any one of Embodiments 0-0, wherein at least one base of the non-naturally occurring RNA comprises a chemical modification.

Embodiment 60. The non-naturally occurring RNA of any one of Embodiments 0-0, wherein at least one sugar of the non-naturally occurring RNA comprises a chemical modification.

Embodiment 61. A nucleic acid comprising an RNA editing entity recruiting domain and an antisense domain sequence, wherein when the nucleic acid is contacted with an RNA editing entity and a target nucleic acid complementary to at least a portion of the antisense domain, modifies at least one base pair of the target nucleic acid at an efficiency of at least about 4 times greater than a comparable nucleic acid complexed with a Cas13b protein or an active fragment thereof, as determined by Sanger Method sequencing of the target nucleic acid.

Embodiment 62. A nucleic acid comprising an RNA editing entity recruiting domain and an antisense domain, wherein the nucleic acid when contacted with an RNA editing entity and a target nucleic acid complementary to at least a portion of the antisense domain, modifies at least one base pair of the target nucleic acid at an efficiency of at least about 4 times greater than a comparable nucleic acid complexed with a GluR2 domain and the antisense domain, as determined by Sanger Method sequencing of the target nucleic acid.

Embodiment 63. The nucleic acid of Embodiment 0 or 62, wherein the nucleic acid comprises RNA.

Embodiment 64. The nucleic acid of any one of Embodiments 51-63, wherein the target nucleic acid comprises RNA.

Embodiment 65. The nucleic acid of Embodiment 64, wherein the RNA is mRNA.

Embodiment 66. The nucleic acid of Embodiment 65, wherein the mRNA encodes a protein or a portion thereof.

Embodiment 67. The nucleic acid of Embodiment 0, wherein a dysfunction of the protein or portion thereof is implicated in a disease or condition.

Embodiment 68. The nucleic acid of Embodiment 0, wherein the disease or condition is selected from the group consisting of: a neurodegenerative disorder, a muscular disorder, a metabolic disorder, an ocular disorder, a cell proliferative disorder and any combination thereof.

Embodiment 69. The nucleic acid of Embodiment 64, wherein the RNA is small interfering RNA (siRNA).

Embodiment 70. The nucleic acid of any one of Embodiments 0-0, wherein the RNA editing entity recruiting domain comprises at least about 80% identity to a GluR2 domain.

Embodiment 71. The nucleic acid of any one of Embodiments 0-0, wherein the RNA editing entity recruiting domain comprises at least about 80% identity to an Alu domain.

Embodiment 72. The nucleic acid of any one of Embodiments 0-0, wherein the RNA editing entity recruiting domain comprises at least about 80% identity to an APOBEC recruiting domain.

Embodiment 73. The nucleic acid of any one of Embodiments 0-0, wherein the RNA editing entity recruiting domain is configured to recruit an ADAR protein.

Embodiment 74. The nucleic acid of Embodiment 0, wherein the ADAR protein is an ADAR1, ADAR2, or ADAR3 protein.

Embodiment 75. The nucleic acid of Embodiment 0 or 0, wherein the ADAR protein is a human ADAR protein.

Embodiment 76. The nucleic acid of Embodiment 0 or 0, wherein the ADAR protein is a recombinant ADAR protein.

Embodiment 77. The nucleic acid of Embodiment 0 or 0, wherein the ADAR protein is a modified ADAR protein.

Embodiment 78. The nucleic acid of any one of Embodiments 0-0, wherein the RNA editing entity recruiting domain is configured to recruit an APOBEC protein.

Embodiment 79. The nucleic acid of Embodiment 0, wherein the APOBEC protein is an APOBEC1, APOBEC2, APOBEC3A, APOBEC3B, APOBEC3C, APOBEC3E, APOBEC3F, APOBEC3G, APOBEC3H, or APOBEC4 protein.

Embodiment 80. The nucleic acid of Embodiment 0 or 0, wherein the ADAR protein is a human ADAR protein.

Embodiment 81. The nucleic acid of Embodiment 0 or 0, wherein the ADAR protein is a recombinant ADAR protein.

Embodiment 82. The nucleic acid of Embodiment 0 or 0, wherein the ADAR protein is a modified ADAR protein.

Embodiment 83. The nucleic acid of any one of Embodiments 0-0, wherein the nucleic acid is chemically synthesized.

Embodiment 84. The nucleic acid of any one of Embodiments 0-0, wherein the nucleic acid is genetically encoded.

Embodiment 85. A nucleic acid that comprises sequences comprising an antisense domain, a first stem-loop forming sequence, and a second stem-loop forming sequence, wherein the nucleic acid when contacted with (a) a first polypeptide comprising a first portion of an RNA editing entity and a first polynucleotide binding domain configured to bind to the first stem-loop forming sequence, and (b) a second polypeptide comprising a second portion of an RNA editing entity and a second polynucleotide binding domain configured to bind to the second stem-loop forming sequence, and (c) a target nucleic acid complementary to at least a portion of the antisense domain, modifies at least one base pair of the target nucleic acid.

Embodiment 86. The nucleic acid of Embodiment 0, wherein the first stem-loop or the second stem-loop are an MS2 stem loop.

Embodiment 87. The nucleic acid of Embodiment 0, wherein the first stem loop or the second stem-loop are a BoxB stem-loop.

Embodiment 88. The nucleic acid of Embodiment 0, wherein the first stem-loop or the second stem-loop are a U1A stem-loop.

Embodiment 89. The nucleic acid of any one of Embodiments 0-0, wherein the first portion of the RNA editing entity or the second portion of the RNA editing entity comprise an N-terminal fragment of an ADAR deaminase domain encoding sequence.

Embodiment 90. The nucleic acid of any one of Embodiments 0-0, wherein the first portion of the RNA editing entity or the second portion of the RNA editing entity comprise an C-terminal fragment of an ADAR deaminase domain encoding sequence.

Embodiment 91. The nucleic acid of any one of Embodiments 0-0, wherein the first polynucleotide binding domain or the second polynucleotide binding domain comprise an MS2 coat protein.

Embodiment 92. The nucleic acid of any one of Embodiments 0-0, wherein the first polynucleotide binding domain or the second polynucleotide binding domain comprise a Lambda N peptide.

Embodiment 93. The nucleic acid of any one of Embodiments 0-0, wherein the first polynucleotide binding domain or the second polynucleotide binding domain comprise a human nucleic acid binding protein.

Embodiment 94. The nucleic acid of Embodiment 0, wherein the human nucleic acid binding protein is a U1A protein, a TBP6.7 protein, a human histone stem-loop binding protein, or a DNA binding domain of a glucocorticoid receptor.

Embodiment 95. The nucleic acid of any one of Embodiments 0-0, wherein the RNA editing entity is capable of performing an adenosine to inosine mutation on the target nucleic acid.

Embodiment 96. The nucleic acid of any one of Embodiments 0-0, wherein the RNA editing entity is capable of performing a cytosine to thymine mutation on the target nucleic acid.

Embodiment 97. A kit that comprises the vector of any one of Embodiments 1-0 in a container, the non-naturally occurring RNA of any one of Embodiments 0-0 in a container, or the nucleic acid of any one of Embodiments 0-0 in a container.

Embodiment 98. The kit of Embodiment 97, further comprising a syringe.

Embodiment 99. The kit of Embodiment 98, wherein the container is the syringe.

Embodiment 100. An isolated cell that comprises the vector of any one of Embodiments 1-0, the non-naturally occurring RNA of any one of Embodiments 0-0, or the nucleic acid of any one of Embodiments 0-0.

Embodiment 101. A pharmaceutical composition that comprises the vector of any one of Embodiments 1-0 in unit dose form, the non-naturally occurring RNA of any one of Embodiments 0-0 in unit dose form, or the nucleic acid of any one of Embodiments 0-0 in unit dose form.

Embodiment 102. The pharmaceutical composition of Embodiment 0, further comprising a pharmaceutically acceptable excipient, diluent, or carrier.

Embodiment 103. The pharmaceutical composition of Embodiment 0, wherein the pharmaceutical composition comprises a second active ingredient.

Embodiment 104. A method of treating a disease or condition in a subject comprising administering to the subject the vector of any one of Embodiments 0-0, the non-naturally occurring RNA of any one of Embodiments 0-0, or the nucleic acid of any one of Embodiments 0-0.

Embodiment 105. The method of Embodiment 104, wherein the administering is by intravenous injection, intramuscular injection, an intrathecal injection, an intraorbital injection, a subcutaneous injection, or any combination thereof.

Embodiment 106. The method of Embodiment 104, further comprising administering a second therapy to the subject.

Embodiment 107. The method of Embodiment 104, wherein the disease or condition is selected from the group consisting of: a neurodegenerative disorder, a muscular disorder, a metabolic disorder, an ocular disorder, and any combination thereof.

Embodiment 108. The method of Embodiment 0, wherein the disease or condition is Alzheimer's disease.

Embodiment 109. The method of Embodiment 0, wherein the disease or condition is muscular dystrophy.

Embodiment 110. The method of Embodiment 0, wherein the disease or condition is retinitis pigmentosa.

Embodiment 111. The method of Embodiment 0, wherein the disease or condition is Parkinson disease.

Embodiment 112. The method of Embodiment 0, wherein the disease or condition is pain.

Embodiment 113. The method of Embodiment 0, wherein the disease or condition is Stargardt macular dystrophy.

Embodiment 114. The method of Embodiment 0, wherein the disease or condition is Charcot-Marie-Tooth disease.

Embodiment 115. The method of Embodiment 0, wherein the disease or condition is Rett syndrome.

Embodiment 116. The method of any one of Embodiments 0-0, wherein the administering is sufficient to decrease expression of a gene relative to prior to the administering.

Embodiment 117. The method of any one of Embodiments 0-0, wherein the administering is sufficient to edit at least one point mutation in the subject.

Embodiment 118. The method of any one of Embodiments 0-0, wherein the administering is sufficient to edit at least one stop codon in the subject, thereby producing a readthrough of the stop codon.

Embodiment 119. The method of any one of Embodiments 0-0, wherein the administering is sufficient to produce an exon skip in the subject.

Embodiment 120. A method of treating muscular dystrophy in a subject comprising administering to the subject a pharmaceutical composition comprising an adeno-associated virus (AAV) vector that comprises a first nucleic acid encoding a second nucleic acid, wherein the second nucleic acid comprises (a) an antisense region that is at least partially complementary to an RNA sequence implicated in muscular dystrophy, and (b) at least one RNA editing entity recruiting domain, wherein the at least one RNA editing entity recruiting domain does not comprise a stem-loop, or wherein the at least one RNA editing entity recruiting domain comprises at least about 80% sequence identity to at least one of: an Alu domain, an Apolipoprotein B mRNA Editing Catalytic Polypeptide-like (APOBEC) recruiting domain, and any combination thereof.

Embodiment 121. The method of Embodiment 0, wherein the pharmaceutical composition is in unit dose form.

Embodiment 122. The method of Embodiment 0 or 0, wherein the administering is at least once a week.

Embodiment 123. The method of Embodiment 0 or 0, wherein the administering is at least once a month.

Embodiment 124. The method of any one of Embodiments 0-0, wherein the administering is by injection.

Embodiment 125. The method of Embodiment 0, wherein the injection is subcutaneous, intravenous, infusion, intramuscular, intrathecal, or intraperitoneal injection.

Embodiment 126. The method of any one of Embodiments 0-0, wherein the administering is transdermal, transmucosal, oral, or pulmonary.

Embodiment 127. The method of any one of Embodiments 0-0, further comprising administering a second therapy to the subject.

Embodiment 128. A method of making a vector comprising: cloning at least one copy of a nucleic acid into the vector, wherein the nucleic acid encodes for at least one RNA editing entity recruiting domain, and wherein a sequence encoding the at least one RNA editing entity recruiting domain does not form a secondary structure that comprises a stem-loop, or wherein the nucleic acid that encodes the at least one RNA editing entity recruiting domain comprises at least about 80% sequence identity to a sequence selected from: an Alu domain encoding sequence, an Apolipoprotein B mRNA Editing Catalytic Polypeptide-like (APOBEC) recruiting domain encoding sequence, and any combination thereof.

Embodiment 129. The method of Embodiment 128, wherein the vector is a viral vector

Embodiment 130. The method of Embodiment 0, wherein the viral vector is an AAV vector.

Embodiment 131. The method of Embodiment 129 or 130, wherein the viral vector comprises a modified VP1 protein.

Embodiment 132. The method of Embodiment 128, wherein the vector is a liposome.

Embodiment 133. The method of Embodiment 128, wherein the vector is a nanoparticle.

Embodiment 134. The method of any one of Embodiments 128-133, further comprising transfecting or transducing the vector into an isolated human cell.

## Claims

1. A vector comprising a nucleic acid encoding an engineered ADAR1 or ADAR2 guide RNA comprising a sequence complementary to a target RNA, wherein the sequence complementary to the target RNA comprises 100 to 200 base pairs and wherein the engineered ADAR1 or ADAR2 guide RNA does not have an ADAR recruiting domain.

2. The vector according to claim 1, wherein the sequence complementary to a target RNA comprises 105 to 120 base pairs, 120 to 135 base pairs 135 to 150 base pairs, 150 to 165 base pairs, 165 to 180 base pairs, or 180 to 200 base pairs.

3. The vector according to any preceding claim, wherein the sequence complementary to the target RNA forms one or more mismatches with the corresponding base(s) in the sequence of the target RNA at or near the site to be edited.

4. The vector according to claim 3, wherein the sequence complementary to the target RNA forms a cytosine mismatch with a corresponding base in the sequence of the target RNA, which directs ADAR1 or ADAR2 to a target adenosine.

5. The vector according to any preceding claim, wherein the sequence complementary to the target RNA comprises one or more wobble-bases due to mismatches with one or more of the complementary base(s) in the targeted RNA sequence at or near the site to be edited

6. The vector according to any preceding claim, wherein the target RNA is an mRNA.

7. The vector according to claim 5, wherein the mRNA encodes a protein or a portion thereof and a dysfunction of the protein or portion thereof is implicated in a disease or condition.

8. The vector according to any preceding claim, wherein the nucleic acid encoding an engineered ADAR1 or ADAR2 guide RNA is operatively linked to a regulatory sequence.

9. The vector according to claim 8, wherein the regulatory sequence comprises a promotor.

10. The vector according to claim 9, wherein the promotor is a human U6, a mouse U6 promoter, a CMV promoter, a pol III promoter, or a pol II promoter.

11. The vector according to any proceeding claim, wherein the vector is a viral vector.

12. The viral vector of claim 11, wherein the viral vector is an adeno associated viral (AAV) vector.

13. The vector according to any one of claims 1 to 10, wherein the vector is a plasmid, liposome or nanoparticle.

14. A recombinant cell comprising the vector of any preceding claim.

15. A pharmaceutical composition comprising the vector of any one of claims 1 to 13 or the cell of claim 14.

16. The vector of any one of claims 1 to 13, the cell of claim 14 or the composition of claim 15, for use as a medicament.

17. The vector of any one of claims 1 to 13, the cell of claim 14 or the composition of claim 15, for use a method of treating a disease or disorder associated with aberrant protein expression.

18. The vector of any one of claims 1 to 13, the cell of claim 14 or the composition of claim 15, for use in the treatment of Duchenne Muscular Dystrophy.

19. The vector of any one of claims 1 to 13, the cell of claim 14 or the composition of claim 15, for use in treatment of Alzheimer's disease, muscular dystrophy, retinitis pigmentosa, Parkinson disease, pain, Stargardt macular dystrophy, Charcot-Marie-Tooth disease, or Rett syndrome.
